# EUROPEAN PATENT APPLICATION

(11) **EP 3 907 218 A1**
(43) Date of publication of application: **10.11.2021**
(21) Application number: 20736089.2
(22) Date of filing: 03.01.2020
(51) Int. Cl.: C07D 237/22, C07C 235/64, A61K 31/166, A61K 31/50, A61P 25/00, A61P 29/00

(54) **6-OXO-1,6-DIHYDROPYRIDAZINE DERIVATIVE, PREPARATION METHOD THEREFOR AND MEDICAL USE THEREOF**

(30) Priority: 04.01.2019 CN 201910006240; 08.05.2019 CN 201910380318; 09.05.2019 CN 201910384118; 27.06.2019 CN 201910567915
(71) Applicant: Jiangsu Hengrui Medicine Co., Ltd., Lianyungang, Jiangsu 222047 (CN); Shanghai Hengrui Pharmaceutical Co., Ltd., Shanghai 200245 (CN)
(72) Inventor: YANG, Fanglong, Shanghai 200245 (CN); YU, Nan, Shanghai 200245 (CN); CHI, Jiangtao, Shanghai 200245 (CN); HE, Feng, Shanghai 200245 (CN); TAO, Weikang, Shanghai 200245 (CN)
(74) Representative: Potter Clarkson
(86) International application number: PCT/CN2020/070186
(87) International publication number: WO 2020/140959

(57) **Abstract**

A 6-oxo-1,6-dihydropyridazine derivative, a preparation method therefor and medical use thereof, in particular, a 6-oxo-1,6-dihydropyridazine derivative represented by general formula (I), a preparation method therefor, and a pharmaceutical composition containing the derivative, and use thereof as a Na_{V} inhibitor and use thereof in the preparation of a drug for the treatment and/or prevention of pain and pain-related diseases. Each substituent in general formula (I) is the same as defined in the description.

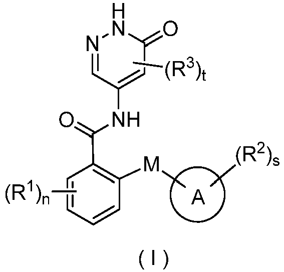

## Description

### TECHNICAL FIELD

The present disclosure belongs to the field of medicine, and relates to a 6-oxo-1,6-dihydropyridazine derivative, a method for preparing the same, and a use thereof in medicine. In particular, the present disclosure relates to a 6-oxo-1,6-dihydropyridazine derivative of formula (I), a method for preparing the same, a pharmaceutical composition comprising the same, a use thereof as a Na_{V} inhibitor, and a use thereof in the preparation of a medicament for treating and/or alleviating pain and pain-related diseases.

### BACKGROUND

Pain is a complex physical and psychological activity, and is one of the most common clinical symptoms. International Association for the Study of Pain defines pain as "an unpleasant sensory and emotional experience associated with actual or potential tissue damage, which is a subjective feeling". Pain can act as a warning signal to remind the body to pay attention to potential dangers, and has an indispensable protective effect on the body's normal life activities. Moreover, pain is also a common clinical symptom. After the external stimulus that causes the pain disappears, the strong or persistent pain can lead to the disorder of the physiological function and seriously affect the quality of life of the living body. Statistics show that about one-fifth of people in the world suffer from moderate to severe chronic pain.

Pain originates from the nociceptors in the peripheral nervous system. The nociceptors are a kind of free nerve ending, and widely distributed in the skin, muscles, joints and visceral tissues of the whole body. The nociceptors can convert thermal, mechanical or chemical stimuli into nerve impulses (action potentials), transmit them to the cell body in the dorsal root ganglia (DRG) through the afferent nerve fibers and ultimately to the advanced nerve center, thereby causing pain. The generation and conduction of action potentials in neurons depend on the voltage-gated sodium channels (Na_{V}) located on the cytomembrane. When the cytomembrane is depolarized, the sodium ion channel is activated. The channel is opened, causing sodium ion influx, and further depolarizing the cytomembrane, resulting in the generation of action potentials. Therefore, the inhibition of abnormal sodium ion channel activity contributes to the treatment and alleviation of pain.

Na_{V} is a kind of transmembrane ion channel protein. The protein consists of an alpha subunit with a molecular weight of 260 kD and a beta subunit with a molecular weight of 30-40 kD. According to the different α subunits, it can be divided into 9 subtypes, namely Na_{V}l.l to Na_{V}1.9. Different subtypes show different tissue distribution and electrophysiological and pharmacological characteristics (Rush A.M., et al. J. Physiol. 2007, 579, 1-14.). According to whether it can be effectively inhibited by nanomolar tetrodotoxin (TTX), the sodium ion channels are divided into TTX-sensitive type (TTX-S) and TTX- resistant type (TTX-R). Among them, Na_{V}1.1, Na_{V}1.2, Na_{V}1.3 and Na_{V}1.7 are TTX-S type, and the coding genes thereof are located in human chromosome 2q23-24, and they are expressed in large amounts in neurons. Na_{V}1.5, Na_{V}1.8 and Na_{V}1.9 are TTX-R type, and the coding genes thereof are located in human chromosome 3p21-24. Among them, Na_{V}1.5 mainly exists in cardiomyocytes, and Na_{V} 1.8 and Na_{V} 1.9 exist in the peripheral nervous system (Goldin A.L., et al. Annu. Rev. Physiol. 2001, 63, 871-894.). Na_{V}1.4 and Na_{V}1.6 are TTX-S type, and exist in skeletal muscle and central nervous system in large amounts, respectively (Fozzard H.A., et al. Physiol. Rev. 1996, 76, 887-926.). The local anesthetic lidocaine relieves pain by inhibiting Na_{V}. Non-selective Na_{V} inhibitors, such as lamotrigine, lacosamide and mexiletine have been successfully used to treat chronic pain.

Na_{V}1.8 is TTX-R type, the coding gene thereof is SCN10A. It mainly exists in trigeminal ganglion neurons and DRG neurons, and has the electrophysiological characteristics of slow inactivation and rapid recovery (Dib-Hajj S.D., et al. Annu. Rev. Neurosci. 2010, 33, 325-347.). In neurons expressing Na_{V}1.8, the rise of action potential is mainly composed of Na_{V}1.8 current. In some models for the study of neuropathic pain, nerve damage can increase the expression level of Na_{V}1.8 in axons and neuron cell bodies (Sleeper A.A., et al. J.Neurosci. 2000, 20, 7279-7289). The use of Na_{V}1.8 antisense oligonucleotide can significantly alleviate pain while reducing the expression of Na_{V}1.8 (Yoshimura N., et al. J.Neurosci. 2001, 21, 8690-8696). After carrageenan was injected into the paws of rats, the expression of Na_{V}1.8 in DRG neurons increased (Tanaka M., et al. G. NeuroReport 1998, 9, 967-972.). Na_{V}1.8-knockout mouse cannot show normal visceral inflammation pain (Kerr B.J., et al. NeuroReport 2001, 12, 3077-3080). After the human Na_{V}1.8 gene has a functional gain mutation, it will cause peripheral neuralgia (Faber C.G., et al. Proc. Natl. Acad. Sci. USA 2012, 109, 19444-19449.). According to a series of animal experiments and human genetic evidence, selective inhibition of Na_{V}1.8 has the potential to become a new type of analgesic therapy, which can be used for treating various types of pain such as inflammatory pain, neuropathic pain, postoperative pain and cancer pain.

The clinically used Na_{V} inhibitors can inhibit sodium channels expressed in the heart and central nervous system due to lack of subtype selectivity. Therefore, the therapeutic window is narrow and the scope of application is limited. Na_{V}1.8 is mainly distributed in the peripheral nervous system, thus selective inhibition of Na_{V}1.8 can effectively reduce side effects. Therefore, it is necessary to develop Na_{V}1.8 inhibitors with higher activity, better selectivity, better pharmacokinetic properties and fewer side effects.

### SUMMARY OF THE INVENTION

The object of the present disclosure is to provide a compound of formula (I) or a tautomer, mesomer, racemate, enantiomer, diastereomer thereof, or mixture thereof, or a pharmaceutically acceptable salt thereof: 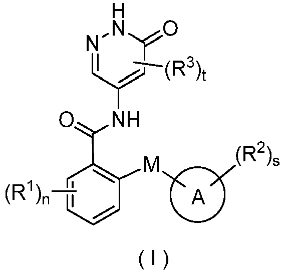 wherein:
M is selected from the group consisting of O atom, CR⁴R⁵ and S atom;
ring A is an aryl or heteroaryl, wherein the aryl or heteroaryl is optionally fused to a cycloalkyl or heterocyclyl;
each R¹ is identical or different and each is independently selected from the group consisting of hydrogen atom, halogen, alkyl, deuterated alkyl, deuterated alkoxy, alkoxy, haloalkyl, haloalkoxy, cyano, amino, nitro, hydroxy, hydroxyalkyl, cycloalkyl, heterocyclyl, aryl and heteroaryl;
each R² is identical or different and each is independently selected from the group consisting of hydrogen atom, halogen, alkyl, alkoxy, deuterated alkyl, deuterated alkoxy, haloalkyl, haloalkoxy, cyano, amino, nitro, hydroxy, hydroxyalkyl, cycloalkyl, cycloalkyloxy, heterocyclyl, aryl and heteroaryl, wherein the alkyl, cycloalkyl, heterocyclyl, aryl and heteroaryl are each optionally substituted by one or more substituents selected from the group consisting of alkyl, haloalkyl, halogen, amino, nitro, cyano, hydroxy, alkoxy, haloalkoxy, hydroxyalkyl, cycloalkyl, heterocyclyl, aryl and heteroaryl;
each R³ is identical or different and each is independently selected from the group consisting of hydrogen atom, halogen, alkyl, alkoxy, haloalkyl, cyano, amino, nitro, hydroxy, hydroxyalkyl, cycloalkyl, heterocyclyl, aryl and heteroaryl;
R⁴ and R⁵ are identical or different and are each independently selected from the group consisting of hydrogen atom, deuterium atom, halogen, alkyl, alkoxy, haloalkyl, cyano, amino, nitro, hydroxy, hydroxyalkyl, cycloalkyl, heterocyclyl, aryl and heteroaryl;
n is 0, 1, 2, 3 or 4;
s is 0, 1, 2, 3 or 4; and
t is 0, 1 or 2.

In some embodiments, the present disclosure provides a compound of formula (I) or a tautomer, mesomer, racemate, enantiomer, diastereomer thereof, or mixture thereof, or a pharmaceutically acceptable salt thereof: 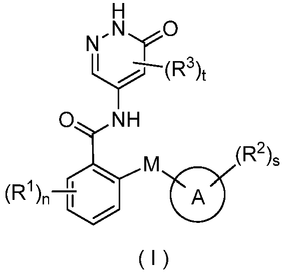 wherein:
M is selected from the group consisting of O atom, CR⁴R⁵ and S atom;
ring A is an aryl or heteroaryl;
R¹ is selected from the group consisting of hydrogen atom, halogen, alkyl, alkoxy, haloalkyl, haloalkoxy, cyano, amino, nitro, hydroxy, hydroxyalkyl, cycloalkyl, heterocyclyl, aryl and heteroaryl;
R² is selected from the group consisting of hydrogen atom, halogen, alkyl, alkoxy, haloalkyl, haloalkoxy, cyano, amino, nitro, hydroxy, hydroxyalkyl, cycloalkyl, cycloalkyloxy, heterocyclyl, aryl and heteroaryl, wherein the alkyl, cycloalkyl, heterocyclyl, aryl and heteroaryl are each optionally substituted by one or more substituents selected from the group consisting of alkyl, haloalkyl, halogen, amino, nitro, cyano, hydroxy, alkoxy, haloalkoxy, hydroxyalkyl, cycloalkyl, heterocyclyl, aryl and heteroaryl;
R³ is selected from the group consisting of hydrogen atom, halogen, alkyl, alkoxy, haloalkyl, cyano, amino, nitro, hydroxy, hydroxyalkyl, cycloalkyl, heterocyclyl, aryl and heteroaryl;
R⁴ and R⁵ are identical or different and are each independently selected from the group consisting of hydrogen atom, halogen, alkyl, alkoxy, haloalkyl, cyano, amino, nitro, hydroxy, hydroxyalkyl, cycloalkyl, heterocyclyl, aryl and heteroaryl;
n is 0, 1, 2, 3 or 4;
s is 0, 1, 2, 3 or 4; and
t is 0, 1 or 2.

In some embodiments, the present disclosure provides a compound of formula (I): 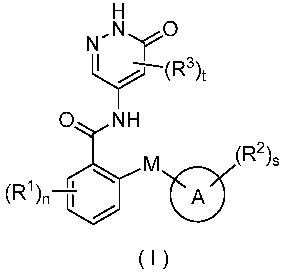 or a tautomer, mesomer, racemate, enantiomer, diastereomer thereof, or mixture thereof, or a pharmaceutically acceptable salt thereof,
wherein:
M is selected from the group consisting of O, CR⁴R⁵ and S;
ring A is an aryl or heteroaryl;
R¹ is selected from the group consisting of hydrogen atom, halogen, alkyl, alkoxy, haloalkyl, haloalkoxy, cyano, amino, nitro, hydroxy, hydroxyalkyl, cycloalkyl, heterocyclyl, aryl and heteroaryl;
R² is selected from the group consisting of hydrogen atom, halogen, alkyl, alkoxy, haloalkyl, haloalkoxy, cyano, amino, nitro, hydroxy, hydroxyalkyl, cycloalkyl, heterocyclyl, aryl and heteroaryl, wherein the alkyl, cycloalkyl, heterocyclyl, aryl and heteroaryl are each optionally substituted by one or more substituents selected from the group consisting of alkyl, haloalkyl, halogen, amino, nitro, cyano, hydroxy, alkoxy, haloalkoxy, hydroxyalkyl, cycloalkyl, heterocyclyl, aryl and heteroaryl;
R³ is selected from the group consisting of hydrogen atom, halogen, alkyl, alkoxy, haloalkyl, cyano, amino, nitro, hydroxy, hydroxyalkyl, cycloalkyl, heterocyclyl, aryl and heteroaryl;
R⁴ and R⁵ are identical or different and are each independently selected from the group consisting of hydrogen atom, halogen, alkyl, alkoxy, haloalkyl, cyano, amino, nitro, hydroxy, hydroxyalkyl, cycloalkyl, heterocyclyl, aryl and heteroaryl;
n is 0, 1, 2, 3 or 4;
s is 0, 1, 2, 3 or 4; and
t is 0, 1 or 2.

In some embodiments of the present disclosure, in the compound of formula (I) or the tautomer, mesomer, racemate, enantiomer, diastereomer thereof, or mixture thereof, or the pharmaceutically acceptable salt thereof, ring A is selected from the group consisting of phenyl, 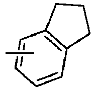 and pyridyl.

In some embodiments of the present disclosure, in the compound of formula (I) or the tautomer, mesomer, racemate, enantiomer, diastereomer thereof, or mixture thereof, or the pharmaceutically acceptable salt thereof, ring A is a phenyl or pyridyl.

In some embodiments of the present disclosure, in the compound of formula (I) or the tautomer, mesomer, racemate, enantiomer, diastereomer thereof, or mixture thereof, or the pharmaceutically acceptable salt thereof, M is selected from the group consisting of O atom, CH₂ and S atom.

In some embodiments of the present disclosure, in the compound of formula (I) or the tautomer, mesomer, racemate, enantiomer, diastereomer thereof, or mixture thereof, or the pharmaceutically acceptable salt thereof, M is an O atom.

In some embodiments of the present disclosure, the compound of formula (I) or the tautomer, mesomer, racemate, enantiomer, diastereomer thereof, or mixture thereof, or the pharmaceutically acceptable salt thereof is a compound of formula (II) or a tautomer, mesomer, racemate, enantiomer, diastereomer thereof, or mixture thereof, or a pharmaceutically acceptable salt thereof: 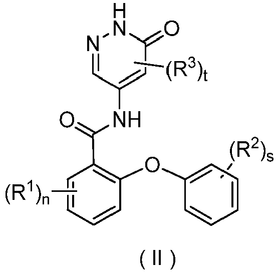 wherein:
R¹, R², R³, n, s and t are as defined in formula (I).

In some embodiments of the present disclosure, the compound of formula (I) or the tautomer, mesomer, racemate, enantiomer, diastereomer thereof, or mixture thereof, or the pharmaceutically acceptable salt thereof is a compound of formula (III) or a tautomer, mesomer, racemate, enantiomer, diastereomer thereof, or mixture thereof, or a pharmaceutically acceptable salt thereof: 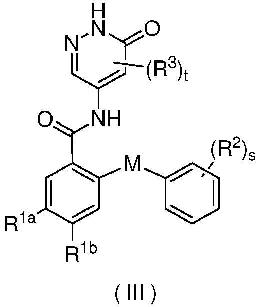 wherein:
M is selected from the group consisting of O atom, CH₂ and S atom;
R^{1a} is a halogen, and preferably selected from the group consisting of Cl, Br and F;
R^{1b} is selected from the group consisting of halogen, alkyl, alkoxy, haloalkyl and haloalkoxy, and preferably haloalkyl; and
R², R³, s and t are as defined in formula (I).

In some embodiments of the present disclosure, in the compound of formula (I) or the tautomer, mesomer, racemate, enantiomer, diastereomer thereof, or mixture thereof, or the pharmaceutically acceptable salt thereof, each R¹ is identical or different and each is independently selected from the group consisting of hydrogen atom, halogen, alkyl, alkoxy, haloalkyl and haloalkoxy.

In some embodiments of the present disclosure, in the compound of formula (I) or the tautomer, mesomer, racemate, enantiomer, diastereomer thereof, or mixture thereof, or the pharmaceutically acceptable salt thereof, each R¹ is identical or different and each is independently selected from the group consisting of hydrogen atom, halogen, alkyl and haloalkyl.

In some embodiments of the present disclosure, in the compound of formula (I) or the tautomer, mesomer, racemate, enantiomer, diastereomer thereof, or mixture thereof, or the pharmaceutically acceptable salt thereof, each R² is identical or different and each is independently selected from the group consisting of hydrogen atom, halogen, alkyl, deuterated alkyl, alkoxy, deuterated alkoxy, hydroxy, haloalkyl, haloalkoxy, cycloalkyl and cycloalkyloxy; preferably, each R² is identical or different and each is independently selected from the group consisting of hydrogen atom, halogen, C₁₋₆ alkyl, deuterated C₁₋₆ alkyl, C₁₋₆ alkoxy, deuterated C₁₋₆ alkoxy, haloC₁₋₆ alkyl, haloC₁₋₆ alkoxy, hydroxy, C₃₋₆ cycloalkyl and C₃₋₆ cycloalkyloxy; and more preferably, each R² is identical or different and each is independently selected from the group consisting of hydrogen atom, halogen, C₁₋₆ alkyl, C₁₋₆ alkoxy and deuterated C₁₋₆ alkoxy.

In some embodiments of the present disclosure, in the compound of formula (I) or the tautomer, mesomer, racemate, enantiomer, diastereomer thereof, or mixture thereof, or the pharmaceutically acceptable salt thereof, each R² is identical or different and each is independently selected from the group consisting of hydrogen atom, halogen, alkyl, alkoxy, deuterated alkoxy, hydroxy, haloalkyl, haloalkoxy, cycloalkyl and cycloalkyloxy; and preferably, each R² is identical or different and each is independently selected from the group consisting of hydrogen atom, halogen, alkyl, alkoxy, haloalkyl, haloalkoxy, cycloalkyl and cycloalkyloxy.

In some embodiments of the present disclosure, in the compound of formula (I) or the tautomer, mesomer, racemate, enantiomer, diastereomer thereof, or mixture thereof, or the pharmaceutically acceptable salt thereof, each R² is identical or different and each is independently selected from the group consisting of hydrogen atom, halogen, alkyl, alkoxy, haloalkyl and haloalkoxy.

In some embodiments of the present disclosure, in the compound of formula (I) or the tautomer, mesomer, racemate, enantiomer, diastereomer thereof, or mixture thereof, or the pharmaceutically acceptable salt thereof, s is 2.

In some embodiments of the present disclosure, the compound of formula (I) or the tautomer, mesomer, racemate, enantiomer, diastereomer thereof, or mixture thereof, or the pharmaceutically acceptable salt thereof is a compound of formula (IV) or a tautomer, mesomer, racemate, enantiomer, diastereomer thereof, or mixture thereof, or a pharmaceutically acceptable salt thereof: 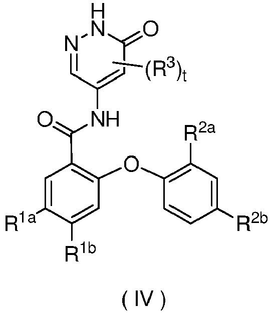 wherein:
R^{1a} is a halogen;
R^{1b} is selected from the group consisting of halogen, alkyl, alkoxy, haloalkyl and haloalkoxy;
R^{2a} is an alkoxy or deuterated alkoxy;
R^{2b} is selected from the group consisting of hydrogen atom, halogen, alkyl, alkoxy and haloalkoxy; and
R³ and t are as defined in formula (I).

In some embodiments of the present disclosure, in the compound of formula (I) or the tautomer, mesomer, racemate, enantiomer, diastereomer thereof, or mixture thereof, or the pharmaceutically acceptable salt thereof, R³ is a hydrogen atom.

In some embodiments of the present disclosure, in the compound of formula (I) or the tautomer, mesomer, racemate, enantiomer, diastereomer thereof, or mixture thereof, or the pharmaceutically acceptable salt thereof, R^{1a} is a chlorine atom, and R^{1b} is a trifluoromethyl.

Typical compounds of formula (I) include, but are not limited to:

| Example No. | Structure and name of the compound |
|---|---|
| 1 | 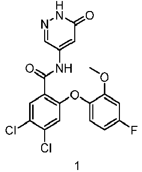 |
| | 4,5-Dichloro-2-(4-fluoro-2-methoxyphenoxy)-*N*-(6-oxo-1,6-dihydropyrid azin-4-yl)benzamide **1** |
| 2 | 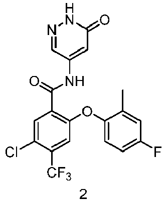 |
| | 5-Chloro-2-(4-fluoro-2-methylphenoxy)-*N*-(6-oxo-1,6-dihydropyridazin-4 -yl)-4-(trifluoromethyl)benzamide **2** |
| 3 | 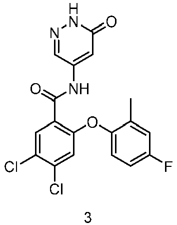 |
| | 4,5-Dichloro-2-(4-fluoro-2-methylphenoxy)-*N*-(6-oxo-1,6-dihydropyridazin-4-yl)benzamide **3** |
| 4 | 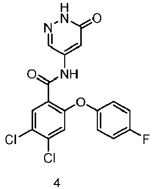 |
| | 4,5-Dichloro-2-(4-fluorophenoxy)-*N*-(6-oxo-1,6-dihydropyridazin-4-yl)be nzamide **4** |
| 5 | 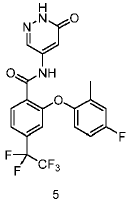 |
| | 2-(4-Fluoro-2-methylphenoxy)-*N*-(6-oxo-1,6-dihydropyridazin-4-yl)-4-(p erfluoroethyl)benzamide **5** |
| 6 | 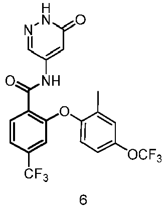 |
| | 2-(2-Methyl-4-(trifluoromethoxy)phenoxy)-*N*-(6-oxo-1,6-dihydropyridazi n-4-yl)-4-(trifluoromethyl)benzamide **6** |
| 7 | 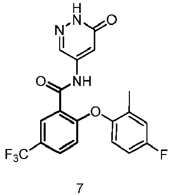 |
| | 2-(4-Fluoro-2-methylphenoxy)-*N*-(6-oxo-1,6-dihydropyridazin-4-yl)-5-(tr ifluoromethyl)benzamide **7** |
| 8 | 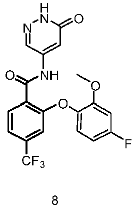 |
| | 2-(4-Fluoro-2-methoxyphenoxy)-*N*-(6-oxo-1,6-dihydropyridazin-4-yl)-4-( trifluoromethyl)benzamide **8** |
| 9 | 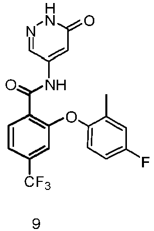 |
| | 2-(4-Fluoro-2-methylphenoxy)-*N*-(6-oxo-1,6-dihydropyridazin-4-yl)-4-(tr ifluoromethyl)benzamide **9** |
| 10 | 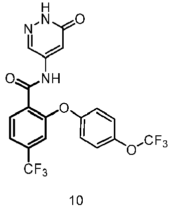 |
| | *N*-(6-Oxo-1,6-dihydropyridazin-4-yl)-2-(4-(trifluoromethoxy)phenoxy)-4-(trifluoromethyl)benzamide **10** |
| 11 | 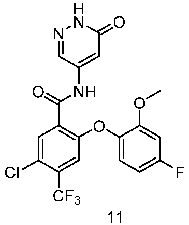 |
| | 5-Chloro-2-(4-fluoro-2-methoxyphenoxy)-*N*-(6-oxo-1,6-dihydropyridazin -4-yl)-4-(trifluoromethyl)benzamide **11** |
| 12 | 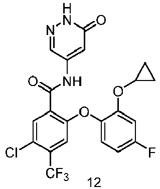 |
| | 5-Chloro-2-(2-cyclopropoxy-4-fluorophenoxy)-*N*-(6-oxo-1,6-dihydropyri dazin-4-yl)-4-(trifluoromethyl)benzamide **12** |
| 13 | 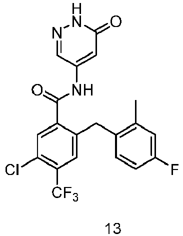 |
| | 5-Chloro-2-(4-fluoro-2-methylbenzyl)-*N-*(6-oxo-1,6-dihydropyridazin-4-yl)-4-(trifluoromethyl)benzamide **13** |
| 14 | 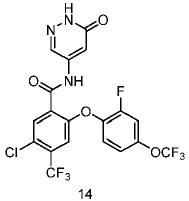 |
| | 5-Chloro-2-(2-fluoro-4-(trifluoromethoxy)phenoxy)-*N*-(6-oxo-1,6-dihydr opyridazin-4-yl)-4-(trifluoromethyl)benzamide **14** |
| 15 | 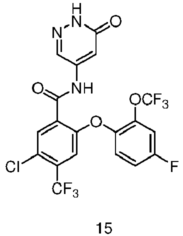 |
| | 5-Chloro-2-(4-fluoro-2-(trifluoromethoxy)phenoxy)-*N*-(6-oxo-1,6-dihydr opyridazin-4-yl)-4-(trifluoromethyl)benzamide **15** |
| 16 | 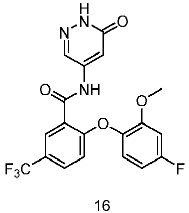 |
| | 2-(4-Fluoro-2-methoxyphenoxy)-*N*-(6-oxo-1,6-dihydropyridazin-4-yl)-5-( trifluoromethyl)benzamide **16** |
| 17 | 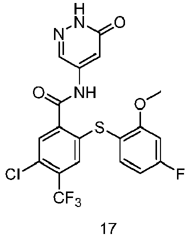 |
| | 5-Chloro-2-((4-fluoro-2-methoxyphenyl)thio)-*N*-(6-oxo-1,6-dihydropyrid azin-4-yl)-4-(trifluoromethyl)benzamide **17** |
| 18 | 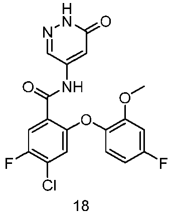 |
| | 4-Chloro-5-fluoro-2-(4-fluoro-2-methoxyphenoxy)-*N*-(6-oxo-1,6-dihydro pyridazin-4-yl)benzamide **18** |
| 19 | 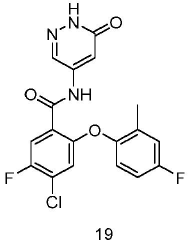 |
| | 4-Chloro-5-fluoro-2-(4-fluoro-2-methylphenoxy)-*N*-(6-oxo-1,6-dihydropy ridazin-4-yl)benzamide **19** |
| 20 | 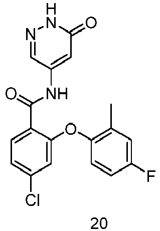 |
| | 4-Chloro-2-(4-fluoro-2-methylphenoxy)-*N*-(6-oxo-1,6-dihydropyridazin-4 -yl)benzamide **20** |
| 21 | 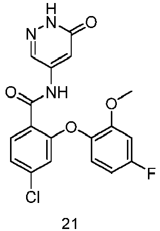 |
| | 4-Chloro-2-(4-fluoro-2-methoxyphenoxy)-*N*-(6-oxo-1,6-dihydropyridazin -4-yl)benzamide **21** |
| 22 | 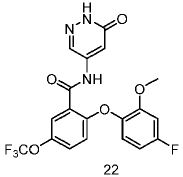 |
| | 2-(4-Fluoro-2-methoxyphenoxy)-*N*-(6-oxo-1,6-dihydropyridazin-4-yl)-5-( trifluoromethoxy)benzamide **22** |
| 23 | 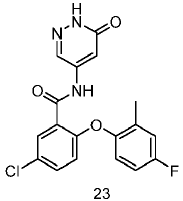 |
| | 5-Chloro-2-(4-fluoro-2-methylphenoxy)-*N*-(6-oxo-1,6-dihydropyridazin-4 -yl)benzamide **23** |
| 24 | 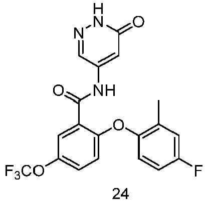 |
| | 2-(4-Fluoro-2-methylphenoxy)-*N*-(6-oxo-1,6-dihydropyridazin-4-yl)-5-(tr ifluoromethoxy)benzamide **24** |
| 25 | 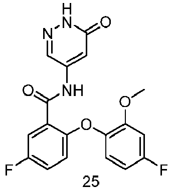 |
| | 5-Fluoro-2-(4-fluoro-2-methoxyphenoxy)-*N*-(6-oxo-1,6-dihydropyridazin -4-yl)benzamide **25** |
| 26 | 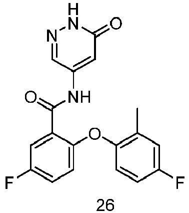 |
| | 5-Fluoro-2-(4-fluoro-2-methylphenoxy)-*N*-(6-oxo-1,6-dihydropyridazin-4 -yl)benzamide **26** |
| 27 | 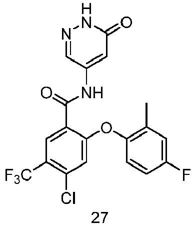 |
| | 4-Chloro-2-(4-fluoro-2-methylphenoxy)-*N*-(6-oxo-1,6-dihydropyridazin-4 -yl)-5-(trifluoromethyl)benzamide **27** |
| 28 | 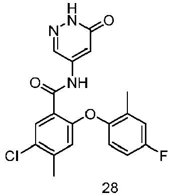 |
| | 5-Chloro-2-(4-fluoro-2-methylphenoxy)-4-methyl-*N*-(6-oxo-1,6-dihydrop yridazin-4-yl)benzamide **28** |
| 29 | 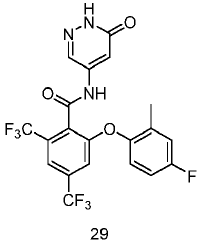 |
| | 2-(4-Fluoro-2-methylphenoxy)-*N*-(6-oxo-1,6-dihydropyridazin-4-yl)-4,6-bis(trifluoromethyl)benzamide **29** |
| 30 | 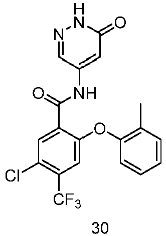 |
| | 5-Chloro-*N*-(6-oxo-1,6-dihydropyridazin-4-yl)-2-(*o*-tolyloxy)-4-(trifluoro methyl)benzamide **30** |
| 31 | 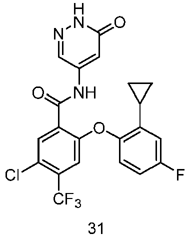 |
| | 5-Chloro-2-(2-cyclopropyl-4-fluorophenoxy)-*N*-(6-oxo-1,6-dihydropyrida zin-4-yl)-4-(trifluoromethyl)benzamide **31** |
| 32 | 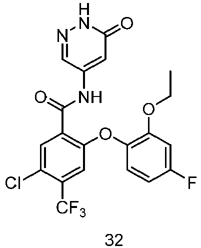 |
| | 5-Chloro-2-(2-ethoxy-4-fluorophenoxy)-*N*-(6-oxo-1,6-dihydropyridazin-4 -yl)-4-(trifluoromethyl)benzamide **32** |
| 33 | 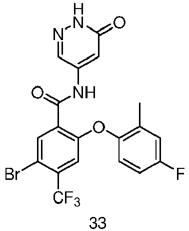 |
| | 5-Bromo-2-(4-fluoro-2-methylphenoxy)-*N*-(6-oxo-1,6-dihydropyridazin-4 -yl)-4-(trifluoromethyl)benzamide **33** |
| 34 | 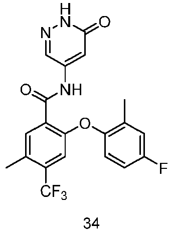 |
| | 2-(4-Fluoro-2-methylphenoxy)-5-methyl-*N*-(6-oxo-1,6-dihydropyridazin-4-yl)-4-(trifluoromethyl)benzamide **34** |
| 35 | 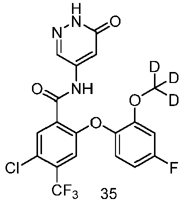 |
| | 5-Chloro-2-(4-fluoro-2-(methoxy-*d*₃)phenoxy)-*N*-(6-oxo-1,6-dihydropyrid azin-4-yl)-4-(trifluoromethyl)benzamide **35** |
| 36 | 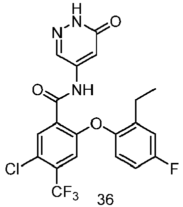 |
| | 5-Chloro-2-(2-ethyl-4-fluorophenoxy)-*N*-(6-oxo-1,6-dihydropyridazin-4-y l)-4-(trifluoromethyl)benzamide **36** |
| 37 | 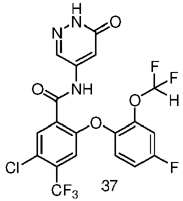 |
| | 5-Chloro-2-(2-(difluoromethoxy)-4-fluorophenoxy)-*N*-(6-oxo-1,6-dihydro pyridazin-4-yl)-4-(trifluoromethyl)benzamide **37** |
| 38 | 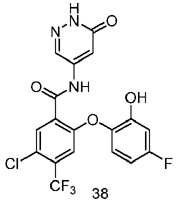 |
| | 5-Chloro-2-(4-fluoro-2-hydroxyphenoxy)-*N*-(6-oxo-1,6-dihydropyridazin-4-yl)-4-(trifluoromethyl)benzamide **38** |
| 39 | 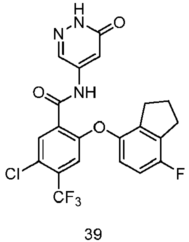 |
| | 5-Chloro-2-((7-fluoro-2,3-dihydro-1*H*-inden-4-yl)oxy)-*N*-(6-oxo-1,6-dihy dropyridazin-4-yl)-4-(trifluoromethyl)benzamide **39** |
| 40 | 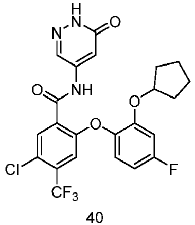 |
| | 5-Chloro-2-(2-(cyclopentyloxy)-4-fluorophenoxy)-*N*-(6-oxo-1,6-dihydrop yridazin-4-yl)-4-(trifluoromethyl)benzamide **40** |
| 41 | 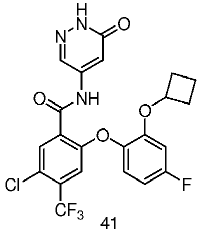 |
| | 5-Chloro-2-(2-cyclobutoxy-4-fluorophenoxy)-*N*-(6-oxo-1,6-dihydropyrid azin-4-yl)-4-(trifluoromethyl)benzamide **41** |
| 42 | 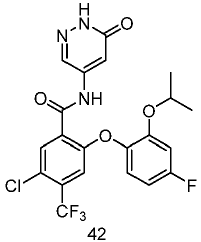 |
| | 5-Chloro-2-(4-fluoro-2-isopropylphenoxy)-*N*-(6-oxo-1,6-dihydropyridazi n-4-yl)-4-(trifluoromethyl)benzamide **42** |
| 43 | 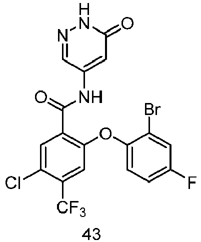 |
| | 2-(2-Bromo-4-fluorophenoxy)-5-chloro-*N-*(6-oxo-1,6-dihydropyridazin-4 -yl)-4-(trifluoromethyl)benzamide **43** |
| 44 | 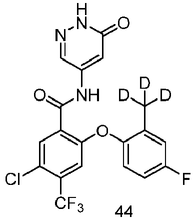 |
| | 5-Chloro-2-(4-fluoro-2-(methyl-*d*₃)phenoxy)-*N*-(6-oxo-1,6-dihydropyrida zin-4-yl)-4-(trifluoromethyl)benzamide **44** |
| 45 | 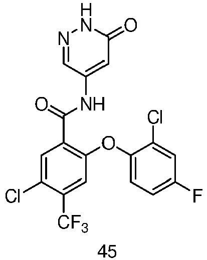 |
| | 5-Chloro-2-(2-chloro-4-fluorophenoxy)-*N*-(6-oxo-1,6-dihydropyridazin-4 -yl)-4-(trifluoromethyl)benzamide **45** |
| 46 | 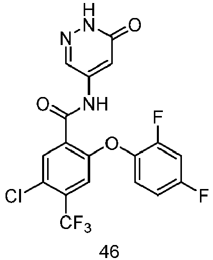 |
| | 5-Chloro-2-(2,4-difluorophenoxy)-*N*-(6-oxo-1,6-dihydropyridazin-4-yl)-4 -(trifluoromethyl)benzamide **46** |

or a tautomer, mesomer, racemate, enantiomer, diastereomer thereof, or mixture thereof, or a pharmaceutically acceptable salt thereof.

In another aspect, the present disclosure provides a compound of formula (IA), 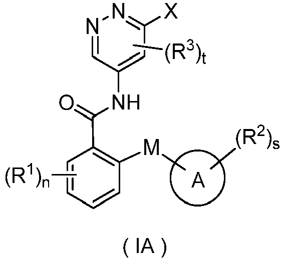 or a tautomer, mesomer, racemate, enantiomer, diastereomer thereof, or mixture thereof, or a pharmaceutically acceptable salt thereof, wherein:
X is a halogen, and preferably Cl; and
ring A, M, R¹, R², R³, n, s and t are as defined in the compound of formula (I). The 10 compound of formula (IA) is an intermediate for preparing the compound of formula (I).

In another aspect, the present disclosure provides a compound of formula (IIA), 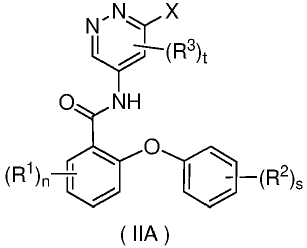 or a tautomer, mesomer, racemate, enantiomer, diastereomer thereof, or mixture thereof, or a pharmaceutically acceptable salt thereof,
wherein:
X is a halogen, and preferably Cl; and
ring A, R¹, R², R³, n, s and t are as defined in the compound of formula (II). The compound of formula (IIA) is an intermediate for preparing the compound of formula (II).

In another aspect, the present disclosure provides a compound of formula (IB), 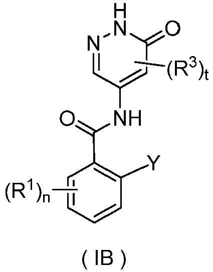 or a tautomer, mesomer, racemate, enantiomer, diastereomer thereof, or mixture thereof, or a pharmaceutically acceptable salt thereof,
wherein:
Y is a halogen, and preferably F; and
R¹, R³, n and t are as defined in the compound of formula (I). The compound of formula (IB) is an intermediate for preparing the compound of formula (I).

In another aspect, the present disclosure provides a compound of formula (IIIA), 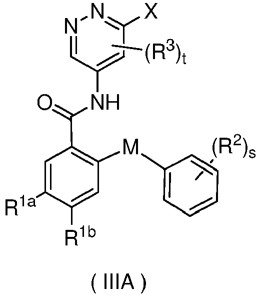 or a tautomer, mesomer, racemate, enantiomer, diastereomer thereof, or mixture thereof, or a pharmaceutically acceptable salt thereof,
wherein:
X is a halogen, and preferably Cl; and
M, R^{1a}, R^{1b}, R², R³, s and t are as defined in the compound of formula (III). The compound of formula (IIIA) is an intermediate for preparing the compound of formula (III) .

In another aspect, the present disclosure provides a compound of formula (IVA), 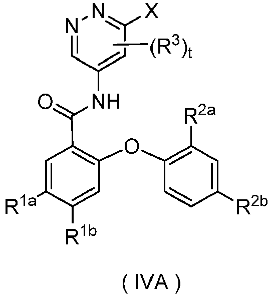 or a tautomer, mesomer, racemate, enantiomer, diastereomer thereof, or mixture thereof, or a pharmaceutically acceptable salt thereof,
wherein:
X is a halogen, and preferably Cl; and
R^{1a}, R^{1b}, R^{2a}, R^{2b}, R³, s and t are as defined in the compound of formula (IV). The compound of formula (IVA) is an intermediate for preparing the compound of formula (IV).

In another aspect, the present disclosure provides a compound of formula (IIIB), 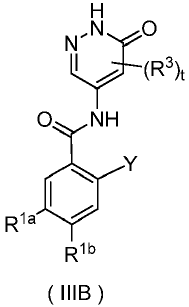 or a tautomer, mesomer, racemate, enantiomer, diastereomer thereof, or mixture thereof, or a pharmaceutically acceptable salt thereof,
wherein:
Y is a halogen, and preferably F; and
R^{1a}, R^{1b}, R³ and t are as defined in the compound of formula (III). The compound of formula (IIIB) is an intermediate for preparing the compound of formula (III).

Typical intermediate compounds include, but are not limited to:

| Example No. | Structure and name of the compound |
|---|---|
| 1d | 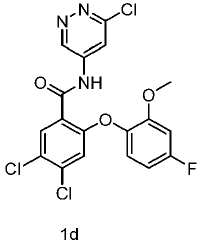 |
| | 4,5-Dichloro-*N*-(6-chloropyridazin-4-yl)-2-(4-fluoro-2-methoxyphenoxy) benzamide **1d** |
| 2g | 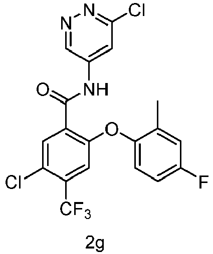 |
| | 5-Chloro-*N*-(6-chloropyridazin-4-yl)-2-(4-fluoro-2-methylphenoxy)-4-(tri fluoromethyl)benzamide **2g** |
| 7d | 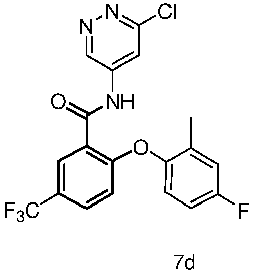 |
| | *N*-(6-Chloropyridazin-4-yl)-2-(4-fluoro-2-methylphenoxy)-5-(trifluorome thyl)benzamide **7d** |
| 5e | 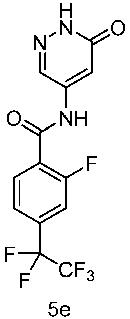 |
| | 2-Fluoro-*N*-(6-oxo-1,6-dihydropyridazin)-4-(perfluoroethyl)benzamide **5e** |
| 6d | 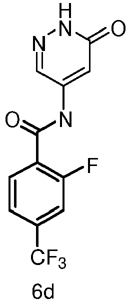 |
| | 2-Fluoro-*N*-(6-oxo-1,6-dihydropyridazin-4-yl)-4-(trifluoromethyl)benzam ide **6d** |
| 11b | 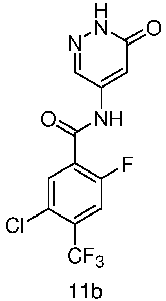 |
| | 5-Chloro-2-fluoro-*N*-(6-oxo-1,6-dihydropyridazin-4-yl)-4-(trifluoromethy l)benzamide **11b** |
| 13i | 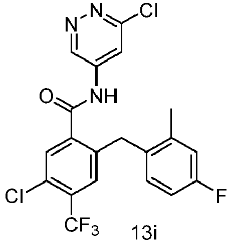 |
| | 5-Chloro-*N*-(6-chloropyridazin-4-yl)-2-(4-fluoro-2-methylbenzyl)-4-(trifl uoromethyl)benzamide **13i** |
| 14j | 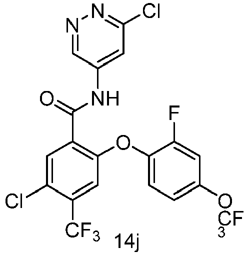 |
| | 5-Chloro-*N*-(6-chloropyridazin-4-yl)-2-(2-fluoro-4-(trifluoromethoxy)phe noxy)-4-(trifluoromethyl)benzamide **14j** |
| 27i | 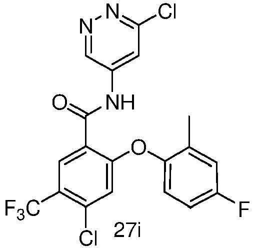 |
| | 4-Chloro-*N*-(6-chloropyridin-4-yl)-2-(4-fluoro-2-methylphenoxy)-5-(trifl uoromethyl)benzamide **27i** |

In another aspect, the present disclosure relates to a method for preparing the compound of formula (I), comprising a step of: 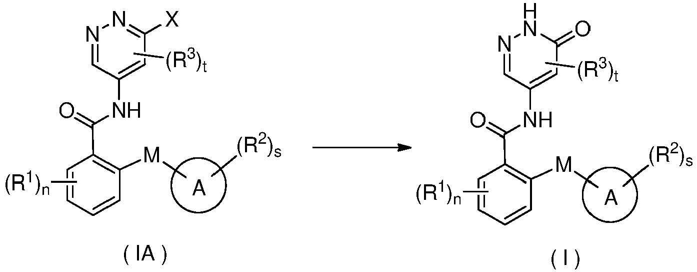 reacting a compound of formula (IA) to obtain the compound of formula (I);
wherein:
X is a halogen, and preferably Cl; and
ring A, M, R¹, R², R³, n, s and t are as defined in the compound of formula (I).

In another aspect, the present disclosure relates to a method for preparing the compound of formula (I), comprising a step of: 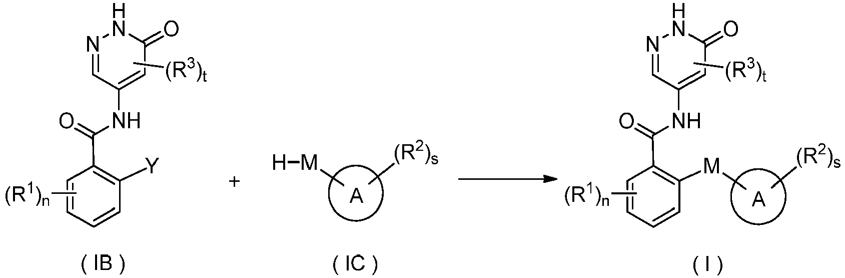 reacting a compound of formula (IB) and a compound of formula (IC) to obtain the compound of formula (I);
wherein:
Y is a halogen, and preferably F; and
ring A, M, R¹, R², R³, n, s and t are as defined in the compound of formula (I).

In another aspect, the present disclosure relates to a method for preparing the compound of formula (II), comprising a step of: 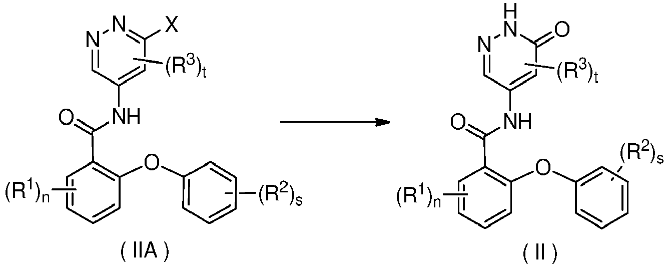 reacting a compound of formula (IIA) to obtain the compound of formula (II);
wherein:
X is a halogen, and preferably Cl; and
R¹, R², R³, n, s and t are as defined in the compound of formula (II).

In another aspect, the present disclosure relates to a method for preparing the compound of formula (II), comprising a step of: 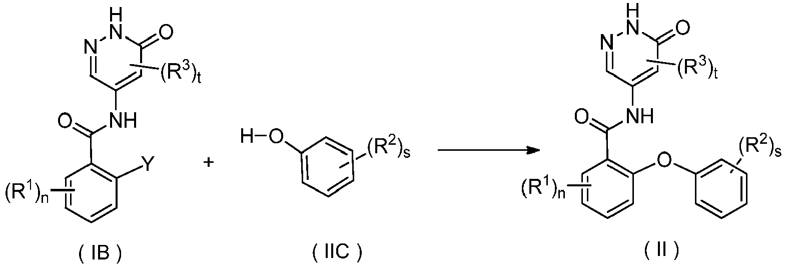 reacting a compound of formula (IB) and a compound of formula (IIC) to obtain the compound of formula (II);
wherein:
Y is a halogen, and preferably F; and
R¹, R², R³, n, s and t are as defined in the compound of formula (II).

In another aspect, the present disclosure relates to a method for preparing the compound of formula (III), comprising a step of: 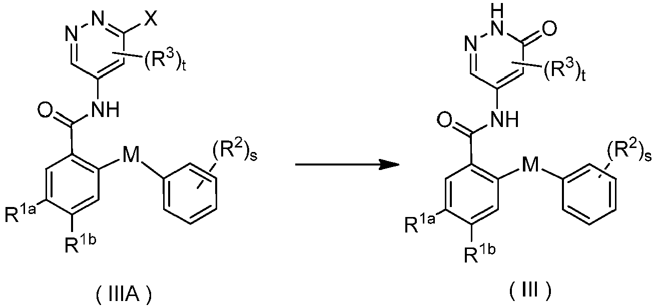 reacting a compound of formula (IIIA) to obtain the compound of formula (III);
wherein:
X is a halogen, and preferably Cl; and
M, R^{1a}, R^{1b}, R², R³, s and t are as defined in the compound of formula (III).

In another aspect, the present disclosure relates to a method for preparing the compound of formula (III), comprising a step of: 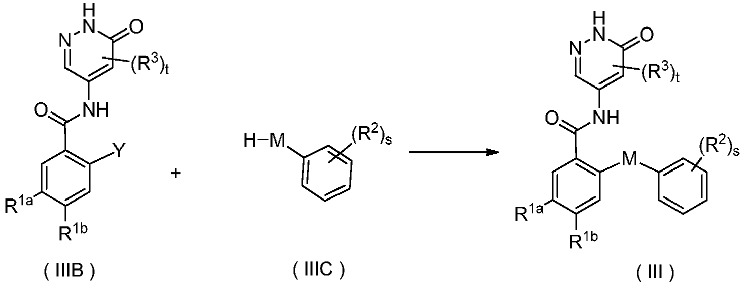 reacting a compound of formula (IIIB) and a compound of formula (IIIC) to obtain the compound of formula (III);
wherein:
Y is a halogen, and preferably F; and
M, R^{1a}, R^{1b}, R², R³, s and t are as defined in the compound of formula (III).

In another aspect, the present disclosure relates to a method for preparing the compound of formula (IV), comprising a step of: 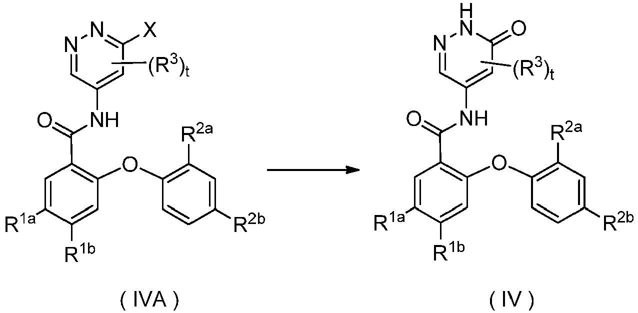 reacting a compound of formula (IVA) to obtain the compound of formula (IV);
wherein:
X is a halogen, and preferably Cl; and
R^{1a}, R^{1b}, R^{2a}, R^{2b}, R³ and t are as defined in the compound of formula (IV).

In another aspect, the present disclosure relates to a method for preparing the compound of formula (IV), comprising a step of: 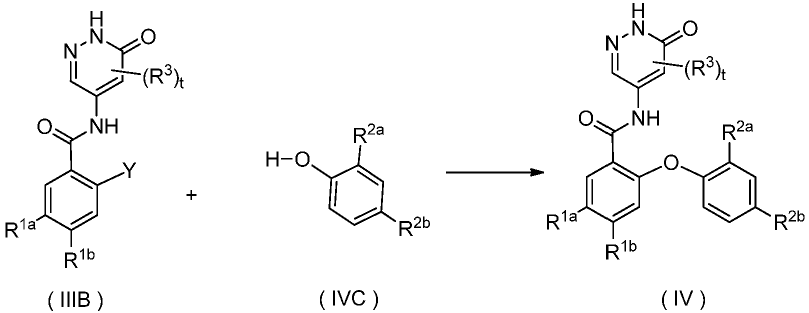 reacting a compound of formula (IIIB) and a compound of formula (IVC) to obtain the compound of formula (IV);
wherein:
Y is a halogen, and preferably F; and
R^{1a}, R^{1b}, R^{2a}, R^{2b}, R³ and t are as defined in the compound of formula (IV).

In another aspect, the present disclosure relates to a pharmaceutical composition comprising the aforementioned compound of formula (I) or the tautomer, mesomer, racemate, enantiomer, diastereomer thereof, or mixture thereof, or the pharmaceutically acceptable salt thereof, and one or more pharmaceutically acceptable carriers, diluents or excipients.

The present disclosure also relates to a method for preparing the aforementioned pharmaceutical composition, comprising a step of mixing the aforementioned compound of formula (I), or the tautomer, mesomer, racemate, enantiomer, diastereomer thereof, or mixture thereof, or the pharmaceutically acceptable salt thereof with the pharmaceutically acceptable carriers, diluents or excipients.

The present disclosure also relates to a use of the aforementioned compound of formula (I), or the tautomer, mesomer, racemate, enantiomer, diastereomer thereof, or mixture thereof, or the pharmaceutically acceptable salt thereof, or the aforementioned pharmaceutical composition in the preparation of a medicament for inhibiting the voltage-gated sodium channel in a subject. The voltage-gated sodium channel is preferaby Na_{V}1.8.

The present disclosure also relates to a use of the aforementioned compound of formula (I), or the tautomer, mesomer, racemate, enantiomer, diastereomer thereof, or mixture thereof, or the pharmaceutically acceptable salt thereof, or the aforementioned pharmaceutical composition in the preparation of a medicament for treating and/or alleviating pain and pain-related diseases, multiple sclerosis, Charcot-Marie-Tooth syndrome, incontinence or cardiac arrhythmia. The pain is preferaby selected from the group consisting of chronic pain, acute pain, inflammatory pain, cancer pain, neuropathic pain, musculoskeletal pain, primary pain, intestinal pain and idiopathic pain.

The present disclosure also relates to a method for inhibiting the voltage-gated sodium channel in a subject, comprising a step of administrating to a patient in need thereof the aforementioned compound of formula (I), or the tautomer, mesomer, racemate, enantiomer, diastereomer thereof, or mixture thereof, or the pharmaceutically acceptable salt thereof, or the aforementioned pharmaceutical composition of the present disclosure. The voltage-gated sodium channel is preferaby Na_{V}1.8.

The present disclosure also relates to a method for treating and/or alleviating pain and pain-related diseases, multiple sclerosis, Charcot-Marie-Tooth syndrome, incontinence or cardiac arrhythmia, comprising a step of administrating to a patient in need thereof the aforementioned compound of formula (I), or the tautomer, mesomer, racemate, enantiomer, diastereomer thereof, or mixture thereof, or the pharmaceutically acceptable salt thereof, or the aforementioned pharmaceutical composition of the present disclosure. The pain is preferaby selected from the group consisting of chronic pain, acute pain, inflammatory pain, cancer pain, neuropathic pain, musculoskeletal pain, primary pain, intestinal pain and idiopathic pain.

The present disclosure also relates to the compound of formula (I), or the tautomer, mesomer, racemate, enantiomer, diastereomer thereof, or mixture thereof, or the pharmaceutically acceptable salt thereof, or the aforementioned pharmaceutical composition, for use as a medicament.

The present disclosure also relates to the compound of formula (I), or the tautomer, mesomer, racemate, enantiomer, diastereomer thereof, or mixture thereof, or the pharmaceutically acceptable salt thereof, or the aforementioned pharmaceutical composition, for use as a medicament for inhibiting the voltage-gated sodium channel in a subject. The voltage-gated sodium channel is preferaby Na_{V}1.8.

The present disclosure also relates to the aforementioned compound of formula (I), or the tautomer, mesomer, racemate, enantiomer, diastereomer thereof, or mixture thereof, or the pharmaceutically acceptable salt thereof, or the aforementioned pharmaceutical composition, for use in treating and/or alleviating pain and pain-related diseases, multiple sclerosis, Charcot-Marie-Tooth syndrome, incontinence or cardiac arrhythmia. The pain is preferaby selected from the group consisting of chronic pain, acute pain, inflammatory pain, cancer pain, neuropathic pain, musculoskeletal pain, primary pain, intestinal pain and idiopathic pain.

The neuropathic pain in the present disclosure is preferaby selected from the group consisting of trigeminal neuralgia, postherpetic neuralgia, diabetic neuralgia, painful HIV-associated sensory neuropathy, burning syndrome, post-amputation pain, post spinal cord injury pain, phantom pain, painful neuroma, traumatic neuroma, Morton neuroma, nerve crush injury, spinal canal stenosis, carpal tunnel syndrome, radicular pain, sciatica pain, nerve avulsion, brachial plexus avulsion injury, complex regional pain syndrome, neuralgia caused by drug therapy, neuralgia caused by cancer chemotherapy, neuralgia caused by antiretroviral therapy, primary small fiber neuropathy, primary sensory neuralgia and trigeminal autonomic headache.

The musculoskeletal pain in the present disclosure is preferaby selected from the group consisting of osteoarthritis pain, back pain, cold pain, burning pain and dental pain.

The intestinal pain in the present disclosure is preferaby selected from the group consisting of inflammatory bowel disease pain, Crohn's disease pain and interstitial cystitis pain.

The inflammatory pain in the present disclosure is preferaby selected from the group consisting of rheumatoid arthritis pain and vulvar pain.

The idiopathic pain in the present disclosure includes fibromyalgia.

The dosage of the compound or composition used in the treatment method of the present disclosure will generally vary according to the severity of the disease, the weight of the patient, and the relative efficacy of the compound. However, as a general guide, a suitable unit dose can be 0.1 to 1000 mg.

In addition to the active compound, the pharmaceutical composition of the present disclosure can also comprise one or more auxiliaries including a filler (diluent), binder, wetting agent, disintegrant, excipient and the like. Depending on the administration mode, the composition can comprise 0.1 to 99% by weight of the active compound.

The pharmaceutical composition containing the active ingredient can be in a form suitable for oral administration, for example, a tablet, troche, lozenge, aqueous or oily suspension, dispersible powder or granule, emulsion, hard or soft capsule, syrup or elixir. An oral composition can be prepared according to any known method in the art for the preparation of pharmaceutical composition. Such a composition can contain one or more ingredient(s) selected from the group consisting of sweeteners, flavoring agents, colorants and preservatives, in order to provide a pleasing and palatable pharmaceutical formulation. The tablet contains the active ingredient in admixture with nontoxic, pharmaceutically acceptable excipients suitable for the manufacture of tablets. These excipients can be inert excipients, granulating agents, disintegrating agents and lubricants. The tablet can be uncoated or coated by means of a known technique to mask drug taste or delay the disintegration and absorption of the active ingredient in the gastrointestinal tract, thereby providing sustained release over a long period of time.

An oral formulation can also be provided as soft gelatin capsules in which the active ingredient is mixed with an inert solid diluent, or the active ingredient is mixed with a water-soluble carrier, an oil medium or olive oil.

An aqueous suspension comprises an active ingredient in admixture with excipients suitable for the manufacture of an aqueous suspension. Such excipients are suspending agents, dispersants or wetting agents. The aqueous suspension can also comprise one or more preservatives such as ethyl paraben or *n*-propyl paraben, one or more colorants, one or more flavoring agents, and one or more sweeteners.

An oil suspension can be formulated by suspending the active ingredient in a vegetable oil or mineral oil. The oil suspension can contain a thickener. The aforementioned sweeteners and flavoring agents can be added to provide a palatable formulation. These compositions can be preserved by adding an antioxidant.

The active ingredient in admixture with the dispersants or wetting agents, suspending agents or one or more preservatives can be prepared as dispersible powders or granules suitable for the preparation of an aqueous suspension by adding water. Suitable dispersants or wetting agents and suspending agents are exemplified by those already mentioned above. Additional excipients, such as sweeteners, flavoring agents and colorants, can also be added.

The pharmaceutical composition of the present disclosure can also be in the form of an oil-in-water emulsion. The oil phase can be a vegetable oil, or a mineral oil (such as liquid paraffin), or a mixture thereof. Suitable emulsifying agents can be naturally occurring phospholipids or partial esters. The emulsion can also contain a sweetening agent, flavoring agent, preservative and antioxidant.

The pharmaceutical composition of the present disclosure can be in the form of a sterile injectable aqueous solution. Acceptable vehicles or solvents that can be used are water, Ringer's solution or isotonic sodium chloride solution. The sterile injectable formulation can be a sterile injectable oil-in-water micro-emulsion in which the active ingredient is dissolved in the oil phase. The injectable solution or micro-emulsion can be introduced into a patient's bloodstream by local bolus injection.

The pharmaceutical composition of the present disclosure can be in the form of a sterile injectable aqueous or oily suspension for intramuscular and subcutaneous administration. Such a suspension can be formulated with suitable dispersants or wetting agents and suspending agents as described above according to known techniques. The sterile injectable formulation can also be a sterile injectable solution or suspension prepared in a nontoxic parenterally acceptable diluent or solvent. Moreover, sterile fixed oils can easily be used as a solvent or suspending medium.

The compound of the present disclosure can be administered in the form of a suppository for rectal administration. These pharmaceutical compositions can be prepared by mixing the drug with a suitable non-irritating excipient that is solid at ordinary temperatures, but liquid in the rectum, thereby melting in the rectum to release the drug.

It is well known to those skilled in the art that the dosage of a drug depends on a variety of factors including but not limited to, the following factors: activity of a specific compound, age of the patient, weight of the patient, general health of the patient, behavior of the patient, diet of the patient, administration time, administration route, excretion rate, drug combination and the like. In addition, the optimal treatment, such as treatment mode, daily dose of the compound of formula (I) or the type of pharmaceutically acceptable salt thereof can be verified by traditional therapeutic regimens.

### TERM DEDINITIONS

Unless otherwise stated, the terms used in the specification and claims have the meanings described below.

The term "alkyl" refers to a saturated aliphatic hydrocarbon group, which is a straight or branched chain group comprising 1 to 20 carbon atoms, preferably an alkyl having 1 to 12 carbon atoms, and more preferably an alkyl having 1 to 6 carbon atoms. Non-limiting examples include methyl, ethyl, *n*-propyl, isopropyl, *n*-butyl, isobutyl, *tert*-butyl*, sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, *n*-octyl*,* 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n*-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof. More preferably, the alkyl group is a lower alkyl having 1 to 6 carbon atoms, and non-limiting examples include methyl, ethyl, *n*-propyl, isopropyl, *n*-butyl, isobutyl, *tert*-butyl*, sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl and the like. The alkyl can be substituted or unsubstituted. When substituted, the substituent group(s) can be substituted at any available connection point. The substituent group(s) is one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy and alkoxycarbonyl.

The term "alkoxy" refers to an -O-(alkyl) or an -O-(unsubstituted cycloalkyl) group, wherein the alkyl is as defined above. Non-limiting examples of alkoxy include methoxy, ethoxy, propoxy, butoxy, cyclopropyloxy, cyclobutyloxy, cyclopentyloxy, cyclohexyloxy. The alkoxy can be optionally substituted or unsubstituted. When substituted, the substituent group(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and alkoxycarbonyl.

The term "cycloalkyl" refers to a saturated or partially unsaturated monocyclic or polycyclic hydrocarbon substituent group having 3 to 20 carbon atoms, preferably 3 to 12 carbon atoms, more preferably 3 to 6 carbon atoms (for example 3, 4, 5 or 6 carbon atoms), and most preferably 5 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl and the like. Polycyclic cycloalkyl includes a cycloalkyl having a spiro ring, fused ring or bridged ring.

The term "spiro cycloalkyl" refers to a 5 to 20 membered polycyclic group with individual rings connected through one shared carbon atom (called a spiro atom), wherein the rings can contain one or more double bonds, but none of the rings has a completely conjugated π-electron system. The spiro cycloalkyl is preferably a 6 to 14 membered spiro cycloalkyl, and more preferably a 7 to 10 membered spiro cycloalkyl (for example 7, 8, 9 or 10 membered spiro cycloalkyl). According to the number of the spiro atoms shared between the rings, the spiro cycloalkyl can be divided into a mono-spiro cycloalkyl, di-spiro cycloalkyl, or poly-spiro cycloalkyl, and the spiro cycloalkyl is preferably a mono-spiro cycloalkyl or di-spiro cycloalkyl, and more preferably a 4-membered/4-membered, 4-membered/5-membered, 4-membered/6-membered, 5-membered/5-membered, or 5-membered/6-membered mono-spiro cycloalkyl. Non-limiting examples of spiro cycloalkyl include:

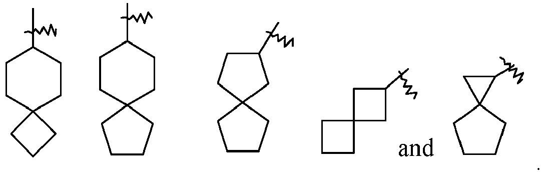

The term "fused cycloalkyl" refers to a 5 to 20 membered all-carbon polycyclic group, wherein each ring in the system shares an adjacent pair of carbon atoms with another ring, one or more rings can contain one or more double bonds, but none of the rings has a completely conjugated π-electron system. The fused cycloalkyl is preferably a 6 to 14 membered fused cycloalkyl, and more preferably a 7 to 10 membered fused cycloalkyl. According to the number of membered rings, the fused cycloalkyl can be divided into a bicyclic, tricyclic, tetracyclic or polycyclic fused cycloalkyl, and the fused cycloalkyl is preferably a bicyclic or tricyclic fused cycloalkyl, and more preferably a 5-membered/5-membered, or 5-membered/6-membered bicyclic fused cycloalkyl. Non-limiting examples of fused cycloalkyl include:

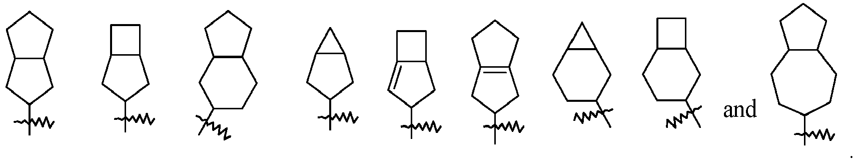

The term "bridged cycloalkyl" refers to a 5 to 20 membered all-carbon polycyclic group, wherein every two rings in the system share two disconnected carbon atoms, the rings can have one or more double bonds, but none of the rings has a completely conjugated π-electron system. The bridged cycloalkyl is preferably a 6 to 14 membered bridged cycloalkyl, and more preferably a 7 to 10 membered bridged cycloalkyl. According to the number of membered rings, the bridged cycloalkyl can be divided into a bicyclic, tricyclic, tetracyclic or polycyclic bridged cycloalkyl, and the bridged cycloalkyl is preferably a bicyclic, tricyclic or tetracyclic bridged cycloalkyl, and more preferably a bicyclic or tricyclic bridged cycloalkyl. Non-limiting examples of bridged cycloalkyl include:

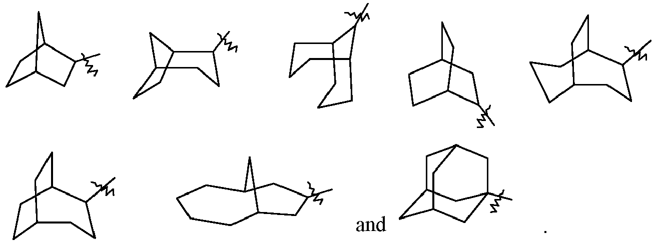

The cycloalkyl (including cycloalkyl, spiro cycloalkyl, fused cycloalkyl and bridged cycloalkyl) ring can be fused to the ring of aryl, heteroaryl or heterocyclyl, wherein the ring bound to the parent structure is cycloalkyl. Non-limiting examples include indanyl, tetrahydronaphthyl, benzocycloheptyl and the like, and preferably benzocyclopentyl, tetrahydronaphthyl. The cycloalkyl can be optionally substituted or unsubstituted. When substituted, the substituent group(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy and alkoxycarbonyl.

The term "heterocyclyl" refers to a 3 to 20 membered saturated or partially unsaturated monocyclic or polycyclic hydrocarbon substituent group, wherein one or more ring atoms are heteroatoms selected from the group consisting of N, O and S(O)ₘ (wherein m is an integer of 0 to 2), but excluding -O-O-, -O-S- or -S-S- in the ring, with the remaining ring atoms being carbon atoms. Preferably, the heterocyclyl has 3 to 12 ring atoms wherein 1 to 4 atoms are heteroatoms; most preferably, 3 to 8 ring atoms wherein 1 to 3 atoms are heteroatoms; and most preferably 5 to 6 ring atoms wherein 1 to 2 or 1 to 3 atoms are heteroatoms. Non-limiting examples of monocyclic heterocyclyl include pyrrolidinyl, imidazolidinyl, tetrahydrofuranyl, tetrahydropyranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl and the like, and preferably tetrahydropyranyl, piperidinyl, pyrrolidinyl. Polycyclic heterocyclyl includes a heterocyclyl having a spiro ring, fused ring or bridged ring.

The term "spiro heterocyclyl" refers to a 5 to 20 membered polycyclic heterocyclyl group with individual rings connected through one shared atom (called a spiro atom), wherein one or more ring atoms are heteroatoms selected from the group consisting of N, O and S(O)ₘ (wherein m is an integer of 0 to 2), with the remaining ring atoms being carbon atoms, where the rings can contain one or more double bonds, but none of the rings has a completely conjugated π-electron system. The spiro heterocyclyl is preferably a 6 to 14 membered spiro heterocyclyl, and more preferably a 7 to 10 membered spiro heterocyclyl. According to the number of the spiro atoms shared between the rings, the spiro heterocyclyl can be divided into a mono-spiro heterocyclyl, di-spiro heterocyclyl, or poly-spiro heterocyclyl, and the spiro heterocyclyl is preferably a mono-spiro heterocyclyl or di-spiro heterocyclyl, and more preferably a 4-membered/4-membered, 4-membered/5-membered, 4-membered/6-membered, 5-membered/5-membered, or 5-membered/6-membered mono-spiro heterocyclyl. Non-limiting examples of spiro heterocyclyl include:

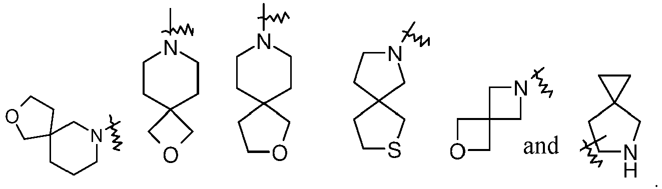

The term "fused heterocyclyl" refers to a 5 to 20 membered polycyclic heterocyclyl group, wherein each ring in the system shares an adjacent pair of atoms with another ring, wherein one or more rings can contain one or more double bonds, but none of the rings has a completely conjugated π-electron system, and wherein one or more ring atoms are heteroatoms selected from the group consisting of N, O and S(O)ₘ (wherein m is an integer of 0 to 2), with the remaining ring atoms being carbon atoms. The fused heterocyclyl is preferably a 6 to 14 membered fused heterocyclyl, and more preferably a 7 to 10 membered fused heterocyclyl. According to the number of membered rings, the fused heterocyclyl can be divided into a bicyclic, tricyclic, tetracyclic or polycyclic fused heterocyclyl, and the fused heterocyclyl is preferably a bicyclic or tricyclic fused heterocyclyl, and more preferably a 5-membered/5-membered or 5-membered/6-membered bicyclic fused heterocyclyl. Non-limiting examples of fused heterocyclyl include:

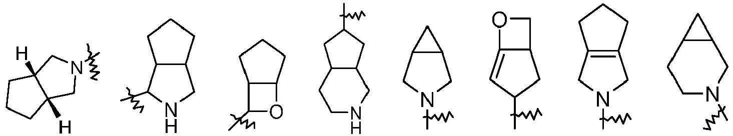

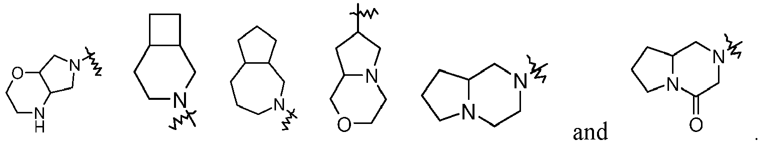

The term "bridged heterocyclyl" refers to a 5 to 14 membered polycyclic heterocyclyl group, wherein every two rings in the system share two disconnected atoms, wherein the rings can have one or more double bonds, but none of the rings has a completely conjugated π-electron system, and wherein one or more ring atoms are heteroatoms selected from the group consisting of N, O and S(O)ₘ (wherein m is an integer of 0 to 2), with the remaining ring atoms being carbon atoms. The bridged heterocyclyl is preferably a 6 to 14 membered bridged heterocyclyl, and more preferably a 7 to 10 membered bridged heterocyclyl. According to the number of membered rings, the bridged heterocyclyl can be divided into a bicyclic, tricyclic, tetracyclic or polycyclic bridged heterocyclyl, and the bridged heterocyclyl is preferably a bicyclic, tricyclic or tetracyclic bridged heterocyclyl, and more preferably a bicyclic or tricyclic bridged heterocyclyl. Non-limiting examples of bridged heterocyclyl include:

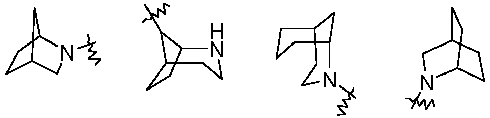

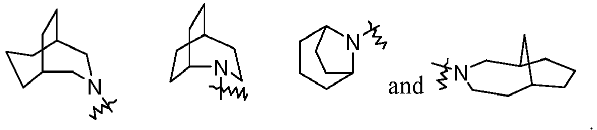

The heterocyclyl (including heterocyclyl, spiro heterocyclyl, fused heterocyclyl and bridged heterocyclyl) ring can be fused to the ring of aryl, heteroaryl or cycloalkyl, wherein the ring bound to the parent structure is heterocyclyl. Non-limiting examples thereof include: 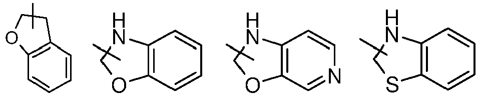 and the like.

The heterocyclyl can be optionally substituted or unsubstituted. When substituted, the substituent group(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy and alkoxycarbonyl.

The term "aryl" refers to a 6 to 14 membered all-carbon monocyclic ring or polycyclic fused ring (*i.e.* each ring in the system shares an adjacent pair of carbon atoms with another ring in the system) having a conjugated π-electron system, preferably a 6 to 10 membered aryl, for example, phenyl and naphthyl. The aryl ring can be fused to the ring of heteroaryl, heterocyclyl or cycloalkyl, wherein the ring bound to the parent structure is aryl ring. Non-limiting examples thereof include:

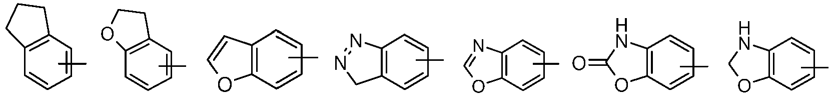

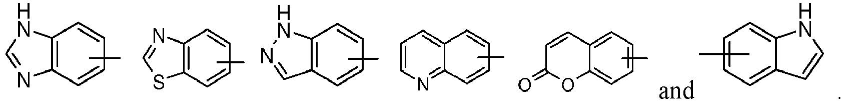

The aryl can be substituted or unsubstituted. When substituted, the substituent group(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and alkoxycarbonyl.

The term "heteroaryl" refers to a 5 to 14 membered heteroaromatic system having 1 to 4 heteroatoms selected from the group consisting of O, S and N. The heteroaryl is preferably a 5 to 10 membered heteroaryl having 1 to 3 heteroatoms, more preferably a 5 or 6 membered heteroaryl having 1 to 2 heteroatoms; preferably for example, imidazolyl, furyl, thienyl, thiazolyl, pyrazolyl, oxazolyl, pyrrolyl, tetrazolyl, pyridyl, pyrimidinyl, thiadiazolyl, pyrazinyl, pyridazinyl and the like, preferably pyridazinyl and pyridinyl, and more preferably pyridazinyl. The heteroaryl ring can be fused to the ring of aryl, heterocyclyl or cycloalkyl, wherein the ring bound to the parent structure is heteroaryl ring. Non-limiting examples thereof include:

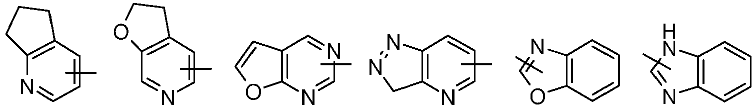

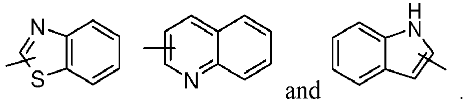

The heteroaryl can be optionally substituted or unsubstituted. When substituted, the substituent group(s) is preferably one or more groups independently selected from the group consisting of alkyl, alkenyl, alkynyl, alkoxy, alkylthio, alkylamino, halogen, thiol, hydroxy, oxo, nitro, cyano, cycloalkyl, heterocyclyl, aryl, heteroaryl, cycloalkoxy, heterocycloalkoxy, cycloalkylthio, heterocyclylthio, carboxy and alkoxycarbonyl, and non-limiting examples thereof include

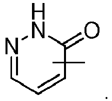

The term "hydroxyalkyl" refers to an alkyl group substituted by hydroxy(s), wherein the alkyl is as defined above.

The term "haloalkyl" refers to an alkyl group substituted by one or more halogens, wherein the alkyl is as defined above.

The term "haloalkoxy" refers to an alkoxy group substituted by one or more halogens, wherein the alkoxy is as defined above.

The term "deuterated alkyl" refers to an alkyl group substituted by one or more deuterium atoms, wherein the alkyl is as defined above.

The term "deuterated alkoxy" refers to an alkoxy group substituted by one or more deuterium atoms, wherein the alkoxy is as defined above.

The term "cycloalkylalkyl" refers to an alkyl group substituted by one or more cycloalkyls, wherein the cycloalkyl and alkyl are as defined above.

The term "cycloalkyloxy" refers to a -O-cycloalkyl group, wherein the cycloalkyl is as defined above.

The term "heterocyclylalkyl" refers to an alkyl group substituted by one or more heterocyclyls, wherein the heterocyclyl and alkyl are as defined above.

The term "arylalkyl" refers to an alkyl group substituted by one or more aryls, wherein the aryl and alkyl are as defined above.

The term "hydroxy" refers to an -OH group.

The term "halogen" refers to fluorine, chlorine, bromine or iodine.

The term "amino" refers to a -NH₂ group.

The term "cyano" refers to a -CN group.

The term "nitro" refers to a -NO₂ group.

The term "carboxy" refers to a -C(O)OH group.

The term "alkoxycarbonyl" refers to a -C(O)O(alkyl) or -C(O)O(cycloalkyl) group, wherein the alkyl and cycloalkyl are as defined above.

The term "acyl halide" refers to a compound containing a -C(O)-halogen group.

The compound of the present disclosure can also comprise isotopic derivatives thereof. The term "isotopic derivatives" refers to compounds that differ in structure only in the presence of one or more isotopically enriched atoms. For example, a compound having the structure of the present disclosure except replacing hydrogen with "deuterium" or "tritium", or replacing fluorine with an ¹⁸F-fluorine labeling (¹⁸F isotope), or replacing carbon with ¹¹C-, ¹³C-, or ¹⁴C-enriched carbon (¹¹C-, ¹³C-, or ¹⁴C-carbon labeling; ¹¹C-, ¹³C-, or ¹⁴C-isotope) is within the scope of the present disclosure. Such compounds can be used, for example, as analytical tools or probes in biological assays, or as tracers for *in vivo* diagnostic imaging of disease, or as tracers for pharmacodynamics, pharmacokinetics or receptor studies.

The present disclosure also comprises the compounds of formula (I) in various deuterated forms. Each of the available hydrogen atoms attached to the carbon atom can be independently replaced by a deuterium atom. Those skilled in the art can synthesize a compound of formula (I) in a deuterated form with reference to the relevant literatures. The compound of formula (I) in deuterated form can be prepared by employing commercially available deuterated raw materials, or they can be synthesized by conventional techniques with deuterated reagents including, but not limited to, deuterated borane, trideuterated borane in tetrahydrofuran, deuterated lithium aluminum hydride, deuterated iodoethane, deuterated iodomethane and the like.

"Optional" or "optionally" means that the event or circumstance described subsequently can, but need not, occur, and such a description includes the situation in which the event or circumstance does or does not occur. For example, "the heterocyclyl optionally substituted by an alkyl" means that an alkyl group can be, but need not be, present, and such a description includes the situation of the heterocyclyl being substituted by an alkyl and the heterocyclyl being not substituted by an alkyl.

"Substituted" refers to one or more hydrogen atoms in a group, preferably up to 5, and more preferably 1 to 3 hydrogen atoms, independently substituted by a corresponding number of substituents. It goes without saying that the substituents only exist in their possible chemical position. The person skilled in the art is able to determine whether the substitution is possible or impossible by experiments or theory without excessive effort. For example, the combination of amino or hydroxy having free hydrogen and carbon atoms having unsaturated bonds (such as olefinic) may be unstable.

The term "pharmaceutical composition" refers to a mixture of one or more of the compounds described herein or physiologically/pharmaceutically acceptable salts or prodrugs thereof with other chemical components, and other components such as physiologically/pharmaceutically acceptable carriers and excipients. The purpose of the pharmaceutical composition is to facilitate administration of a compound to an organism, which is conducive to the absorption of the active ingredient so as to show biological activity.

A "pharmaceutically acceptable salt" refers to a salt of the compound of the present disclosure, which is safe and effective in mammals and has the desired biological activity.

### Synthesis Method of the Compound of the Present Disclosure

In order to achieve the object of the present disclosure, the present disclosure applies the following technical solutions:

### Scheme I

The present disclosure provides a method for preparing the compound of formula (I) or the tautomer, mesomer, racemate, enantiomer, diastereomer thereof, or mixture thereof, or the pharmaceutically acceptable salt thereof, comprising the following steps of: 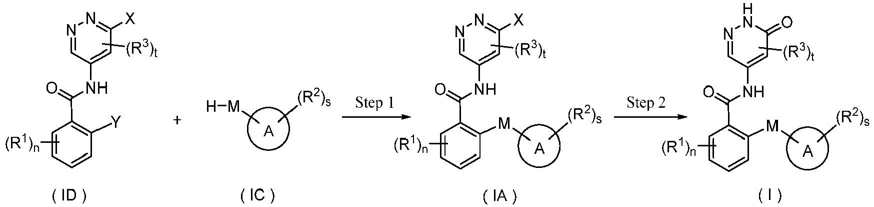 wherein:
X is a halogen, and preferably Cl;
Y is a halogen, and preferably F; and
ring A, M, R¹, R², R³, n, s and t are as defined in the compound of formula (I);
in Step 1, a compound of formula (ID) and a compound of formula (IC) are reacted under an alkaline condition to obtain a compound of formula (IA);
in Step 2, the compound of formula (IA) is reacted under an alkaline condition to obtain the compound of formula (I).

The reagent that provides an alkaline condition includes organic bases and inorganic bases. The organic bases include, but are not limited to, pyridine, hexahydropyridine, triethylamine, N,N-diisopropylethylamine, *n*-butyllithium, lithium diisopropylamide, potassium acetate, sodium *tert-*butoxide and potassium *tert*-butoxide. The inorganic bases include, but are not limited to, sodium hydride, potassium phosphate, sodium carbonate, sodium acetate, potassium carbonate, potassium acetate, cesium carbonate, sodium hydroxide and lithium hydroxide.

The above reactions are preferably carried out in a solvent. The solvent used includes, but is not limited to, acetic acid, trifluoroacetic acid, methanol, ethanol, toluene, tetrahydrofuran, dichloromethane, petroleum ether, ethyl acetate, *n*-hexane, dimethyl sulfoxide, 1,4-dioxane, water, *N*-methylpyrrolidone, *N*,*N*-dimethylformamide and mixtures thereof.

### Scheme II

The present disclosure provides a method for preparing the compound of formula (I) or the tautomer, mesomer, racemate, enantiomer, diastereomer thereof, or mixture thereof, or the pharmaceutically acceptable salt thereof, comprising the following steps of: 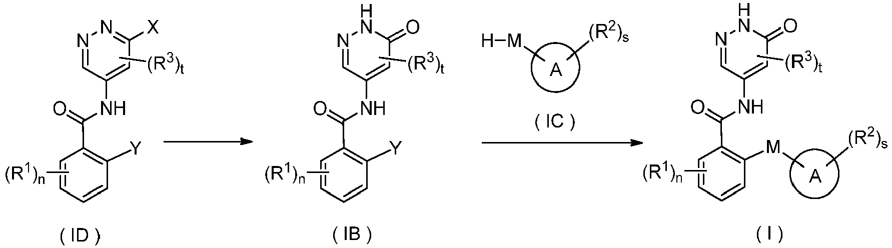 wherein:
X is a halogen, and preferably Cl;
Y is a halogen, and preferably F; and
ring A, M, R¹, R², R³, n, s and t are as defined in the compound of formula (I);
in Step 1, a compound of formula (ID) is reacted under an alkaline condition to obtain a compound of formula (IB);
in Step 2, the compound of formula (IB) and a compound of formula (IC) are reacted under an alkaline condition to obtain the compound of formula (I).

The reagent that provides an alkaline condition includes organic bases and inorganic bases. The organic bases include, but are not limited to, pyridine, hexahydropyridine, triethylamine, N,N-diisopropylethylamine, *n*-butyllithium, lithium diisopropylamide, potassium acetate, sodium *tert-*butoxide and potassium *tert*-butoxide. The inorganic bases include, but are not limited to, sodium hydride, potassium phosphate, sodium carbonate, sodium acetate, potassium carbonate, potassium acetate, cesium carbonate, sodium hydroxide and lithium hydroxide.

The above reactions are preferably carried out in a solvent. The solvent used includes, but is not limited to, acetic acid, trifluoroacetic acid, methanol, ethanol, toluene, tetrahydrofuran, dichloromethane, petroleum ether, ethyl acetate, *n*-hexane, dimethyl sulfoxide, 1,4-dioxane, water, *N*-methylpyrrolidone, *N*,*N*-dimethylformamide and mixtures thereof.

### Scheme III

The present disclosure provides a method for preparing the compound of formula (II) or the tautomer, mesomer, racemate, enantiomer, diastereomer thereof, or mixture thereof, or the pharmaceutically acceptable salt thereof, comprising the following steps of: 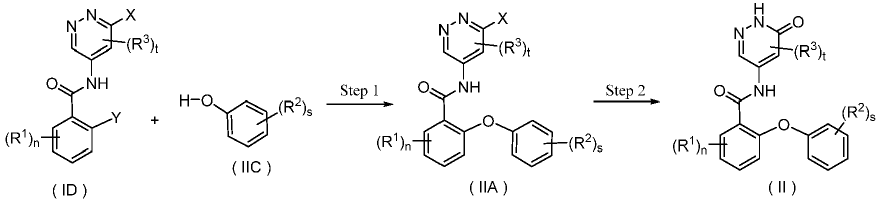 wherein:
X is a halogen, and preferably Cl;
Y is a halogen, and preferably F; and
R¹, R², R³, n, s and t are as defined in the compound of formula (II);
in Step 1, a compound of formula (ID) and a compound of formula (IIC) are reacted under an alkaline condition to obtain a compound of formula (IIA);
in Step 2, the compound of formula (IIA) is reacted under an alkaline condition to obtain the compound of formula (II).

The reagent that provides an alkaline condition includes organic bases and inorganic bases. The organic bases include, but are not limited to, pyridine, hexahydropyridine, triethylamine, N,N-diisopropylethylamine, *n*-butyllithium, lithium diisopropylamide, potassium acetate, sodium *tert-*butoxide and potassium *tert*-butoxide. The inorganic bases include, but are not limited to, sodium hydride, potassium phosphate, sodium carbonate, sodium acetate, potassium carbonate, potassium acetate, cesium carbonate, sodium hydroxide and lithium hydroxide.

The above reactions are preferably carried out in a solvent. The solvent used includes, but is not limited to, acetic acid, trifluoroacetic acid, methanol, ethanol, toluene, tetrahydrofuran, dichloromethane, petroleum ether, ethyl acetate, *n*-hexane, dimethyl sulfoxide, 1,4-dioxane, water, *N*-methylpyrrolidone, *N*,*N*-dimethylformamide and mixtures thereof.

### Scheme IV

The present disclosure provides a method for preparing the compound of formula (II) or the tautomer, mesomer, racemate, enantiomer, diastereomer thereof, or mixture thereof, or the pharmaceutically acceptable salt thereof, comprising the following step of: 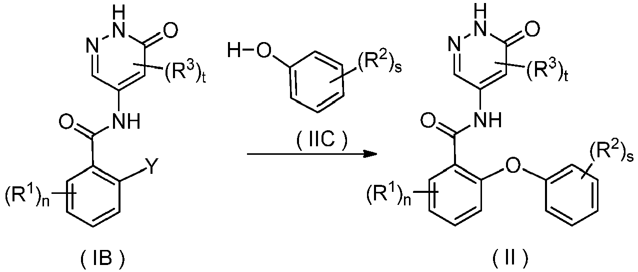 wherein:
Y is a halogen, and preferably F; and
R¹, R², R³, n, s and t are as defined in the compound of formula (II);
a compound of formula (IB) and a compound of formula (IIC) are reacted under an alkaline condition to obtain the compound of formula (II).

The reagent that provides an alkaline condition includes organic bases and inorganic bases. The organic bases include, but are not limited to, pyridine, hexahydropyridine, triethylamine, N,N-diisopropylethylamine, *n*-butyllithium, lithium diisopropylamide, potassium acetate, sodium *tert-*butoxide and potassium *tert*-butoxide. The inorganic bases include, but are not limited to, sodium hydride, potassium phosphate, sodium carbonate, sodium acetate, potassium carbonate, potassium acetate, cesium carbonate, sodium hydroxide and lithium hydroxide.

The above reaction is preferably carried out in a solvent. The solvent used includes, but is not limited to, acetic acid, trifluoroacetic acid, methanol, ethanol, toluene, tetrahydrofuran, dichloromethane, petroleum ether, ethyl acetate, *n*-hexane, dimethyl sulfoxide, 1,4-dioxane, water, *N*-methylpyrrolidone, *N*,*N*-dimethylformamide and mixtures thereof.

### Scheme V

The present disclosure provides a method for preparing the compound of formula (III) or the tautomer, mesomer, racemate, enantiomer, diastereomer thereof, or mixture thereof, or the pharmaceutically acceptable salt thereof, comprising the following step of: 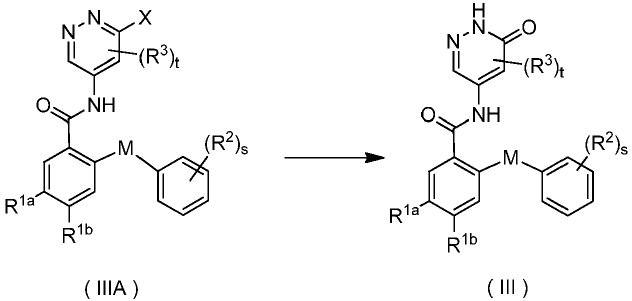 wherein:
X is a halogen, and preferably Cl; and
M, R^{1a}, R^{1b}, R², R³, s and t are as defined in the compound of formula (III);
a compound of formula (IIIA) is reacted under an alkaline condition to obtain the compound of formula (III).

The reagent that provides an alkaline condition includes organic bases and inorganic bases. The organic bases include, but are not limited to, pyridine, hexahydropyridine, triethylamine, N,N-diisopropylethylamine, *n*-butyllithium, lithium diisopropylamide, potassium acetate, sodium *tert-*butoxide and potassium *tert*-butoxide. The inorganic bases include, but are not limited to, sodium hydride, potassium phosphate, sodium carbonate, sodium acetate, potassium carbonate, potassium acetate, cesium carbonate, sodium hydroxide and lithium hydroxide, and preferably potassium acetate.

The above reaction is preferably carried out in a solvent. The solvent used includes, but is not limited to, acetic acid, trifluoroacetic acid, methanol, ethanol, toluene, tetrahydrofuran, dichloromethane, petroleum ether, ethyl acetate, *n*-hexane, dimethyl sulfoxide, 1,4-dioxane, water, *N*-methylpyrrolidone, *N*,*N*-dimethylformamide and mixtures thereof.

### Scheme VI

The present disclosure provides a method for preparing the compound of formula (III) or the tautomer, mesomer, racemate, enantiomer, diastereomer thereof, or mixture thereof, or the pharmaceutically acceptable salt thereof, comprising the following step of: 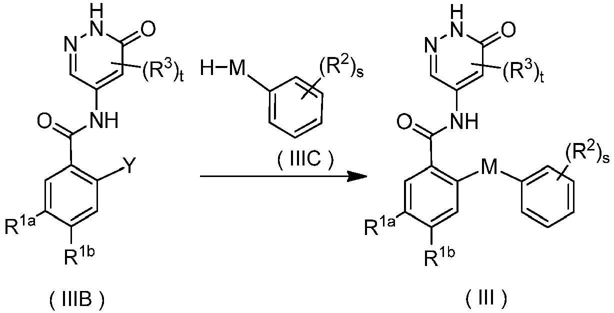 wherein:
Y is a halogen, and preferably F; and
M, R^{1a}, R^{1b}, R², R³, s and t are as defined in the compound of formula (III);
a compound of formula (IIIB) and a compound of formula (IIIC) are reacted under an alkaline condition to obtain the compound of formula (III).

The reagent that provides an alkaline condition includes organic bases and inorganic bases. The organic bases include, but are not limited to, pyridine, hexahydropyridine, triethylamine, N,N-diisopropylethylamine, *n*-butyllithium, lithium diisopropylamide, potassium acetate, sodium *tert-*butoxide and potassium *tert*-butoxide. The inorganic bases include, but are not limited to, sodium hydride, potassium phosphate, sodium carbonate, sodium acetate, potassium carbonate, potassium acetate, cesium carbonate, sodium hydroxide and lithium hydroxide, and preferably cesium carbonate.

The above reaction is preferably carried out in a solvent. The solvent used includes, but is not limited to, acetic acid, trifluoroacetic acid, methanol, ethanol, toluene, tetrahydrofuran, dichloromethane, petroleum ether, ethyl acetate, *n*-hexane, dimethyl sulfoxide, 1,4-dioxane, water, *N*-methylpyrrolidone, *N*,*N*-dimethylformamide and mixtures thereof.

### Scheme VII

The present disclosure provides a method for preparing the compound of formula (IV) or the tautomer, mesomer, racemate, enantiomer, diastereomer thereof, or mixture thereof, or the pharmaceutically acceptable salt thereof, comprising the following step of: 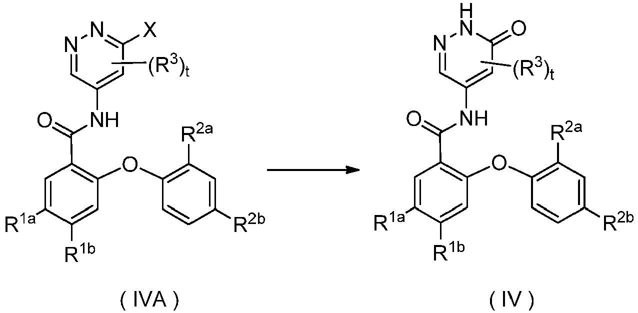 wherein:
X is a halogen, and preferably Cl; and
R^{1a}, R^{1b}, R^{2a}, R^{2b}, R³ and t are as defined in the compound of formula (IV);
a compound of formula (IVA) is reacted under an alkaline condition to obtain the compound of formula (IV).

The reagent that provides an alkaline condition includes organic bases and inorganic bases. The organic bases include, but are not limited to, pyridine, hexahydropyridine, triethylamine, N,N-diisopropylethylamine, *n*-butyllithium, lithium diisopropylamide, potassium acetate, sodium *tert-*butoxide and potassium *tert*-butoxide. The inorganic bases include, but are not limited to, sodium hydride, potassium phosphate, sodium carbonate, sodium acetate, potassium carbonate, potassium acetate, cesium carbonate, sodium hydroxide and lithium hydroxide, and preferably potassium acetate.

The above reaction is preferably carried out in a solvent. The solvent used includes, but is not limited to, acetic acid, trifluoroacetic acid, methanol, ethanol, toluene, tetrahydrofuran, dichloromethane, petroleum ether, ethyl acetate, *n*-hexane, dimethyl sulfoxide, 1,4-dioxane, water, *N*-methylpyrrolidone, *N*,*N*-dimethylformamide and mixtures thereof.

### Scheme VIII

The present disclosure provides a method for preparing the compound of formula (IV) or the tautomer, mesomer, racemate, enantiomer, diastereomer thereof, or mixture thereof, or the pharmaceutically acceptable salt thereof, comprising the following step of: 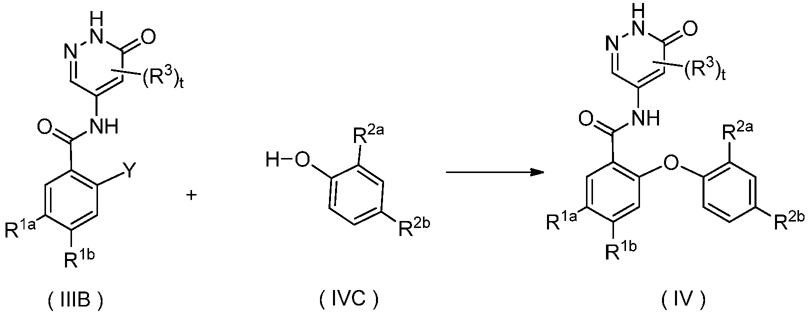 wherein:
Y is a halogen, and preferably F; and
ring A, R^{1a}, R^{1b}, R^{2a}, R^{2b}, R³ and t are as defined in the compound of formula (IV);
a compound of formula (IIIB) and a compound of formula (IVC) are reacted under an alkaline condition to obtain the compound of formula (IV).

The reagent that provides an alkaline condition includes organic bases and inorganic bases. The organic bases include, but are not limited to, pyridine, hexahydropyridine, triethylamine, N,N-diisopropylethylamine, *n*-butyllithium, lithium diisopropylamide, potassium acetate, sodium *tert-*butoxide and potassium *tert*-butoxide. The inorganic bases include, but are not limited to, sodium hydride, potassium phosphate, sodium carbonate, sodium acetate, potassium carbonate, potassium acetate, cesium carbonate, sodium hydroxide and lithium hydroxide, and preferably cesium carbonate.

The above reaction is preferably carried out in a solvent. The solvent used includes, but is not limited to, acetic acid, trifluoroacetic acid, methanol, ethanol, toluene, tetrahydrofuran, dichloromethane, petroleum ether, ethyl acetate, *n*-hexane, dimethyl sulfoxide, 1,4-dioxane, water, *N*-methylpyrrolidone, *N*,*N*-dimethylformamide and mixtures thereof.

### DETAILED DESCRIPTION

The present disclosure will be further described with reference to the following examples, but the examples should not be considered as limiting the scope of the present disclosure.

### EXAMPLES

The structures of the compounds were identified by nuclear magnetic resonance (NMR) and/or mass spectrometry (MS). NMR shifts (δ) are given in 10⁻⁶ (ppm). NMR is determined by a Bruker AVANCE-400 machine. The solvents for determination are deuterated-dimethyl sulfoxide (DMSO-*d*₆), deuterated-chloroform (CDCl₃) and deuterated-methanol (CD₃OD), and the internal standard is tetramethylsilane (TMS).

MS was determined by a FINNIGAN LCQAd (ESI) mass spectrometer (manufacturer: Thermo, type: Finnigan LCQ advantage MAX).

High performance liquid chromatography (HPLC) was determined on an Agilent HPLC 1200DAD, Agilent HPLC 1200VWD and Waters HPLC e2695-2489 high pressure liquid chromatograph.

Chiral HPLC was determined on an Agilent 1260 DAD high performance liquid chromatograph.

Preparative chromatography was carried out on Waters 2767, Waters 2767-SQ Detecor2, Shimadzu LC-20AP and Gilson-281 preparative chromatographs.

Chiral preparation was carried out on a Shimadzu LC-20AP preparative chromatograph.

CombiFlash rapid preparation instrument used was Combiflash Rf200 (TELEDYNE ISCO).

Yantai Huanghai HSGF254 or Qingdao GF254 silica gel plate was used as the thin-layer silica gel chromatography (TLC) plate. The dimension of the silica gel plate used in TLC was 0.15 mm to 0.2 mm, and the dimension of the silica gel plate used in product purification was 0.4 mm to 0.5 mm.

Yantai Huanghai 200 to 300 mesh silica gel was generally used as a carrier for silica gel column chromatography.

The average kinase inhibition rates and IC₅₀ values were determined by a NovoStar microplate reader (BMG Co., Germany).

The known starting materials of the present disclosure can be prepared by the known methods in the art, or can be purchased from ABCR GmbH & Co. KG, Acros Organnics, Aldrich Chemical Company, Accela ChemBio Inc., Dari Chemical Company etc.

Unless otherwise stated, the reactions were carried out under argon atmosphere or nitrogen atmosphere.

"Argon atmosphere" or "nitrogen atmosphere" means that a reaction flask is equipped with an argon or nitrogen balloon (about1 L).

"Hydrogen atmosphere" means that a reaction flask is equipped with a hydrogen balloon (about1 L).

Pressurized hydrogenation reaction was performed on a Parr 3916EKX hydrogenation instrument and a Qinglan QL-500 hydrogen generator or HC2-SS hydrogenation instrument.

In hydrogenation reactions, the reaction system was generally vacuumed and filled with hydrogen, and the above operation was repeated three times.

CEM Discover-S 908860 type microwave reactor was used in microwave reactions.

Unless otherwise stated, the solution refers to an aqueous solution.

Unless otherwise stated, the reaction temperature is room temperature from 20°C to 30°C.

The reaction process in the examples was monitored by thin layer chromatography (TLC). The developing solvent used in the reactions, the eluent system in column chromatography and the developing solvent system in thin layer chromatography for purification of the compounds included: A: dichloromethane/methanol system, B: *n*-hexane/ethyl acetate system, C: petroleum ether/ethyl acetate system, D: acetone, E: dichloromethane/acetone system, F: ethyl acetate/dichloromethane system, G: ethyl acetate/dichloromethane/*n*-hexane, and H: ethyl acetate/dichloromethane/acetone. The ratio of the volume of the solvent was adjusted according to the polarity of the compounds, and a small quantity of alkaline reagent such as triethylamine or acidic reagent such as acetic acid could also be added for adjustment.

### Example 1

### 4,5-Dichloro-2-(4-fluoro-2-methoxyphenoxy)-N-(6-oxo-1,6-dihydropyridazin-4-yl)benz amide 1

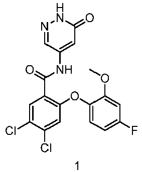

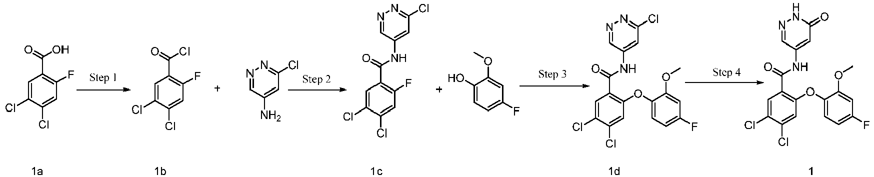

### Step 1

### 4,5-Dichloro-2-fluorobenzoyl chloride 1b

Compound 4,5-dichloro-2-fluorobenzoic acid **1a** (1.5 g, 7.18 mmol, Accela ChemBio (Shanghai) Inc.) was dissolved in thionyl chloride (10 mL), and the reaction solution was reacted at 80°C for 16 hours. The reaction solution was concentrated under reduced pressure to obtain the title compound **1b** (1.6 g), which was used directly in the next step without purification.

### Step 2

### 4,5-Dichloro-N-(6-chloropyridazin-4-yl)-2-fluorobenzamide 1c

The crude compound **1b** (1.6 g, 7.03 mmol) and 6-chloropyridazine-4-amine (500 mg, 3.86 mmol, Pharmablock Sciences (Nanjing), Inc.) were dissolved in pyridine (10 mL), and the reaction solution was stirred for 16 hours. The reaction solution was concentrated under reduced pressure, and the resulting residue was purified by silica gel column chromatography with eluent system B to obtain the title compound **1c** (650 mg, yield: 53%) as a white solid.
MS m/z (ESI): 321.9 [M+1]

### Step 3

### 4,5-Dichloro-N-(6-chloropyridazin-4-yl)-2-(4-fluoro-2-methoxyphenoxy)benzamide 1d

Compound **1c** (100 mg, 0.31 mmol), 4-fluoro-2-methoxyphenol (50 mg, 0.35 mmol, Accela ChemBio (Shanghai) Inc.) and cesium carbonate (153 mg, 0.47 mmol) were added to N,N-dimethylformamide (10 mL), and the reaction solution was reacted at 100°C for 2 hours. The reaction solution was cooled and filtered through diatomaceous earth. The filtrate was concentrated under reduced pressure, and the resulting residue was purified by silica gel column chromatography with eluent system A to obtain the title compound **1d** (100 mg, yield: 72%).
MS m/z (ESI): 443.7 [M+1]

### Step 4

### 4,5-Dichloro-2-(4-fluoro-2-methoxyphenoxy)-N-(6-oxo-1,6-dihydropyridazin-4-yl)benz amide 1

Compound **1d** (100 mg, 0.22 mmol) and potassium acetate (45 mg, 0.46 mmol) were added to acetic acid (5 mL), and the reaction solution was reacted at 120°C for 1.5 hours. The reaction solution was concentrated under reduced pressure, and the resulting residue was purified by silica gel column chromatography with eluent system A to obtain the title compound **1** (70 mg, yield: 73%).
MS m/z (ESI): 425.8 [M+1]
¹H NMR (400 MHz, DMSO-*d*₆) δ 12.81 (s, 1H), 10.85 (s, 1H),7.93 (s, 1H), 7.91 (d, 1H), 7.25-7.20 (m, 1H), 7.11(dd, 1H), 6.88 (s, 1H), 6.82-6.82 (m, 1H), 5.73 (s, 1H), 3.73 (s, 3H).

### Example 2

### 5-Chloro-2-(4-fluoro-2-methylphenoxy)-N-(6-oxo-1,6-dihydropyridazin-4-yl)-4-(trifluo romethyl)benzamide 2

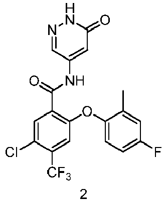

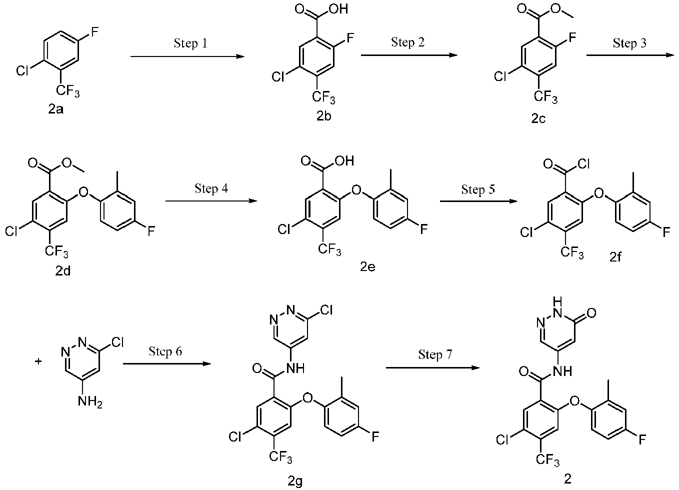

### Step 1

### 5-Chloro-2-fluoro-4-(trifluoromethyl)benzoic acid 2b

2,2,6,6-Tetramethylpiperidine (19.2 g, 135.93 mmol, Accela ChemBio (Shanghai) Inc.) was added to tetrahydrofuran (200 mL) under an argon atmosphere. The reaction solution was cooled to 0°C, then *n*-butyl lithium (1.6 M, 85.1 mL) was added dropwise within about 45 minutes at a controlled temperature below 3°C. The reaction solution was reacted at 0°C for 1 hour, and then cooled to -78°C. Compound 1-chloro-4-fluoro-2-(trifluoromethyl)benzene **2a** (18 g, 90.66 mmol, Shanghai Titan Scientific Co., Ltd.) was added dropwise, and the reaction solution was reacted for 3 hours. Excess dry ice was added, and the reaction solution was naturally warmed up to 0°C, followed by the addition of 150 mL of ice water. The reaction solution was separated into two phases. The aqueous phase was adjusted to pH 5 to 6 with concentrated hydrochloric acid and extracted with ethyl acetate (50 mL), and the organic phase was concentrated under reduced pressure. The crude product was washed with *n*-hexane (50 mL), then purified by silica gel column chromatography with eluent system A to obtain the title compound **2b** (15 g, yield: 68%).
MS m/z (ESI): 241.1 [M-1]

### Step 2

### Methyl 5-chloro-2-fluoro-4-(trifluoromethyl)benzoate 2c

Compound **2b** (5 g, 20.61 mmol) was added to thionyl chloride (49.2 g, 413.55 mmol), and the reaction solution was reacted at 80°C for 2 hours. The reaction solution was concentrated under reduced pressure. The resulting oil was added dropwise to methanol (100 mL), and the reaction solution was reacted at room temperature for 1 hour. The reaction solution was concentrated under reduced pressure, and the resulting residue was purified by silica gel column chromatography with eluent system B to obtain the title compound **2c** (2.78 g, yield: 52%).

### Step 3

Methyl 5-chloro-2-(4-fluoro-2-methylphenoxy)-4-(trifluoromethyl)benzoate **2d** Compound **2c** (2.78 g, 10.83 mmol), 4-fluoro-2-methyl-phenol (1.5 g, 11.89 mmol, Shanghai Bide Pharmatech Ltd.) and cesium carbonate (6 g, 18.41 mmol) were added to *N*,*N*-dimethylformamide (20 mL), and the reaction solution was reacted at 100°C for 1 hour. The reaction solution was cooled and filtered. The filtrate was concentrated to obtain the target compound **2d** (3.92 g), which was used directly in the next step without purification.
MS m/z (ESI): 363.1 [M+1]

### Step 4

### 5-Chloro-2-(4-fluoro-2-methylphenoxy)-4-(trifluoromethyl)benzoic acid 2e

Compound **2d** (3.92 g, 10.81 mmol) was dissolved in methanol (30 mL), followed by the addition of water (10 mL) and sodium hydroxide (1.3 g, 32.5 mmol), and the reaction solution was reacted for 16 hours. The reaction solution was concentrated, followed by the addition of 10 mL of water, and the pH was adjusted to 1 with concentrated hydrochloric acid. The resulting solution was extracted with ethyl acetate (20 mL×3), and the organic phase was dried over anhydrous sodium sulfate and filtered. The filtrate was concentrated under reduced pressure, and the resulting residue was purified by silica gel column chromatography with eluent system A to obtain the title compound **2e** (3.67 g, yield: 97%).
MS m/z (ESI): 346.8 [M-1]

### Step 5

### 5-Chloro-2-(4-fluoro-2-methylphenoxy)-4-(trifluoromethyl)benzoyl chloride 2f

Compound **2e** (3.67 g, 10.52 mmol) was added to thionyl chloride (20 g, 168.1 mmol), and the reaction solution was reacted at 80°C for 2 hours. The reaction solution was concentrated to obtain the title compound **2f** (3.86 g), which was used directly in the next step without purification.

### Step 6

### 5-Chloro-N-(6-chloropyridazin-4-yl)-2-(4-fluoro-2-methylphenoxy)-4-(trifluoromethyl) benzamide 2g

4-Dimethylaminopyridine (130 mg, 1.05 mmol) and 6-chloropyridazin-4-amine (1.51 g, 11.57 mmol, Pharmablock Sciences (Nanjing), Inc.) were dissolved in pyridine (40 mL), and the resulting solution was dried over molecular sieves. Compound **2f** (3.86 g, 10.51 mmol) was added, and the reaction solution was reacted for 16 hours under an argon atmosphere. The reaction solution was concentrated under reduced pressure, and the resulting residue was purified by silica gel column chromatography with eluent system B to obtain the title compound **2g** (1.3 g, yield: 39%).
MS m/z (ESI): 460.0 [M+1]

### Step 7

### 5-Chloro-2-(4-fluoro-2-methylphenoxy)-N-(6-oxo-1,6-dihydropyridazin-4-yl)-4-(trifluo romethyl)benzamide 2

Compound **2g** (1.3 g, 2.82 mmol) and potassium acetate (555 mg, 5.65 mmol) were added to acetic acid (20 mL), and the reaction solution was reacted at 130°C for 3 hours. The reaction solution was concentrated, and the resulting residue was purified by silica gel column chromatography with eluent system B to obtain the title compound **2** (800 mg, yield: 64%).
MS m/z (ESI): 442.0 [M+1]
1H NMR (400 MHz, DMSO-*d*₆) δ 12.83 (s, 1H), 11.03 (s, 1H), 8.07 (s, 1H), 7.86 (s, 1H), 7.05-7.25 (m,5H), 2.14 (s, 3H).

### Example 3

### 4,5-Dichloro-2-(4-fluoro-2-methylphenoxy)-N-(6-oxo-1,6-dihydropyridazin-4-yl)benza mide 3

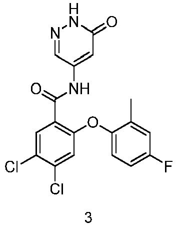

In accordance with the synthetic route in Example **1,** the starting compound 4-fluoro-2-methoxyphenol in Step 3 was replaced with compound 4-fluoro-2-methylphenol, accordingly, the title compound **3** (20 mg) was prepared.
MS m/z (ESI): 407.8 [M+1]
¹H NMR (400 MHz, DMSO-*d*₆) δ 12.82 (s, 1H), 10.92 (s, 1H), 7.99 (s, 1H), 7.90 (d, 1H), 7.21-7.18 (m, 2H), 7.08-7.05 (m, 2H), 6.99 (s, 1H), 2.14 (s, 3H).

### Example 4

### 4,5-Dichloro-2-(4-fluorophenoxy)-N-(6-oxo-1,6-dihydropyridazin-4-yl)benzamide 4

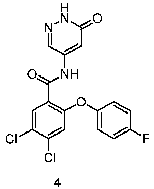

In accordance with the synthetic route in Example **1,** the starting compound 4-fluoro-2-methoxyphenol in Step 3 was replaced with compound 4-fluorophenol, accordingly, the title compound **4** (55 mg) was prepared.
MS m/z (ESI): 395.8 [M+1]
¹H NMR (400 MHz, DMSO-*d₆*) δ 12.81 (s, 1H), 10.89 (s, 1H), 7.98 (s, 1H), 7.88 (d, 1H), 7.26-7.22 (m, 2H), 7.19 (s, 1H), 7.16-7.13 (m, 3H).

### Example 5

### 2-(4-Fluoro-2-methylphenoxy)-N-(6-oxo-1,6-dihydropyridazin-4-yl)-4-(perfluoroethyl) benzamide 5

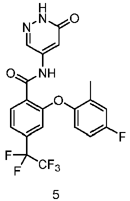

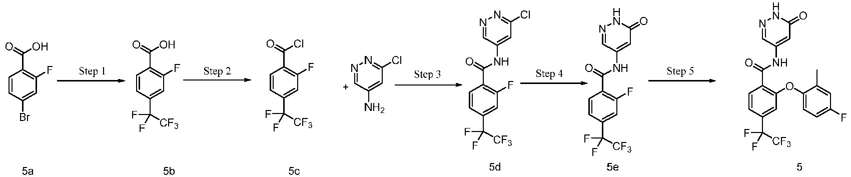

### Step 1

### 2-Fluoro-4-(perfluoroethyl)benzoic acid 5b

4-Bromo-2-fluorobenzoic acid **5a** (2.19 g, 10 mmol, J&K Scientific Ltd.) was dissolved in dimethyl sulfoxide (41 mL) under an argon atmosphere. Copper powder (6.36 g, 100 mmol) and pentafluoroiodoethane (17.22 g, 70 mmol, 8.24 mL, Sichuan Shang Fluoro Technology Co., Ltd.) were added, and the reaction solution was sealed in a tube and reacted at 120°C for 72 hours. The reaction solution was cooled, followed by the addition of 100 mL of water and 100 mL of ethyl acetate, stirred well and filtered. The filtrate was separated into two phases, and the aqueous phase was extracted with ethyl acetate (20 mL×3). The organic phases were combined, washed with saturated sodium chloride solution, dried over anhydrous sodium sulfate and filtered. The filtrate was concentrated, and the resulting residue was purified by silica gel column chromatography with eluent system B to obtain the title compound **5b** (2.4 g, yield: 93%).
MS m/z (ESI): 257.0 [M-1]

### Step 2

### 2-Fluoro-4-(perfluoroethyl)benzoyl chloride 5c

Compound **5b** (500 mg, 1.93mmol) was added to thionyl chloride (8.19 g, 68.8 mmol), and the reaction solution was reacted at 80°C for 16 hours. The reaction solution was concentrated to obtain the crude title compound **5c** (535 mg), which was used directly in the next step without purification.

### Step 3

### N-(6-Chloropyridazin-4-yl)-2-fluoro-4-(perfluoroethyl)benzamide 5d

The crude compound **5c** (535 mg, 1.93 mmol), 6-chloropyridazine-4-amine (150 mg, 1.16 mmol) and pyridine (916 mg, 11.58 mmol) were dissolved in dichloromethane (5 mL), and the reaction solution was reacted for 16 hours. 50 mL ethyl acetate was added, and the reaction solution was washed with saturated sodium chloride solution, dried over anhydrous sodium sulfate and filtered. The filtrate was concentrated, and the resulting residue was purified by silica gel column chromatography with eluent system B to obtain the title compound **5d** (664 mg, yield: 93%).
MS m/z (ESI): 370.0 [M+1]

### Step 4

### 2-Fluoro-N-(6-oxo-1,6-dihydropyridazin)-4-(perfluoroethyl)benzamide 5e

Compound **5d** (400 mg, 1.08 mmol) and potassium acetate (213 mg, 2.17 mmol) were added to acetic acid (5 mL), and the reaction solution was reacted at 130°C for 5 hours. The reaction solution was concentrated, and the resulting residue was purified by silica gel column chromatography with eluent system A to obtain the title compound **5e** (190 mg, yield: 50%).
MS m/z (ESI): 352.0 [M+1]

### Step 5

### 2-(4-Fluoro-2-methylphenoxy)-N-(6-oxo-1,6-dihydropyridazin-4-yl)-4-(perfluoroethyl) benzamide 5

Compound **5e** (190 mg, 0.54 mmol), 4-fluoro-2-methylphenol (103 mg, 0.82 mmol) and cesium carbonate (265 mg, 0.81 mmol) were added to *N,N*-dimethylformamide (3 mL), and the reaction solution was reacted at 100°C for 2 hours. The reaction solution was cooled, filtered and purified by preparative high performance liquid chromatography (Waters 2767-SQ Detecor2, eluent system: ammonium bicarbonate, water, acetonitrile) to obtain the title compound **5** (89 mg, yield: 36%).
MS m/z (ESI): 458.1 [M+1]
¹H NMR (400 MHz, CD₃OD): δ 8.05 (d, 1H), 7.92 (d, 1H), 7.52 (d, 1H), 7.49 (d, 1H), 7.12 (dd, 1H), 6.99-7.07 (m, 2H), 6.93 (s, 1H), 2.21(s, 3H).

### Example 6

### 2-(2-Methyl-4-(trifluoromethoxy)phenoxy)-N-(6-oxo-1,6-dihydropyridazin-4-yl)-4-(trifl uoromethyl)benzamide 6

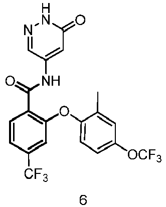

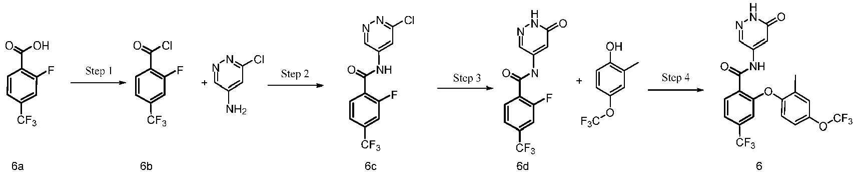

### Step 1

### 2-Fluoro-4-(trifluoromethyl)benzoyl chloride 6b

2-Fluoro-4-(trifluoromethyl)benzoic acid **6a** (1.2 g, 5.76 mmol, Accela ChemBio (Shanghai) Inc.) was added to thionyl chloride (12 mL), and the reaction solution was reacted at 80°C for 16 hours. The reaction solution was concentrated to obtain the crude title compound **6b** (1.3 g), which was used directly in the next step without purification.

### Step 2

### N-(6-Chloropyridazin-4-yl)-2-fluoro-4-(trifluoromethyl)benzamide 6c

The crude compound **6b** (1.3 g, 5.74 mmol), 6-chloro-4-aminopyridazine (300 mg, 2.31 mmol) and pyridine (916 mg, 11.58 mmol) were dissolved in dichloromethane (5 mL), and the reaction solution was reacted for 16 hours. The reaction solution was concentrated, and the resulting residue was purified by silica gel column chromatography with eluent system B to obtain the title compound **6c** (475 mg, yield: 64%).
MS m/z (ESI): 320.0 [M+1]

### Step 3

### 2-Fluoro-N-(6-oxo-1,6-dihydropyridazin-4-yl)-4-(trifluoromethyl)benzamide 6d

Compound **6c** (475 mg, 1.49 mmol) and potassium acetate (292 mg, 2.98 mmol) were added to acetic acid (5 mL), and the reaction solution was reacted at 120°C for 16 hours. The reaction solution was concentrated, and the resulting residue was purified by silica gel column chromatography with eluent system A to obtain the title compound **6d** (300 mg, yield: 67%).
MS m/z (ESI): 302.0 [M+1]

### Step 4

### 2-(2-Methyl-4-(trifluoromethoxy)phenoxy)-N-(6-oxo-1,6-dihydropyridazin-4-yl)-4-(trifl uoromethyl)benzamide 6

Compound **6d** (100 mg, 0.33 mmol), 2-methyl-4-(trifluoromethoxy)phenol (78 mg, 0.4 mmol) and cesium carbonate (216 mg, 0.66 mmol) were added to *N,N*-dimethylformamide (3 mL), and the reaction solution was reacted at 100°C for 8 hours. The reaction solution was cooled and filtered. The filtrate was concentrated, and the resulting residue was purified by silica gel column chromatography with eluent system A to obtain the title compound **6** (60 mg, yield: 38%).
MS m/z (ESI):473.8 [M+1]
¹H NMR (400 MHz, CD₃OD): δ 8.05 (d, 1H), 7.92 (d, 1H), 7.52 (d, 1H), 7.49 (d, 1H), 7.12 (dd, 1H), 6.99-7.07 (m, 2H), 6.93 (s, 1H), 2.29 (s, 3H).

### Example 7

### 2-(4-Fluoro-2-methylphenoxy)-N-(6-oxo-1,6-dihydropyridazin-4-yl)-5-(trifluoromethyl) benzamide 7

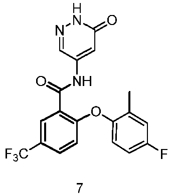

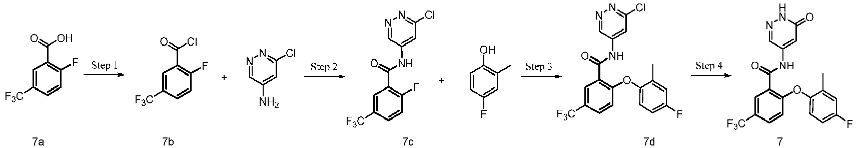

### Step 1

### 2-Fluoro-5-(trifluoromethyl)benzoyl chloride 7b

2-Fluoro-5-(trifluoromethyl)benzoic acid **7a** (382 mg, 1.83 mmol, Shanghai Bide Pharmatech Ltd.) was added to thionyl chloride (5 mL), and the reaction solution was reacted at 80°C for 16 hours. The reaction solution was concentrated to obtain the crude title compound **7b** (400 mg), which was used directly in the next step without purification.

### Step 2

### N-(6-Chloropyridazin-4-yl)-2-fluoro-5-(trifluoromethyl)benzamide 7c

The crude compound **7b** (393 mg, 1.73 mmol), 6-chloro-4-aminopyridazine (150 mg, 1.15 mmol) and pyridine (458 mg, 5.79 mmol) were dissolved in dichloromethane (5 mL), and the reaction solution was reacted for 24 hours. 50 mL ethyl acetate was added, and the reaction solution was washed with saturated sodium chloride solution, dried over anhydrous sodium sulfate and filtered. The filtrate was concentrated under reduced pressure, and the resulting residue was purified by silica gel column chromatography with eluent system B to obtain the title compound **7c** (90 mg, yield: 24%).
MS m/z (ESI):320.0 [M+1]

### Step 3

### N-(6-Chloropyridazin-4-yl)-2-(4-fluoro-2-methylphenoxy)-5-(trifluoromethyl)benzamid e 7d

Compound **7c** (90 mg, 0.28 mmol), 4-fluoro-2-methylphenol (43 mg, 0.34 mmol) and cesium carbonate (183 mg, 0.56 mmol) were added to *N,N*-dimethylformamide (3 mL), and the reaction solution was reacted at 100°C for 1 hour. The reaction solution was filtered, and the filtrate was concentrated. The resulting residue was purified by silica gel column chromatography with eluent system A to obtain the title compound **7d** (46 mg, yield: 37%).
MS m/z (ESI):426.1 [M+1]

### Step 4

### 2-(4-Fluoro-2-methylphenoxy)-N-(6-oxo-1,6-dihydropyridazin-4-yl)-5-(trifluoromethyl) benzamide 7

Compound **7d** (46 mg, 0.11 mmol) and potassium acetate (21 mg, 0.21 mmol) were added to acetic acid (1 mL), and the reaction solution was reacted at 130°C for 4 hours. The reaction solution was concentrated, and the resulting residue was purified by silica gel column chromatography with eluent system A to obtain the title compound **7** (16 mg, yield: 36%).
MS m/z (ESI):407.9 [M+1]
¹H NMR (400 MHz, CD₃OD): δ 8.08-8.07 (m, 2H), 7.77 (dd, 1H), 7.52 (d, 1H), 7.14-7.11 (m, 2H), 7.05-7.00 (m, 1H), 6.89 (d, 1H), 2.21(s, 3H).

### Example 8

### 2-(4-Fluoro-2-methoxyphenoxy)-N-(6-oxo-1,6-dihydropyridazin-4-yl)-4-(trifluorometh yl)benzamide 8

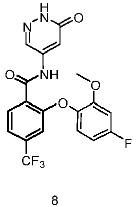

In accordance with the synthetic route in Example **6,** the starting compound 2-methyl-4-(trifluoromethoxy)phenol in Step 3 was replaced with compound 4-fluoro-2-methoxyphenol, accordingly, the title compound **8** (20 mg) was prepared.
MS m/z (ESI):424.1 [M+1]
¹H NMR (400 MHz, CD₃OD): δ 8.07 (d, 1H), 7.94 (d, 1H), 7.53-7.49 (m, 2H), 7.26 (q, 1H), 7.03 (dd, 1H), 6.97 (s, 1H), 6.83-6.79 (m, 1H), 2.89 (s, 3H).

### Example 9

### 2-(4-Fluoro-2-methylphenoxy)-N-(6-oxo-1,6-dihydropyridazin-4-yl)-4-(trifluoromethyl) benzamide 9

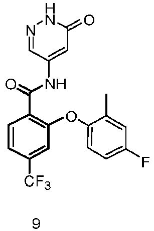

In accordance with the synthetic route in Example **6,** the starting compound 2-methyl-4-(trifluoromethoxy)phenol in Step 3 was replaced with compound 4-fluoro-2-methylphenol, accordingly, the title compound **9** (65 mg) was prepared.
MS m/z (ESI): 408.1 [M+1]
¹H NMR (400 MHz, CD₃OD): δ 8.05 (d, 1H), 7.90 (d, 1H), 7.54 (d, 1H), 7.49 (d, 1H), 7.11 (dd, 1H), 6.99-7.07 (m, 2H), 6.97 (s, 1H), 2.22 (s,3H).

### Example 10

### N-(6-Oxo-1,6-dihydropyridazin-4-yl)-2-(4-(trifluoromethoxy)phenoxy)-4-(trifluorometh yl)benzamide 10

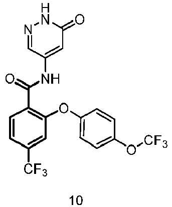

In accordance with the synthetic route in Example **6,** the starting compound 2-methyl-4-(trifluoromethoxy)phenol in Step 3 was replaced with compound 4-trifluoromethoxyphenol, accordingly, the title compound **10** (18 mg) was prepared.
MS m/z (ESI):460.0 [M+1]
¹H NMR (400 MHz, CD₃OD): δ 8.03 (d, 1H), 7.93 (d, 1H), 7.65-7.63 (m, 1H), 7.43 (d, 1H), 7.35-7.32 (m, 3H), 7.19-7.16 (m, 2H).

### Example 11

### 5-Chloro-2-(4-fluoro-2-methoxyphenoxy)-N-(6-oxo-1,6-dihydropyridazin-4-yl)-4-(triflu oromethyl)benzamide 11

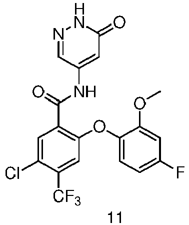

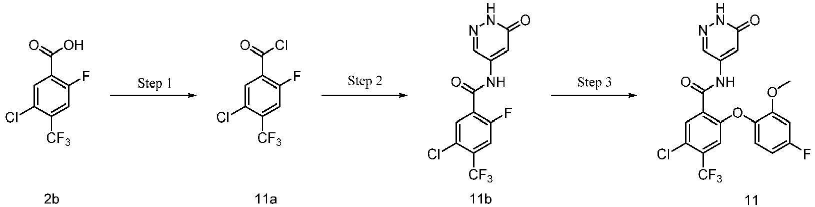

### Step 1

### 5-Chloro-2-fluoro-4-(trifluoromethyl)benzoyl chloride 11a

Compound **2b** (5.00 g, 20.6 mmol) was added to 15 mL of thionyl chloride, and the reaction solution was reacted at 80°C for 2 hours. The reaction solution was concentrated under reduced pressure to obtain the crude title compound **11a** (5.38 g), which was used directly in the next step without purification.

### Step 2

### 5-Chloro-2-fluoro-N-(6-oxo-1,6-dihydropyridazin-4-yl)-4-(trifluoromethyl)benzamide 11b

5-Aminopyridazin-3-one (3.06 g, 24.8 mmol, Shanghai Medicilon Inc.) was dissolved in 40 mL of *N*-methylpyrrolidone. The resulting solution was cooled to 0°C, and sodium hydride (2.06 g, 51.5 mmol, purity: 60%) was slowly add in batches. The reaction solution was stirred at 0°C for 30 minutes. Compound **11a** (5.38 g, 20.6 mmol) was dissolved in 3 mL of *N-*methylpyrrolidone, and the resulting solution was slowly added dropwise to the above reaction solution, which was then stirred at room temperature overnight. The reaction solution containing the title compound **11b** was used directly in the next step without purification.

### Step 3

### 5-Chloro-2-(4-fluoro-2-methoxyphenoxy)-N-(6-oxo-1,6-dihydropyridazin-4-yl)-4-(triflu oromethyl)benzamide 11c

4-Fluoro-2-methoxyphenol (2.34 g, 16.5 mmol, Tokyo Chemical Industry (Shanghai) Co., Ltd.) and cesium carbonate (6.71 g, 20.6 mmol, Accela ChemBio (Shanghai) Inc.) were added directly to the reaction solution containing compound **11b.** The reaction solution was reacted at 60°C overnight, and then cooled to room temperature. Ethyl acetate (250 mL) was added, and the reaction solution was washed with water (100 mL×3). The organic phase was dried over anhydrous sodium sulfate and filtered. The filtrate was concentrated under reduced pressure, and the resulting residue was purified by silica gel column chromatography with eluent system B to obtain the title compound **11c** (3.0 g, yield: 32%).
MS m/z (ESI):458.1 [M+1]
¹H NMR (400 MHz, DMSO-*d*₆): δ 12.87 (s, 1H), 11.03 (s, 1H), 8.05 (s, 1H), 7.92 (s, 1H), 7.27 (dd, 1H), 7.22 (s, 1H), 7.15 (dd, 1H), 7.00 (s, 1H), 6.87-6.82 (m, 1H), 3.71 (s, 3H).

### Example 12

### 5-Chloro-2-(2-cyclopropoxy-4-fluorophenoxy)-N-(6-oxo-1,6-dihydropyridazin-4-yl)-4-( trifluoromethyl)benzamide 12

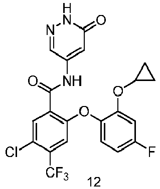

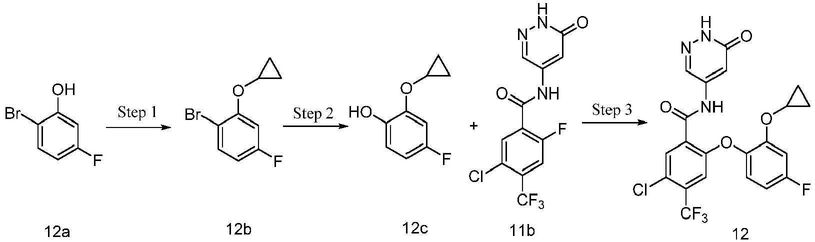

### Step 1

### 1-Bromo-2-cyclopropoxy-4-fluorobenzene 12b

2-Bromo-5-fluorophenol **12a** (2 g, 10.5 mmol, Accela ChemBio (Shanghai) Inc.), cyclopropyl bromide (5 g, 41.3 mmol, Shanghai Titan Scientific Co., Ltd.), cesium carbonate (7 g, 21.5 mmol, Accela ChemBio (Shanghai) Inc.) and potassium iodide (180 mg, 1.1 mmol) were added to *N,N*-dimethylformamide (10 mL). The reaction solution was reacted in a microwave reactor at 130°C for 1.5 hours, and then cooled to room temperature. Ethyl acetate (20 mL) was added, and the reaction solution was washed with water (20 mL×3). The organic phase was dried over anhydrous sodium sulfate and filtered. The filtrate was concentrated under reduced pressure, and the resulting residue was purified by silica gel column chromatography with eluent system A to obtain the title compound **12b** (3.0 g, yield: 70%).

### Step 2

### 2-Cyclopropoxy-4-fluorophenol 12c

Compound **12b** (1.85 g, 8 mmol) and triisopropyl borate (1.96 g, 10.4 mmol, Shanghai Titan Scientific Co., Ltd.) were added to 20 mL of tetrahydrofuran. The air in the reaction flask was replaced with argon. The reaction solution was cooled to -78°C, then *n*-butyl lithium (1.6 M, 7.5 mL, 12 mmol) was slowly added dropwise within 20 minutes. The reaction solution was naturally warmed up to room temperature and stirred overnight. The reaction solution was cooled to 0°C in an ice bath. 50 mL of methanol was added, and hydrogen peroxide (30 wt%, 11 mL) and 10% sodium hydroxide solution (50 mL) were added dropwise. After completion of the addition, 400 mL of saturated sodium chloride solution was added, and the reaction solution was extract with ethyl acetate (200 mL×3). The organic phase was washed with saturated sodium bicarbonate solution (150 mL), dried over anhydrous sodium sulfate and filtered. The filtrate was concentrated under reduced pressure, and the resulting residue was purified by silica gel column chromatography with eluent system B to obtain the title compound **12b** (1.0 g, yield: 74%).

### Step 3

### 5-Chloro-2-(2-cyclopropoxy-4-fluorophenoxy)-N-(6-oxo-1,6-dihydropyridazin-4-yl)-4-( trifluoromethyl)benzamide 12

Compound **11b** (700 mg, 2.1 mmol), compound **12c** (300 mg, 1.78 mmol) and cesium carbonate (700 mg, 2.1 mmol, Accela ChemBio (Shanghai) Inc.) were added to 7 mL of *N-*methylpyrrolidone. The reaction solution was reacted at 80°C for 1 hour, and then cooled to room temperature. Ethyl acetate (20 mL) was added, and the reaction solution was washed with water (10 mL×3). The organic phase was dried over anhydrous sodium sulfate and filtered. The filtrate was concentrated under reduced pressure, and the resulting residue was purified by silica gel column chromatography with eluent system B to obtain the title compound **12** (300 mg, yield: 30%).
MS m/z (ESI):484.0 [M+1]
¹H NMR (400 MHz, DMSO-*d*₆): δ 12.84 (s, 1H), 10.99 (s, 1H), 8.01 (s, 1H), 7.87 (s, 1H), 7.30-7.19 (m, 3H), 6.96 (s, 1H), 6.85-6.83 (m, 1H), 3.90-3.88 (m, 3H), 0.74-0.70 (m, 1H), 0.40-0.38 (m, 1H).

### Example 13

### 5-Chloro-2-(4-fluoro-2-methylbenzyl)-N-(6-oxo-1,6-dihydropyridazin-4-yl)-4-(trifluoro methyl)benzamide 13

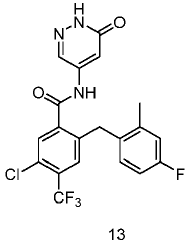

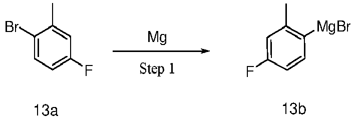

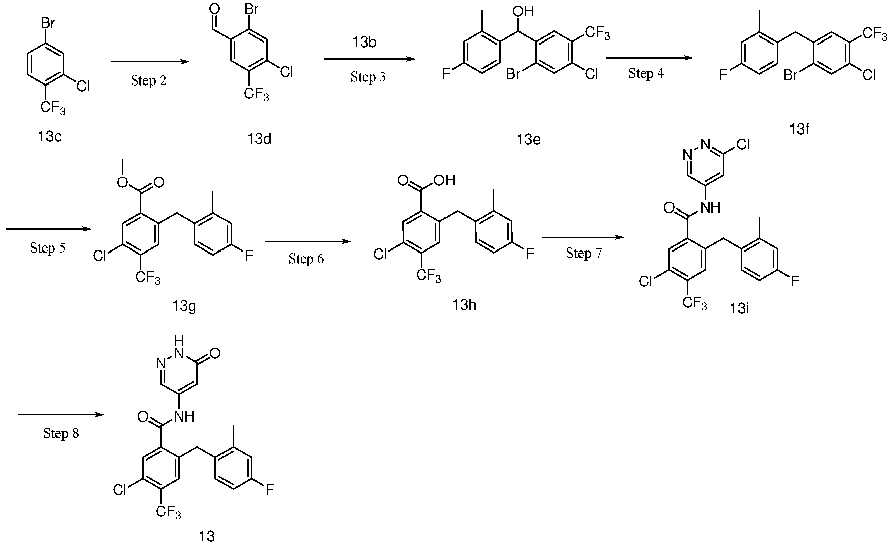

### Step 1

### (4-Fluoro-2-methylphenyl)magnesium bromide 13b

A polished magnesium bar (760 mg, 31.7 mmol, Shanghai Sinopharm Chemical Reagent Co., Ltd.) was cut into small pieces and add to tetrahydrofuran (80 mL) under an argon atmosphere. Trimethylchlorosilane (345 mg, 3.17 mmol, Accela ChemBio (Shanghai) Inc.) was added dropwise at room temperature, followed by the addition of 1-bromo-4-fluoro-2-methylbenzene **13a** (1.5 g, 7.9 mmol, Accela ChemBio (Shanghai) Inc.). After the reaction was initiated by heating, additional compound **13a** (4.5 g, 23.7 mmol, Accela ChemBio (Shanghai) Inc.) was added. The reaction solution was heated to 45°C and reacted for 1 hour. The magnesium bar disappeared completely, and a grey homogeneous liquid was obtained, i.e., a solution of the title compound **13b** (0.4 M, 80 mL), which was used directly in the next step without purification.

### Step 2

### 2-Bromo-4-chloro-5-(trifluoromethyl)benzaldehyde 13d

Tetrahydrofuran (100 mL) and lithium hexamethyldisilazide (1 M, 120 mL, 120 mmol, Titan Scientific Co., Ltd.) were cooled to -78°C under an argon atmosphere. 4-Bromo-2-chloro-1-(trifluoromethyl)benzene **13c** (25 g, 96.36 mmol, Accela ChemBio (Shanghai) Inc.) was added dropwise, and the reaction solution was kept at this low temperature and reacted for 2 hours. *N,N*-Dimethylformamide (14.1 g, 192.9 mmol, J&K Scientific Ltd.) was added dropwise, and the reaction solution was gradually warmed up to room temperature and reacted for 16 hours. Water was added, and the reaction solution was extracted with ethyl acetate (50 mL×3). The organic phase was dried over anhydrous sodium sulfate and filtered. The filtrate was concentrated under reduced pressure, and the resulting residue was purified by silica gel column chromatography with eluent system B to obtain the title compound **13d** (3.14 g, yield: 28%).
¹H NMR (400 MHz, CDCl₃) δ 10.32 (s, 1H), 8.23 (s, 1H),7.87 (s, 1H).

### Step 3

### (2-Bromo-4-chloro-5-(trifluoromethyl)phenyl)(4-fluoro-2-methylphenyl)methanol 13e

Compound **13d** (900 mg, 3.13 mmol) was dissolved in tetrahydrofuran (10 mL). A freshly prepared solution of compound **13b** (7.97 mmol, 19.92 mL) was added dropwise, and the reaction solution was reacted at room temperature for 1 hour. Saturated ammonium chloride solution was added, and the reaction solution was extracted with ethyl acetate (10 mL×3). The organic phase was dried over anhydrous sodium sulfate and filtered. The filtrate was concentrated under reduced pressure, and the resulting residue was purified by silica gel column chromatography with eluent system B to obtain the title compound **13e** (900 mg, yield: 72%).
¹HNMR (400 MHz, CD₃OD) δ 8.04 (s,1H),7.93 (s,1H), 7.0-7.03 (m,1H), 6.87-6.90 (m, 2H), 6.17 (s, 1H), 2.5(s, 3H).

### Step 4

### 1-Bromo-5-chloro-2-(4-fluoro-2-methylbenzyl)-4-(trifluoromethyl)benzene 13f

Compound **13e** (4 g, 10.1 mmol) was dissolved in dichloromethane (50 mL). The resulting solution was cooled to 0°C, trifluoroacetic acid (10 mL, Titan Scientific Co., Ltd.) was added, and then triethylsilane (6 mL, Accela ChemBio (Shanghai) Inc.) was added dropwise. The reaction solution was reacted at 0°C for 1 hour. Water was added, and the reaction solution was extracted with ethyl acetate (10 mL×3). The organic phase was dried over anhydrous sodium sulfate and filtered. The filtrate was concentrated under reduced pressure, and the resulting residue was purified by silica gel column chromatography with eluent system B to obtain the title compound **13f** (3.2 g, yield: 83%).

### Step 5

### Methyl 5-chloro-2-(4-fluoro-2-methylbenzyl)-4-(trifluoromethyl)benzoate 13g

Compound **13f** (3.3 g, 8.64 mmol) was dissolved in methanol (60 mL). Palladium acetate (388.31 mg, 1.73 mmol, J&K Scientific Ltd.), 1,1'-bis(diphenyphosphino)ferrocene (960 mg, 1.73 mmol, Accela ChemBio (Shanghai) Inc.) and triethylamine (2.63 g, 25.94 mmol, Shanghai Sinopharm Chemical Reagent Co., Ltd.) were added. The reaction system was connected to a carbon monoxide balloon, and reacted at 60°C for 16 hours. The reaction solution was filtered through diatomaceous earth. The filtrate was concentrated under reduced pressure, and the resulting residue was purified by silica gel column chromatography with eluent system B to obtain the title compound **13g** (2.2 g, yield: 71%).
MS m/z (ESI): 359.1[M-1]

### Step 6

### 5-Chloro-2-(4-fluoro-2-methylbenzyl)-4-(trifluoromethyl)benzoic acid 13h

Compound **13g** (2.2g, 6.1 mmol) was dissolved in methanol (40 mL) and water (20 mL), followed by the addition of sodium hydroxide solution (5 M, 6 mL, 30 mmol). The reaction solution was warmed up to 40°C and reacted for 3 hours. The reaction solution was cooled, adjusted to pH 2 with 4 M hydrochloric acid, and extracted with dichloromethane (10 mL×3). The organic phase was dried over anhydrous sodium sulfate and filtered. The filtrate was concentrated under reduced pressure to obtain the crude title compound **13h** (2.1 g), which was used directly in the next step without purification.
MS m/z (ESI): 345.1[M-1]

### Step 7

### 5-Chloro-N-(6-chloropyridazin-4-yl)-2-(4-fluoro-2-methylbenzyl)-4-(trifluoromethyl)be nzamide 13i

Compound **13h** (300 mg, 0.87 mmol) was dissolved in dichloromethane (15 mL), followed by the addition of one drop of *N,N*-dimethylformamide. Thionyl chloride (2 mL, Shanghai Sinopharm Chemical Reagent Co., Ltd.) was added dropwise in an ice bath. The reaction solution was reacted at room temperature overnight, and then concentrated under reduced pressure. The resulting residue was dissolved in pyridine (2 mL), followed by the addition of 4-amino-6-chloropyridazine (168 mg, 1.3 mmol, Accela ChemBio (Shanghai) Inc.). The reaction solution was reacted at room temperature overnight, and then concentrated under reduced pressure. Water (20 mL) was added, and the resulting solution was extracted with dichloromethane (10 mL×3). The organic phase was dried over anhydrous sodium sulfate and filtered. The filtrate was concentrated under reduced pressure to obtain the crude compound **13i** (260 mg), which was used directly in the next step without purification.
MS m/z (ESI): 458.1[M+1]

### Step 8

### 5-Chloro-2-(4-fluoro-2-methylbenzyl)-N-(6-oxo-1,6-dihydropyridazin-4-yl)-4-(trifluoro methyl)benzamide 13

Compound **13i** (260 mg, 0.57 mmol) was dissolved in acetic acid (5 mL), followed by the addition of potassium acetate (112 mg, 1.14 mmol), and the reaction solution was reacted at 130°C overnight. The reaction solution was concentrated under reduced pressure, and the resulting residue was purified by preparative high performance liquid chromatography (Waters 2767-SQ Detecor2, eluent system: ammonium bicarbonate, water, acetonitrile) to obtain the title compound **13** (10 mg, yield of two steps: 2.6%).
MS m/z (ESI): 440.1 [M+1]
¹H NMR (400 MHz, DMSO-*d*₆) δ 7.90-7.91 (m, 1H), 7.81 (m, 1H), 7.59 (m, 1H), 7.39 (m, 1H), 6.95-6.97 (m, 1H), 6.85-6.88 (m, 1H), 6.78-6.79 (m, 1H), 4.63 (m, 1H), 4.19 (s, 2H), 2.20 (s, 3H).

### Example 14

### 5-Chloro-2-(2-fluoro-4-(tirfluoromethoxy)phenoxy)-N-(6-oxo-1,6-dihydropyridazin-4-y l)-4-(trifluoromethyl)benzamide 14

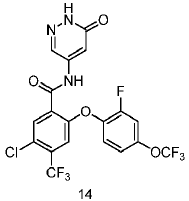

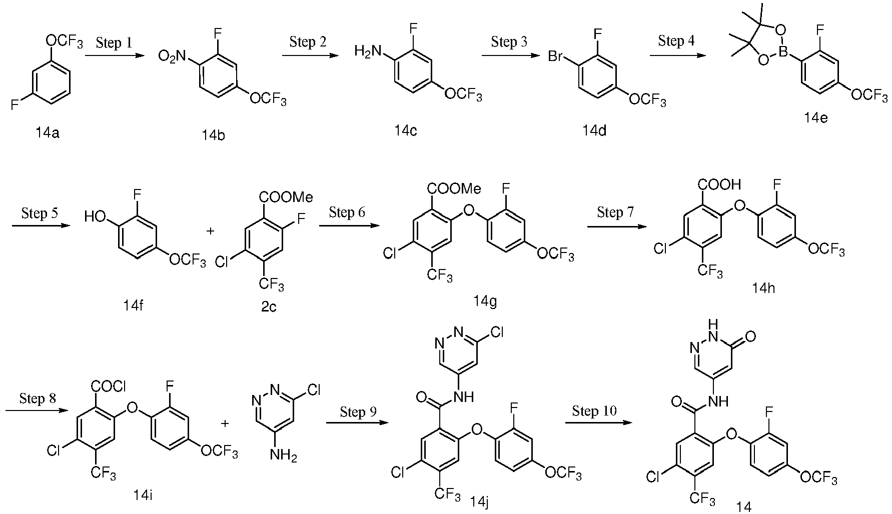

### Step 1

### 2-Fluoro-1-nitro-4-(trifluoromethoxy)benzene 14b

1-Fluoro-3-(trifluoromethoxy)benzene **14a** (5 g, 27.76 mmol, Accela ChemBio (Shanghai) Inc.) was dissolved in sulfuric acid (20 mL), and the resulting solution was cooled in an ice bath. Potassium nitrate (7 g, 69.2 mmol) was added in batches, and the reaction solution was naturally warmed up to room temperature and reacted overnight. The reaction solution was poured into ice water, stirred for 30 minutes, extracted with ethyl acetate three times, dried over sodium sulfate, and concentrated to dryness by rotary evaporation to obtain a crude mixture (5.8 g) containing compound **14b.**

### Step 2

### 2-Fluoro-4-(trifluoromethoxy)aniline 14c

The crude mixture (5.8 g, 25.7 mmol) containing compound **14b** was dissolved in methanol (80 mL). The resulting solution was purged with nitrogen, and Pd/C catalyst (1.54 g, 14.47 mmol) was added. The resulting solution was purged with hydrogen three times, and the hydrogenation reaction was carried out at room temperature overnight. The reaction solution was filtered, and the filtrate wasconcentrated. The resulting residue was purified by silica gel column chromatography with eluent system A to obtain a crude mixture (3.5 g) containing the title compound **14c.**

### Step 3

### 1-Bromo-2-fluoro-4-(trifluoromethoxy)benzene 14d

The mixture (3.4 g, 17.4 mmol) containing compound **14c** was dissolved in acetonitrile (40 mL), followed by the addition of copper bromide (4.67 g, 20.9 mmol, Accela ChemBio (Shanghai) Inc.) and tert-butyl nitrite (2.16 g, 20.9 mmol). The reaction solution was reacted at 60°C for 0.5 hour, and then filtered. The filtrate was concentrated under reduced pressure, and the resulting residue was purified by silica gel column chromatography with eluent system A to obtain a mixture (1.3 g) containing the title compound **14d.**

### Step 4

### 2-(2-Fluoro-4-(trifluoromethoxy)phenyl)-4,4,5,5-tetramethyl-1,3,2-dioxaborolane 14e

The mixture (3 g, 11.6 mmol) containing compound **14d** was dissolved in 1,4-dioxane (50 mL), followed by the addition of pinacol diborate (4.4 g, 17.3 mmol, Accela ChemBio (Shanghai) Inc.), [1,1'-bis(diphenylphosphino)ferrocene]dichloropalladium(II) (424 mg, 0.58 mmol) and potassium acetate (3.41 g, 34.7 mmol). The reaction solution was purged with argon three times, and reacted at 100°C overnight. The reaction solution was filtered through diatomaceous earth, and the filtrate was concentrated under reduced pressure. The resulting residue was purified by silica gel column chromatography with eluent system A to obtain a mixture (950 mg) containing the title compound **14e.**

### Step 5

### 2-Fluoro-4-(trifluoromethoxy)phenol 14f

The mixture (950 mg, 3.1 mmol) containing compound **14e** was dissolved in tetrahydrofuran (20 mL), followed by the addition of sodium hydroxide solution (621 mg, 15.5 mmol, 5 mL). The resulting solution was cooled in an ice bath. Hydrogen peroxide (3.1 mL) was added dropwise, and the reaction solution was reacted at room temperature overnight. The organic phase was washed with saturated sodium bicarbonate solution (50 mL), dried over anhydrous sodium sulfate and filtered. The filtrate was concentrated under reduced pressure, and the resulting residue was purified by silica gel column chromatography with eluent system B to obtain a mixture (220 mg) containing the title compound **14f.**

### Step 6

### Methyl 5-chloro-2-(2-fluoro-4-(trifluoromethoxy)phenoxy)-4-(trifluoromethyl)benzoate 14g

Compound **2c** (300 mg, 1.2 mmol) and the mixture (229 mg, 1.2 mmol) containing compound **14f** were added to *N,N*-dimethylformamide (5 mL), followed by the addition of cesium carbonate (571 mg, 1.8 mmol). The reaction solution was reacted at 100°C for 1 hour, cooled and filtered. The filtrate was concentrated under reduced pressure, and the resulting residue was purified by silica gel column chromatography with eluent system B to obtain a mixture (440 mg) containing the title compound **14g.**

### Step 7

### 5-Chloro-2-(2-fluoro-4-(trifluoromethoxy)phenoxy)-4-(trifluoromethyl)benzoic acid 14h

Compound **14g** (400 mg, 0.92 mmol) was dissolved in tetrahydrofuran (5 mL), followed by the addition of lithium hydroxide solution (194 mg, 4.6 mmol, 1 mL). The reaction solution was reacted at room temperature for 4 hours, concentrated under reduced pressure, followed by the addition of water (5 mL). The resulting solution was adjusted to pH = 4 with diluted hydrochloric acid, and extracted with ethyl acetate three times (20 mL×3). The organic phase was dried over anhydrous sodium sulfate and filtered. The filtrate was concentrated under reduced pressure to obtain a mixture (340 mg) containing the title compound **14h,** which was used directly in the next step.

### Step 8

### 5-Chloro-2-(2-fluoro-4-(trifluoromethoxy)phenoxy)-4-(trifluoromethyl)benzoyl chloride 14i

Compound **14h** (80 mg, 0.19 mmol) was dissolved in thionyl chloride (2 mL), and the reaction solution was reacted at 80°C for 2 hours. The reaction solution was concentrated under reduced pressure to obtain a mixture (80 mg) containing the title compound **14i,** which was used directly in the next step.

### Step 9

### 5-Chloro-N-(6-chloropyridazin-4-yl)-2-(2-fluoro-4-(trifluoromethoxy)phenoxy)-4-(trifl uoromethyl)benzamide 14j

4-Amino-6-chloropyridazine (31 mg, 0.24 mmol, Accela ChemBio (Shanghai) Inc.) was dissolved in tetrahydrofuran (3 mL), and the resulting solution was cooled in an ice bath. Sodium hydride (13 mg, 0.34 mmol, purity: 60%) was added, and the reaction solution was reacted for 30 minutes. A solution (2 mL) of compound **14i** in tetrahydrofuran was added dropwise, and the reaction solution was reacted at room temperature overnight under an argon atmosphere. Water (10 mL) and ethyl acetate (20 mL) were added, and the organic phase was dried over anhydrous sodium sulfate and concentrated under reduced pressure. The resulting residue was purified by silica gel column chromatography with eluent system B to obtain a mixture (65 mg) containing the title compound **14j.**

### Step 10

### 5-Chloro-2-(2-fluoro-4-(trifluoromethoxy)phenoxy)-N-(6-oxo-1,6-dihydropyirdazin-4-y l)-4-(trifluoromethyl)benzamide 14

The mixture (65 mg, 0.12 mmol) containing compound **14j** was dissolved in acetic acid (3 mL), followed by the addition of potassium acetate (60 mg, 0.61 mmol), and the reaction solution was reacted at 130°C for 2 hours. The reaction solution was concentrated under reduced pressure, and the resulting residue was purified by preparative high performance liquid chromatography (Waters 2767-SQ Detecor2, eluent system: ammonium bicarbonate, water, acetonitrile) to obtain the title compound **14** (8 mg, yield: 11%).
MS m/z (ESI): 512.0 [M+1]
H NMR (400 MHz, CD₃OD): *δ* 8.02 (d, 1H), 8.00 (s, 1H), 7.43 (d, 1H), 7.40 (s, 1H), 7.37-7.27 (m, 2H), 7.19 (d, 1H).

### Example 15

### 5-Chloro-2-(4-fluoro-2-(trifluoromethoxy)phenoxy)-N-(6-oxo-1,6-dihydropyridazin-4-y l)-4-(trifluoromethyl)benzamide 15

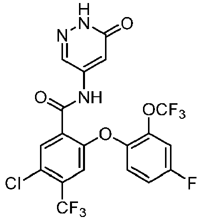

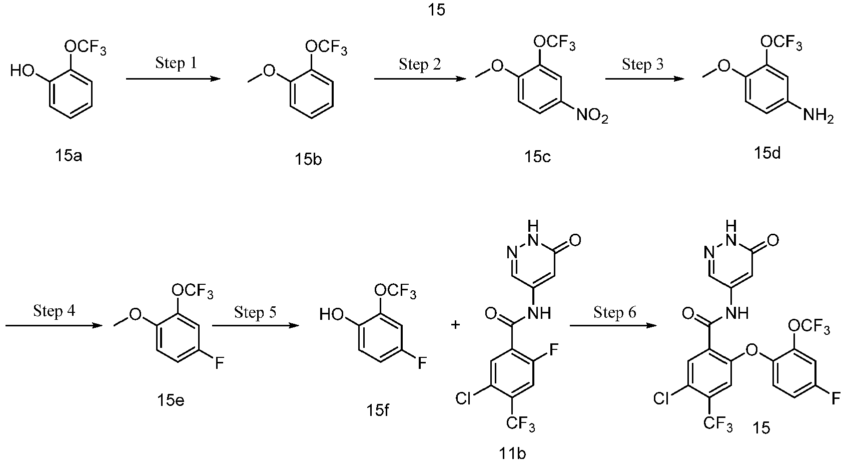

### Step 1

### 1-Methoxy-2-(trifluoromethoxy)benzene 15b

2-(Trifluoromethoxy)phenol **15a** (5 g, 28.1 mmol, Accela ChemBio (Shanghai) Inc.) was dissolved in *N,N*-dimethylformamide (50 mL), followed by the addition of methyl iodide (4.78 g, 33.7 mmol) and potassium carbonate (7.75 g, 56.1594 mmol). The reaction solution was reacted at 80°C for 1 hour. Ethyl acetate and water were added to the reaction solution, and the aqueous phase was extracted with ethyl acetate three times. The organic phases were combined, washed with water four times, dried over sodium sulfate, and concentrated to dryness by rotary evaporation to obtain the title compound **15b** (5.1 g, yield: 94%).

### Step 2

### 1 -Methoxy-4-nitro-2-(trifluoromethoxy)benzene 15c

Sodium nitrate (2.26 g, 26.6 mmol) was dissolved in trifluoroacetic acid (50 mL). The resulting solution was cooled in an ice bath, and a solution (10 mL) of compound **15b** (5.1 g, 26.5 mmol) in trifluoroacetic acid was added. The reaction solution was reacted in the ice bath for 1 hour, and at room temperature for 3 hours. Water (50 mL) and ethyl acetate (50 mL) were added to the reaction solution. The organic phase was washed with saturated sodium bicarbonate solution to be neutral, dried over anhydrous sodium sulfate and concentrated under reduced pressure to obtain the title compound **15c** (5.3 g, yield: 84%).

### Step 3

### 4-Methoxy-3 -(trifluoromethoxy)aniline 15d

Compound **15c** (5.3 g, 22.3 mmol) was dissolved in methanol (100 mL), followed by the addition of concentrated hydrochloric acid (0.5 mL), and Pd/C catalyst (500 mg). The resulting solution was purged with hydrogen three times, and the hydrogenation reaction was carried out at room temperature for 4 hours. The reaction solution was filtered through diatomaceous earth, and the filtrate was concentrated under reduced pressure. The resulting residue was purified by silica gel column chromatography with eluent system A to obtain the title compound **15d** (3.1 g, yield: 67%).

### Step 4

### 4-Fluoro-1-methoxy-2-(trifluoromethoxy)benzene 15e

Compound **15d** (800 mg, 3.9 mmol) was added to a mixed solution of water (15 mL), fluoroboric acid (1.51 g, 7.0 mmol, purity: 40%) and hydrochloric acid (687 mg, 7.0 mmol). The resulting solution was cooled in an ice bath. Sodium nitrite solution (280 mg, 4.06 mmol, 2 mL) was added dropwise, and the reaction solution was reacted in the ice bath for 2 hours. The reaction solution was filtered, and the filter cake was dried. The solid was heated to 130°C under a nitrogen atmosphere, gradually melted and turned red, and reacted for 1 hour. After the reaction system was cooled, the resulting product was dissolved in dichloromethane, dried over sodium sulfate, and concentrated under reduced pressure to obtain the title compound **15e** (600 mg, yield: 74%).

### Step 5

### 4-Fluoro-2-(trifluoromethoxy)phenol 15f

Compound **15e** (300 mg, 1.4 mmol) was dissolved in dichloromethane (5 mL), and the resulting solution was cooled in an ice bath. Boron tribromide solution (1 M, 7 mL) was added, and the reaction solution was naturally warmed up to room temperature and reacted overnight. The reaction solution was added dropwise to methanol, and concentrated under reduced pressure. The resulting residue was purified by silica gel column chromatography with eluent system A to obtain the title compound **15f** (100 mg, yield: 36%).

### Step 6

### 5-Chloro-2-(4-fluoro-2-(trifluoromethoxy)phenoxy)-N-(6-oxo-1,6-dihydropyridazin-4-y l)-4-(trifluoromethyl)benzamide 15

Compound **15f** (300 mg, 0.89 mmol) was added to *N*-methylpyrrolidone (3 mL), followed by the addition of compound **11b** (140 mg, 0.71 mmol) and cesium carbonate (291 mg, 0.9 mmol). The reaction solution was reacted at 80°C for 1 hour. Water (10 mL) and ethyl acetate (20 mL) were added to the reaction solution, and the organic phase was dried over anhydrous sodium sulfate and concentrated under reduced pressure. The resulting residue was purified by preparative high performance liquid chromatography (Waters 2767-SQ Detecor2, eluent system: ammonium bicarbonate, water, acetonitrile) to obtain the title compound **15** (40 mg, yield: 11%).
MS m/z (ESI): 512.0 [M+1]
H NMR (400 MHz, CD₃OD): *δ* 8.03 (d, 1H), 8.01 (s, 1H), 7.46 (d, 1H), 7.37-7.35 (m, 1H), 7.32-7.22 (m, 3H).

### Example 16

### 2-(4-Fluoro-2-methoxyphenoxy)-N-(6-oxo-1,6-dihydropyridazin-4-yl)-5-(trifluorometh yl)benzamide 16

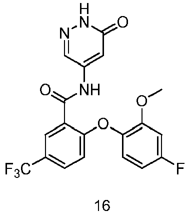

In accordance with the synthetic route in Example **2,** the starting compound 5-chloro-2-fluoro-4-(trifluoromethyl)benzoic acid in Step 2 was replaced with compound 2-fluoro-5-(trifluoromethyl)benzoic acid, and the starting compound 4-fluoro-2-methyl-phenol in Step 3 was replaced with 4-fluoro-2-methoxy-phenol, accordingly, the title compound **16** (27 mg) was prepared.
MS m/z (ESI): 424.1[M+1]
¹H NMR (400 MHz, DMSO-*d*₆) *δ* 12.81 (s, 1H), 10.90 (s, 1H), 7.95 (d, 2H), 7.76 (d, 1H), 7.21-7.33 (m,2H), 7.15 (d, 1H), 6.72-6.90 (m, 2H), 3.71 (s, 3H).

### Example 17

### 5-Chloro-2-((4-fluoro-2-methoxyphenyl)thio)-N-(6-oxo-1,6-dihydropyridazin-4-yl)-4-(t rifluoromethyl)benzamide 17

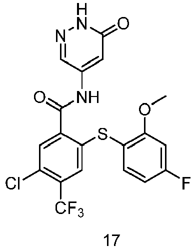

In accordance with the synthetic route in Example **2,** the starting compound 2-methyl-4-fluorophenol in Step 3 was replaced with 2-methoxybenzenethiol, accordingly, the title compound **17** (35 mg) was prepared.
MS m/z (ESI): 474.0 [M+1]
¹H NMR (400 MHz, DMSO-*d*₆) δ 12.90 (s, 1H), 11.06 (s, 1H), 8.12 (s, 1H), 7.97 (s, 1H), 7.59-7.55 (m, 1H), 7.25 (s, 1H), 7.16-7.13 (m, 2H), 6.93-6.89 (m, 1H), 3.74 (s, 3H).

### Example 18

### 4-Chloro-5-fluoro-2-(4-fluoro-2-methoxyphenoxy)-N-(6-oxo-1,6-dihydropyridazin-4-yl )benzamide 18

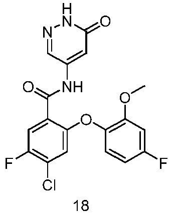

In accordance with the synthetic route in Example **1,** the starting compound 4,5-dichloro-2-fluorobenzoic acid in Step 1 was replaced with compound 4-chloro-2,5-difluorobenzoic acid, accordingly, the title compound **18** (22 mg) was prepared.
MS m/z (ESI): 408.0 [M+1]
¹H NMR (400 MHz, DMSO-*d*₆) *δ* 12.81 (s, 1H), 10.83 (s, 1H), 7.90-7.89 (d, 1H), 7.77-7.75 (d, 1H),7.19-7.16 (m,2H), 7.10-7.06 (m,1H), 6.90-6.81 (m,1H), 6.81-6.77 (m, 1H), 3.71 (s, 3H).

### Example 19

### 4-Chloro-5-fluoro-2-(4-fluoro-2-methylphenoxy)-N-(6-oxo-1,6-dihydropyridazin-4-yl)b enzamide 19

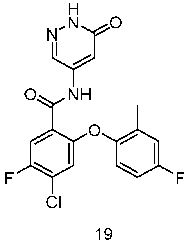

In accordance with the synthetic route in Example **1,** the starting compound 4,5-dichloro-2-fluorobenzoic acid in Step 1 was replaced with compound 4-chloro-2,5-difluorobenzoic acid, and the starting compound 2-methoxy-4-fluorophenol in Step 3 was replaced with 2-methyl-4-fluorophenol, accordingly, the title compound **19** (9 mg) was prepared as a white solid.
MS m/z (ESI): 392.1 [M+1]
¹H NMR (400 MHz, DMSO-*d*₆) *δ* 12.81 (s, 1H), 10.89 (s, 1H), 7.87-7.86 (d, 1H), 7.82-7.80 (d, 1H), 7.18-7.14 (m,2H),7.04-7.03 (m,2H), 7.00-6.93 (m,1H), 2.14 (s, 3H).

### Example 20

### 4-Chloro-2-(4-fluoro-2-methylphenoxy)-N-(6-oxo-1,6-dihydropyridazin-4-yl)benzamid

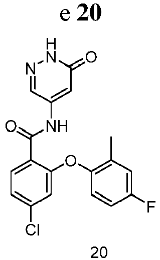

In accordance with the synthetic route in Example **2,** the starting compound 5-chloro-2-fluoro-4-(trifluoromethyl)benzoic acid in Step 2 was replaced with compound 4-chloro-2-fluorobenzoic acid, accordingly, the title compound **20** (40 mg) was prepared.
MS m/z (ESI): 374.1 [M+1]
1H NMR (400 MHz, DMSO-*d*₆) *δ* 12.79 (s, 1H), 10.83 (s, 1H), 7.89 (s, 1H), 7.67 (d, 1H), 7.30 (d,1H), 7.14-7.22 (m, 2H), 7.00-7.12 (m, 2H), 6.74 (s, 1H), 2.13 (s, 3H).

### Example 21

### 4-Chloro-2-(4-fluoro-2-methoxyphenoxy)-N-(6-oxo-1,6-dihydropyridazin-4-yl)benzami de 21

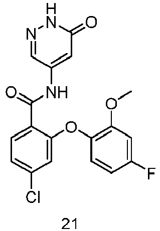

In accordance with the synthetic route in Example **2,** the starting compound 5-chloro-2-fluoro-4-(trifluoromethyl)benzoic acid in Step 2 was replaced with compound 4-chloro-2-fluorobenzoic acid, and the starting compound 2-methyl-4-fluorophenol in Step 3 was replaced with compound 2-methoxy-4-fluorophenol, accordingly, the title compound **21** (40 mg) was prepared.
MS m/z (ESI): 390.1 [M+1]
1H NMR (400 MHz, DMSO-*d*₆) *δ* 12.79 (s, 1H), 10.75 (s, 1H), 7.92 (s, 1H), 7.63 (d, 1H), 7.18-7.30 (m,3H),7.10 (dd,1H), 6.78-6.88 (m,1H), 6.64 (s, 1H), 3.72 (s, 3H).

### Example 22

### 2-(4-Fluoro-2-methoxyphenoxy)-N-(6-oxo-1,6-dihydropyridazin-4-yl)-5-(trifluorometh oxy)benzamide 22

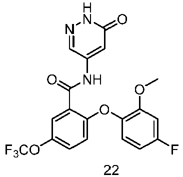

In accordance with the synthetic route in Example **1,** the starting compound 4,5-dichloro-2-fluorobenzoic acid in Step 1 was replaced with compound 2-fluoro-5-(trifluoromethoxy)benzoic acid, accordingly, the title compound **22** (5 mg) was prepared.
MS m/z (ESI): 440.1 [M+1]
¹H NMR (400 MHz, CD₃OD): *δ* 8.05 (d, 1H), 7.74 (d, 1H), 7.54 (d, 1H), 7.41-7.38 (m, 1H), 7.26 (q, 1H), 7.03-7.00 (m, 1H), 6.89 (d, 1H), 6.81-6.77 (m, 1H), 3.81 (s, 3H).

### Example 23

### 5-Chloro-2-(4-fluoro-2-methylphenoxy)-N-(6-oxo-1,6-dihydropyridazin-4-yl)benzamid e 23

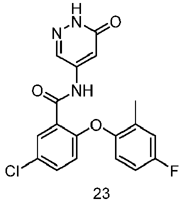

In accordance with the synthetic route in Example **1,** the starting compound 4,5-dichloro-2-fluorobenzoic acid in Step 1 was replaced with compound 5-chloro-2-fluorobenzoic acid, accordingly, the title compound **23** (14 mg) was prepared.
MS m/z (ESI): 374.1 [M+1]
¹H NMR (400 MHz, CD₃OD): *δ* 8.05 (d, 1H), 7.76 (d, 1H), 7.49-7.46 (m, 2H), 7.07 (d, 1H), 7.01-6.98 (m, 2H), 6.78 (d, 1H), 2.22 (s, 3H).

### Example 24

### 2-(4-Fluoro-2-methylphenoxy)-N-(6-oxo-1,6-dihydropyridazin-4-yl)-5-(trifluoromethox y)benzamide 24

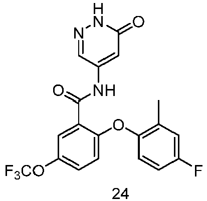

In accordance with the synthetic route in Example **1,** the starting compound 4,5-dichloro-2-fluorobenzoic acid in Step 1 was replaced with compound 2-fluoro-5-(trifluoromethoxy)benzoic acid, accordingly, the title compound **24** (7 mg) was prepared.
MS m/z (ESI): 424.1 [M+1]
¹H NMR (400 MHz, CD₃OD): *δ* 8.05 (d, 1H), 7.71 (d, 1H), 7.50 (d, 1H), 7.44-7.41 (m, 1H), 7.11-6.97 (m, 3H), 6.87 (d, 1H), 2.23 (s, 3H).

### Example 25

### 5-Fluoro-2-(4-fluoro-2-methoxyphenoxy)-N-(6-oxo-1,6-dihydropyridazin-4-yl)benzami de 25

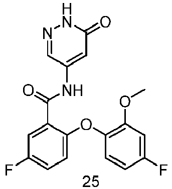

In accordance with the synthetic route in Example **1,** the starting compound 4,5-dichloro-2-fluorobenzoic acid in Step 1 was replaced with compound 2,5-difluorobenzoic acid, accordingly, the title compound **25** (20 mg) was prepared.
MS m/z (ESI): 374.0 [M+1]
1H NMR (400 MHz, DMSO-*d*₆) δ 12.78 (s, 1H), 10.77 (s, 1H), 7.91 (s, 1H), 7.45-7.55 (m, 1H), 7.00-7.32 (m,4H),6.65-6.85 (m, 2H), 3.70 (s, 3H).

### Example 26

### 5-Fluoro-2-(4-fluoro-2-methylphenoxy)-N-(6-oxo-1,6-dihydropyridazin-4-yl)benzamide 26

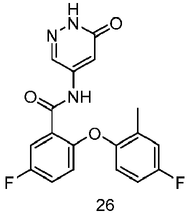

In accordance with the synthetic route in Example **1,** the starting compound 4,5-dichloro-2-fluorobenzoic acid in Step 1 was replaced with compound 2,5-difluorobenzoic acid, and the starting compound 2-methoxy-4-fluorophenol in Step 3 was replaced with 2-methyl-4-fluorophenol, accordingly, the title compound **26** (20 mg) was prepared.
MS m/z (ESI): 358.1 [M+1]
1H NMR (400 MHz, DMSO-*d*₆) δ 12.79 (s, 1H), 10.85 (s, 1H), 7.88 (s, 1H), 7.50-7.60 (m, 1H), 7.30-7.40 (m,1H),7.08-7.19 (m,2H), 6.95-7.05 (m,1H), 6.80-6.95 (m, 2H), 2.14 (s, 3H).

### Example 27

### 4-Chloro-2-(4-fluoro-2-methylphenoxy)-N-(6-oxo-1,6-dihydropyiidazin-4-yl)-5-(trifluo romethyl)benzamide 27

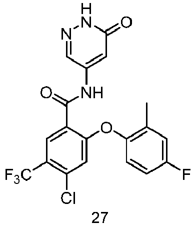

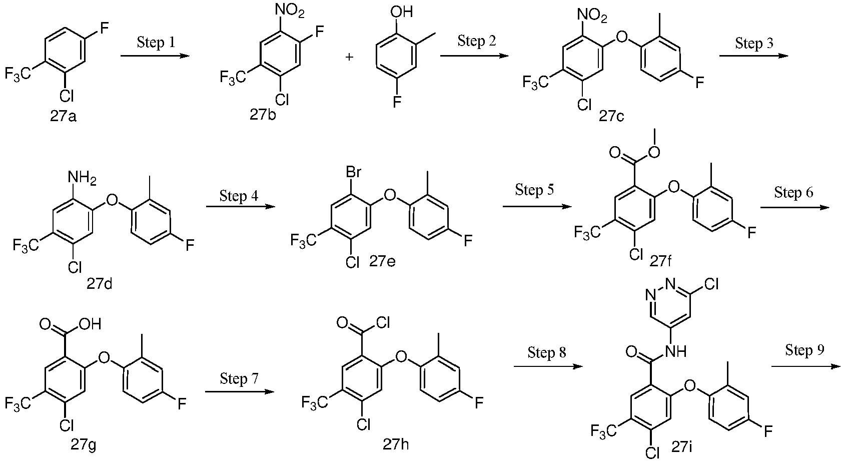

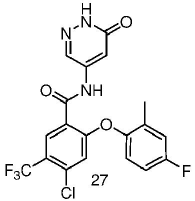

### Step 1

### 1-Chloro-5-fluoro-4-nitro-2-(trifluoromethyl)benzene 27b

2-Chloro-4-fluoro-1-(trifluoromethyl)benzene **27a** (3 g, 15.1 mmol, Adamas Reagent, Co., Ltd.) was dissolved in sulfuric acid (30 mL), and the resulting solution was cooled to -10°C. Potassium nitrate (1.83 g, 18.1 mmol) was added in batches, and the reaction solution was reacted at -10°C for 1 hour, and at room temperature overnight. The reaction solution was poured into ice and extract with ethyl acetate (50 mL×2). The organic phase was dried over anhydrous sodium sulfate and filtered. The filtrate was concentrated under reduced pressure to obtain the title compound **27b** (3.46 g, yield: 94%).

### Step 2

### 1-Chloro-5-(4-fluoro-2-methylphenoxy)-4-nitro-2-(trifluoromethyl)benzene 27c

Compound **27b** (1 g, 4.1 mmol) was dissolved in *N,N*-dimethylformamide (10 mL), followed by the addition of 4-fluoro-2-methylphenol (518 mg, 4.1 mmol) and potassium phosphate (2.61 g, 12.3 mmol). The reaction solution was reacted at 80°C for 1 hour. The reaction solution was filtered, and the filtrate was concentrated under reduced pressure. The resulting residue was purified by silica gel column chromatography with eluent system A to obtain the title compound **27c** (1.27 g, yield: 88%).

### Step 3

### 4-Chloro-2-(4-fluoro-2-methylphenoxy)-5-(trifluoromethyl)aniline 27d

Compound **27c** (1.2 g, 3.4 mmol) was dissolved in ethanol (20 mL) and water (10 mL), followed by the addition of reduced iron powder (1.15 g, 20.6 mmol) and ammonium chloride (1.10 g, 20.6 mmol). The reaction solution was reacted at 80°C for 3 hours. The reaction solution was filtered, and the filtrate was concentrated under reduced pressure. The resulting residue was purified by silica gel column chromatography with eluent system A to obtain the title compound **27d** (900 mg, yield: 82%).

### Step 4

### 1-Bromo-4-chloro-2-(4-fluoro-2-methylphenoxy)-5-(trifluoromethyl)benzene 27e

Compound **27d** (1 g, 3.1 mmol) was dissolved in acetonitrile (10 mL), followed by the addition of copper bromide (839 mg, 3.7 mmol, Adamas Reagent, Co., Ltd.) and tert-butyl nitrite (387 mg, 3.7 mmol). The reaction solution was refluxed for 2 hours. The reaction solution was filtered, and the filtrate was concentrated under reduced pressure to obtain the title compound **27e** (1 g, yield: 83%).

### Step 5

### Methyl 4-chloro-2-(4-fluoro-2-methylphenoxy)-5-(trifluoromethyl)benzoate 27f

Compound **27e** (400 mg, 1.04 mmol) was dissolved in methanol (10 mL), followed by the addition of palladium acetate (47 mg, 0.21 mmol), 1,1'-bis(diphenyphosphino)ferrocene (116 mg, 0.21 mmol) and triethylamine (317 mg, 3.1 mmol). The resulting solution was purged with carbon monoxide three times. The reaction solution was reacted at 70°C overnight under a carbon monoxide atmosphere. The reaction solution was filtered, and the filtrate was concentrated under reduced pressure. The resulting residue was purified by silica gel column chromatography with eluent system A to obtain the title compound **27f** (400 mg, yield: 42%).

### Step 6

### 4-Chloro-2-(4-fluoro-2-methylphenoxy)-5-(trifluoromethyl)benzoic acid 27g

Compound **27f** (480 mg, 1.3 mmol) was dissolved in tetrahydrofuran (5 mL) and water (1 mL), followed by the addition of lithium hydroxide monohydrate (170 mg, 4.0 mmol). The reaction solution was reacted at room temperature overnight. The reaction solution was concentrated under reduced pressure. The resulting residue was dissolved by adding a small amount of water, and the pH was adjusted to 4 with dilute hydrochloric acid. The resulting solution was extracted with ethyl acetate (20 mL×3), and the organic phase was dried over anhydrous sodium sulfate and concentrated under reduced pressure to obtain the title compound **27g** (430 mg, yield: 93%).

### Step 7

### 4-Chloro-2-(4-fluoro-2-methylphenoxy)-5-(trifluoromethyl)benzoyl chloride 27h

Compound **27g** (200 mg, 0.57 mmol) was added to thionyl chloride (2 mL), and the reaction solution was reacted at 80°C for 2 hours. The reaction solution was concentrated under reduced pressure to obtain the crude title compound **27h** (210 mg), which was used directly in the next step.

### Step 8

### 4-Chloro-N-(6-chloropyridin-4-yl)-2-(4-fluoro-2-methylphenoxy)-5-(trifluoromethyl)be nzamide 27i

Compound **27h** (230 mg, 0.73 mmol) was dissolved in pyridine (3 mL), followed by the addition of 4-amino-6-chloropyridazine (95 mg, 0.73 mmol) and 4-dimethylaminopyridine (10 mg, 0.08 mmol). The reaction solution was reacted at room temperature overnight. The reaction solution was concentrated, and the resulting residue was purified by silica gel column chromatography with eluent system B to obtain the title compound **27i** (60 mg, yield: 20%).

### Step 9

### 4-Chloro-2-(4-fluoro-2-methylphenoxy)-N-(6-oxo-1,6-dihydropyridazin-4-yl)-5-(trifluo romethyl)benzamide 27

Compound **27i** (60 mg, 0.13 mmol) was dissolved in acetic acid (2 mL), followed by the addition of potassium acetate (25 mg, 0.25 mmol). The reaction solution was reacted at 130°C for 4 hours, and then concentrated under reduced pressure. The resulting residue was purified by preparative high performance liquid chromatography (Waters 2767-SQ Detecor2, eluent system: ammonium bicarbonate, water, acetonitrile) to obtain the title compound **27** (14 mg, yield: 24%).
MS m/z (ESI): 442.1 [M+1]
1H NMR (400 MHz, CD₃OD): *δ* 8.18 (s, 1H), 8.06 (d, 1H), 7.52 (d, 1H), 7.18-7.15 (m, 2H), 7.09-7.06 (m, 1H), 6.87 (s, 1H), 2.21 (s, 3H).

### Example 28

### 5-Chloro-2-(4-fluoro-2-methylphenoxy)-4-methyl-N-(6-oxo-1,6-dihydropyridazin-4-yl) benzamide 28

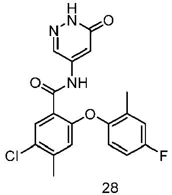

In accordance with the synthetic route in Example **2,** the starting compound 5-chloro-2-fluoro-4-(trifluoromethyl)benzoic acid in Step 2 was replaced with compound 5-chloro-2-fluoro-4-methylbenzoic acid, accordingly, the title compound **28** (3 mg) was prepared.
MS m/z (ESI): 388.2 [M+1]
¹H NMR (400 MHz, CD₃OD): *δ* 8.03 (d, 1H), 7.79 (s, 1H), 7.48 (d, 1H), 7.09-7.06 (m, 1H), 7.00-6.96 (s, 2H), 6.72 (s, 1H), 2.33 (s, 3H), 2.22 (s, 3H).

### Example 29

### 2-(4-Fluoro-2-methylphenoxy)-N-(6-oxo-1,6-dihydropyridazin-4-yl)-4,6-bis(trifluorom ethyl)benzamide 29

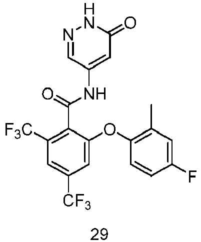

In accordance with the synthetic route in Example **1,** the starting compound 5-chloro-2-fluoro-4-(trifluoromethyl)benzoic acid in Step 1 was replaced with compound 2-fluoro-4,6-bis(trifluoromethyl)benzoic acid, accordingly, the title compound **29** (2.4 mg) was prepared.
MS m/z (ESI): 476.0 [M+1]
1H NMR (400 MHz, CD₃OD) *δ* 8.02 (s, 1H), 7.82 (s, 1H), 7.50 (s, 1H), 7.00-7.20 (m, 4H), 2.19 (s, 3H).

### Example 30

### 5-Chloro-N-(6-oxo-1,6-dihydropyridazin-4-yl)-2-(o-tolyloxy)-4-(trifluoromethyl)benza mide 30

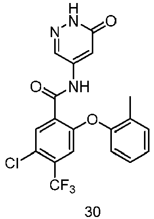

In accordance with the synthetic route in Example **2,** the starting compound 2-methyl-4-fluorophenol in Step 3 was replaced with 2-methylphenol, accordingly, the title compound **30** (75 mg) was prepared.
MS m/z (ESI): 424.1 [M+1]
1H NMR (400 MHz, DMSO-*d*₆) *δ* 12.87 (s, 1H), 11.08 (s, 1H), 8.11 (s, 1H), 7.88 (d, 1H), 7.34-7.32 (m, 1H), 7.27-7.23 (m, 1H), 7.17-7.13 (m, 3H), 7.02-7.00 (m, 1H), 2.16 (s, 3H).

### Example 31

### 5-Chloro-2-(2-cyclopropyl-4-fluorophenoxy)-N-(6-oxo-1,6-dihydropyridazin-4-yl)-4-(tr ifluoromethyl)benzamide 31

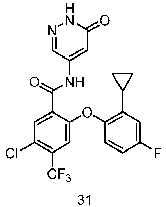

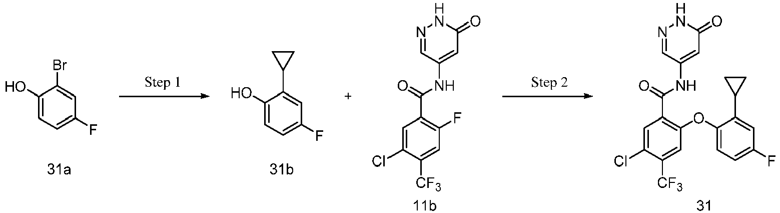

### Step 1

### 2-Cyclopropyl-4-fluorophenol 31b

2-Bromo-4-fluorophenol **31a** (1.77 g, 9.26 mmol, Shanghai Bide Pharmatech Ltd.), tripotassium phosphate (6.89 g, 32.46 mmol), tricyclohexylphosphine (260 mg, 0.93 mmol) and cyclopropylboronic acid (1.20 g, 13.97 mmol, Shanghai Bide Pharmatech Ltd.) were added to a mixed solution of toluene (40 mL)/water (2 mL). The resulting solution was purged with argon three times. Palladium acetate (105 mg, 0.46 mmol) was added, and the reaction solution was purged with argon three times and reacted at 100°C overnight. The reaction solution was cooled, followed by the addition of ethyl acetate (50 mL), and washed with water (50 mL×3). The organic phase was dried over anhydrous sodium sulfate and filtered. The filtrate was concentrated under reduced pressure to obtain the crude title compound **31b** (1.41 g).
MS m/z (ESI): 151.1 [M-1]

### Step 2

### 5-Chloro-2-(2-cyclopropyl-4-fluorophenoxy)-N-(6-oxo-1,6-dihydropyridazin-4-yl)-4-(tr ifluoromethyl)benzamide 31

Compound **11b** (1.03 g, 3.06 mmol), compound **31b** (0.47 g, 3.08 mmol) and cesium carbonate (1.01 g, 3.09 mmol) were added to *N*-methylpyrrolidone (10 mL). The reaction solution was reacted at 60°C overnight. The reaction solution was cooled, followed by the addition of ethyl acetate (150 mL), and washed with water (50 mL×3). The organic phase was dried over anhydrous sodium sulfate and filtered. The filtrate was concentrated under reduced pressure, and the resulting residue was purified by preparative high performance liquid chromatography (Waters 2767-SQ Detecor2, eluent system: ammonium bicarbonate, water, acetonitrile) to obtain the title compound **31** (350 mg, yield: 24%).
MS m/z (ESI): 468.0 [M+1]
¹H NMR (400 MHz, DMSO-*d*₆): *δ* 12.87 (s, 1H), 11.08 (s, 1H), 8.10 (s, 1H), 7.90 (d, 1H), 7.21 (s, 1H), 7.14 (dd, 1H), 7.08-7.03 (m, 2H), 6.87 (dd, 1H), 2.01-1.94 (m, 1H), 0.87-0.83 (m, 2H), 0.71-0.67 (m, 2H).

### Example 32

### 5-Chloro-2-(2-ethoxy-4-fluorophenoxy)-N-(6-oxo-1,6-dihydropyridazin-4-yl)-4-(trifluo romethyl)benzamide 32

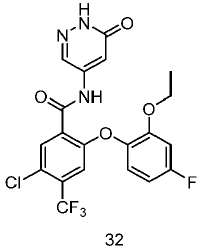

In accordance with the synthetic route in Example **12,** the starting compound cyclopropyl bromide in Step 1 was replaced with compound iodoethane to react at room temperature, accordingly, the title compound **32** (200 mg) was prepared.
MS m/z (ESI): 472.1 [M+1]
¹H NMR (400 MHz, DMSO-*d*₆) *δ* 12.83 (s, 1H), 10.98 (s, 1H), 8.02 (s, 1H), 7.89 (s, 1H), 7.00-7.28 (m,4H), 6.73-6.83 (m,1H), 3.99 (q, 2H), 1.07 (t, 3H).

### Example 33

### 5-Bromo-2-(4-fluoro-2-methylphenoxy)-N-(6-oxo-1,6-dihydropyridazin-4-yl)-4-(trifluo romethyl)benzamide 33

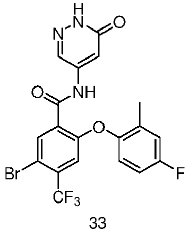

In accordance with the synthetic route in Example **11,** the starting compound 5-chloro-2-fluoro-4-(trifluoromethyl)benzoic acid in Step 1 was replaced with compound 5-bromo-2-fluoro-4-(trifluoromethyl)benzoic acid, accordingly, the title compound **33** (126 mg) was prepared.
MS m/z (ESI): 486.1 [M+1]
¹H NMR (400 MHz, DMSO-*d*₆) *δ* 12.84 (s, 1H), 11.03 (s, 1H), 8.19 (s, 1H), 7.86-7.85 (d, 1H), 7.21-7.16 (m, 2H), 7.07-7.06 (d, 1H), 2.13 (s, 3H).

### Example 34

### 2-(4-Fluoro-2-methylphenoxy)-5-methyl-N-(6-oxo-1,6-dihydropyridazin-4-yl)-4-(trifluo romethyl)benzamide 34

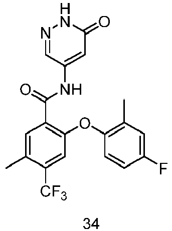

In accordance with the synthetic route in Example **11,** the starting compound 5-chloro-2-fluoro-4-(trifluoromethyl)benzoic acid in Step 1 was replaced with compound 5-methyl-2-fluoro-4-(trifluoromethyl)benzoic acid, and the starting compound 2-methoxy-4-fluorophenol in Step 3 was replaced with 2-methyl-4-fluorophenol, accordingly, the title compound **34** (15 mg) was prepared.
MS m/z (ESI): 422.0 [M+1]
¹H NMR (400 MHz, DMSO-*d*₆) *δ* 12.83 (s, 1H), 11.97 (s, 1H), 7.89-7.88 (d, 1H), 7.74 (d, 1H), 7.19-7.18 (m, 2H), 7.08-7.04 (m, 1H), 7.00-6.97 (m, 2H), 2.44 (s, 3H), 2.15 (s, 3H).

### Example 35

### 5-Chloro-2-(4-fluoro-2-(methoxy-d₃)phenoxy)-N-(6-oxo-1,6-dihydropyridazin-4-yl)-4-( trifluoromethyl)benzamide 35

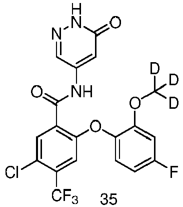

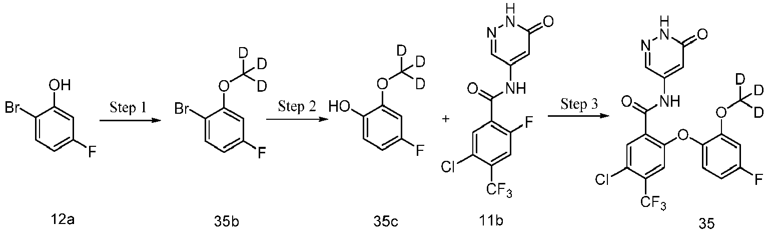

### Step 1

### 1-Bromo-4-fluoro-2-(methoxy-d₃)benzene 35b

2-Bromo-5-fluorophenol **12a** (1 g, 5.2 mmol, Accela ChemBio (Shanghai) Inc.), deuterated methyl iodide (911 mg, 6.3 mmol, Sun Chemical Technology (Shanghai) Co., Ltd.) and potassium carbonate (1.45 g, 10.5 mmol) were added to *N,N*-dimethylformamide (10 mL). The reaction solution was stirred to react for 6 hours. The reaction solution was cooled to room temperature. Ethyl acetate (20 mL) was added, and the reaction solution was washed with water (20 mL×3). The organic phase was dried over anhydrous sodium sulfate and filtered. The filtrate was concentrated under reduced pressure, and the resulting residue was purified by silica gel column chromatography with eluent system A to obtain the title compound **35b** (840 mg, yield: 71%).
¹H NMR (400 MHz, CDCl₃): δ 7.49-7.45 (m, 1H), 6.66-6.57 (m, 2H).

### Step 2

### 4-Fluoro-2-(methoxy-d₃)phenol 35c

Compound **35b** (840 mg, 4 mmol) and triisopropyl borate (987 mg, 5.25 mmol, Shanghai Titan Scientific Co., Ltd.) were added to a mixed solution of tetrahydrofuran/toluene (150 mL/30 mL). The air in the reaction flask was replaced with argon. The reaction solution was cooled to -78°C, then *n*-butyl lithium (1.6 M, 3.8 mL, 6.1 mmol) was slowly added dropwise within 20 minutes. The reaction solution was naturally warmed up to room temperature and stirred overnight. The reaction solution was cooled to 0°C in an ice bath. Methanol (50 mL) was added, and hydrogen peroxide (30 wt%, 10 mL) and 10% sodium hydroxide solution (40 mL) were added dropwise. The reaction solution was stirred at room temperature for 1 hour. Saturated sodium thiosulfate solution (50 mL) was slowly added dropwise, and the reaction solution was extracted with ethyl acetate (200 mL×3). The organic phase was washed with saturated sodium bicarbonate solution (150 mL), dried over anhydrous sodium sulfate and filtered. The filtrate was concentrated under reduced pressure, and the resulting residue was purified by silica gel column chromatography with eluent system B to obtain the title compound **35c** (570 mg, yield: 97%).
MS m/z (ESI): 144.0 [M-1]
¹H NMR (400 MHz, DMSO-*d*₆): *δ* 8.89 (s, 1H), 6.85-6.82 (m, 1H), 6.76-6.72 (m, 1H), 6.59-6.54 (m, 1H).

### Step 3

### 5-Chloro-2-(4-fluoro-2-(methoxy-d₃)phenoxy)-N-(6-oxo-1,6-dihydropyridazin-4-yl)-4-( trifluoromethyl)benzamide 35

Compound **11b** (1 g, 2.98 mmol), compound **35c** (433 mg, 2.98 mmol) and cesium carbonate (1.02 g, 3.13 mmol, Accela ChemBio (Shanghai) Inc.) were added to *N*-methylpyrrolidone (10 mL). The reaction solution was reacted at 80°C for 3 hours, and cooled to room temperature. Ethyl acetate (20 mL) was added, and the reaction solution was washed with water (10 mL×3). The organic phase was dried over anhydrous sodium sulfate and filtered. The filtrate was concentrated under reduced pressure, and the resulting residue was purified by silica gel column chromatography with eluent system B to obtain the title compound **35** (280 mg, yield: 20%).
MS m/z (ESI): 461.0 [M+1]
¹H NMR (400 MHz, DMSO-*d*₆): δ 12.87 (s, 1H), 11.03 (s, 1H), 8.06 (s, 1H), 7.93 (d, 1H), 7.29-7.23 (m, 2H), 7.16-7.13 (m, 1H), 7.01 (s, 1H), 6.88-6.83 (m, 1H).

### Example 36

### 5-Chloro-2-(2-ethyl-4-fluorophenoxy)-N-(6-oxo-1,6-dihydropyiidazin-4-yl)-4-(trifluoro methyl)benzamide 36

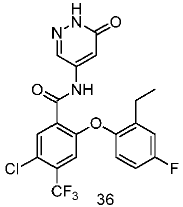

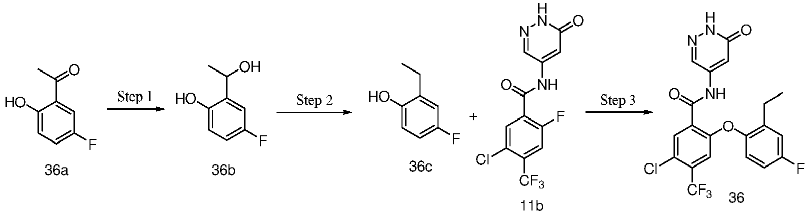

### Step 1

### 4-Fluoro-2-(1-hydroxyethyl)phenol 36b

Compound 1-(5-fluoro-2-hydroxyphenyl)ethan-1-one **36a** (3 g, 19.5 mmol, Accela ChemBio (Shanghai) Inc.) was dissolved in anhydrous methanol (20 mL). Sodium borohydride (1.1 g, 29.1 mmol) was slowly added in batches, and the reaction solution was reacted at room temperature for 1 hour. The reaction solution was concentrated under reduced pressure, and ethyl acetate and water were added. The organic phase was dried over anhydrous sodium sulfate and filtered. The filtrate was concentrated under reduced pressure to obtain the crude title compound **36b** (3.2 g, yield: 100%).
MS m/z (ESI): 155.1[M-1]

### Step 2

### 2-Ethyl-4-fluorophenol 36c

Compound **36b** (1.5 g, 9.6 mmol) was dissolved in dichloromethane (15 mL), followed by the addition of trifluoroacetic acid (11 g, 96.5 mmol, 7.2 mL). Triethylsilane (11.2 g, 96.3 mmol, 15.4 mL) was added dropwise, and the reaction solution was reacted at room temperature for 2 hours. The reaction solution was concentrated under reduced pressure, and dichloromethane and water were added. The organic phase was dried over anhydrous sodium sulfate and filtered. The filtrate was concentrated under reduced pressure to obtain the crude title compound **36c** (1.38 g, yield: 100%).
MS m/z (ESI): 139.1[M-1]

### Step 3

### 5-Chloro-2-(2-ethyl-4-fluorophenoxy)-N-(6-oxo-1,6-dihydropyridazin-4-yl)-4-(trifluoro methyl)benzamide 36

Compound **11b** (100 mg, 0.3 mmol), compound **36c** (42 mg, 0.3 mmol) and cesium carbonate (100 mg, 0.33 mmol) were added to *N*-methylpyrrolidone (2 mL). The reaction solution was reacted at 60°C overnight. The reaction solution was cooled, followed by the addition of ethyl acetate (150 mL), and washed with water (50 mL×3). The organic phase was dried over anhydrous sodium sulfate and filtered. The filtrate was concentrated under reduced pressure, and the resulting residue was purified by preparative high performance liquid chromatography (Waters 2767-SQ Detecor2, eluent system: ammonium bicarbonate, water, acetonitrile) to obtain the title compound **36** (18 mg, yield: 13%).
MS m/z (ESI): 456.0[M+1]
¹H NMR (400 MHz, DMSO-*d*₆) *δ* 12.88 (s, 1H), 11.09 (s, 1H), 8.12 (s, 1H), 7.90-7.90 (d, 1H), 7.25-7.20 (m, 2H), 7.14 (m, 1H), 7.11-7.10 (m, 2H), 2.58-2.54 (m, 2H), 1.10-1.06 (m, 3H).

### Example 37

### 5-Chloro-2-(2-(difluoromethoxy)-4-fluorophenoxy)-N-(6-oxo-1,6-dihydropyridazin-4-y l)-4-(trifluoromethyl)benzamide 37

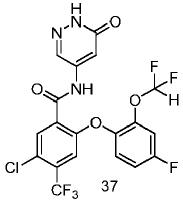

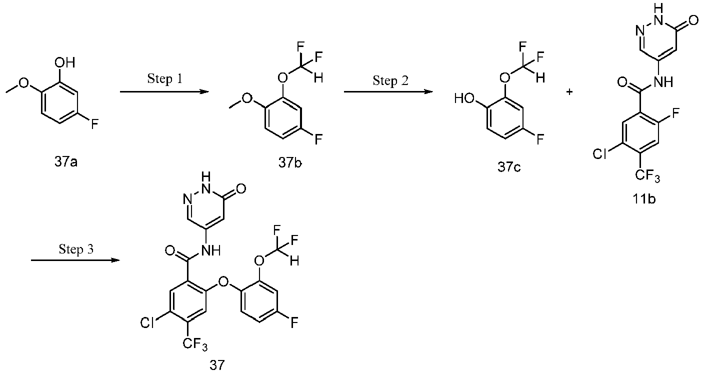

### Step 1

### 2-(Difluoromethoxy)-4-fluoro-1-methoxybenzene 37b

5-Fluoro-2-methoxyphenol **37a** (1 g, 7.03 mmol, Accela ChemBio (Shanghai) Inc.), sodium difluorochloroacetate (2.68 g, 17.57 mmol, Accela ChemBio (Shanghai) Inc.) and cesium carbonate (4.58 g, 14.05 mmol) were added to a mixed solvent of *N,N*-dimethylformamide (14 mL)/water (1.5 mL). The reaction solution was reacted at 100°C overnight. The reaction solution was cooled, followed by the addition of dichloromethane (100 mL), and washed with water (50 mL×3). The organic phase was dried over anhydrous sodium sulfate and filtered. The filtrate was concentrated under reduced pressure to obtain the crude title compound **37b** (1.35 g).

### Step 2

### 2-(Difluoromethoxy)-4-fluorophenol 37c

Sodium iodide (5.27 g, 35.15 mmol, Shanghai Sinopharm Chemical Reagent Co., Ltd.) was dissolved in acetonitrile (20 mL), followed by the addition of trimethylchlorosilane (3.82 g, 35.16 mmol, Shanghai Sinopharm Chemical Reagent Co., Ltd.). The reaction solution was reacted at room temperature for 20 minutes. Compound **37b** (1.35 g, 7.0263 mmol) was added, and the reaction solution was reacted at 80°C overnight. The reaction solution was cooled, followed by the addition of water (50 mL), and extracted with ethyl acetate (50 mL×3). The organic phases were combined, dried over anhydrous sodium sulfate and filtered. The filtrate was concentrated under reduced pressure, and the resulting residue was purified by silica gel column chromatography with eluent system A to obtain the title compound **37c** (540 mg, yield: 43%).
MS m/z (ESI): 177.1 [M-1]

### Step 3

### 5-Chloro-2-(2-(difluoromethoxy)-4-fluorophenoxy)-N-(6-oxo-1,6-dihydropyridazin-4-y l)-4-(trifluoromethyl)benzamide 37

Compound **11b** (1.02 g, 3.03 mmol), compound **37c** (0.54 g, 3.03 mmol) and cesium carbonate (0.99 g, 3.13 mmol) were added to *N*-methylpyrrolidone (10 mL). The reaction solution was reacted at 60°C overnight. The reaction solution was cooled, followed by the addition of ethyl acetate (150 mL), and washed with water (50 mL×3). The organic phase was dried over anhydrous sodium sulfate and filtered. The filtrate was concentrated under reduced pressure, and the resulting residue was purified by preparative high performance liquid chromatography (Waters 2767-SQ Detecor2, eluent system: ammonium bicarbonate, water, acetonitrile) to obtain the title compound **37** (450 mg, yield: 30%).
MS m/z (ESI): 493.9 [M+1]
¹H NMR (400 MHz, DMSO-*d*₆): *δ* 12.86 (s, 1H), 11.0 6(s, 1H), 8.11 (s, 1H), 7.89 (d, 1H), 7.38 (dd, 1H), 7.33 (dd, 1H), 7.27 (s, 1H), 7.23 (t, 1H), 7.22-7.17 (m, 2H).

### Example 38

### 5-Chloro-2-(4-fluoro-2-hydroxyphenoxy)-N-(6-oxo-1,6-dihydropyridazin-4-yl)-4-(triflu oromethyl)benzamide 38

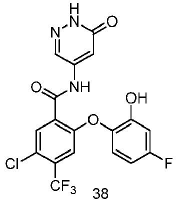

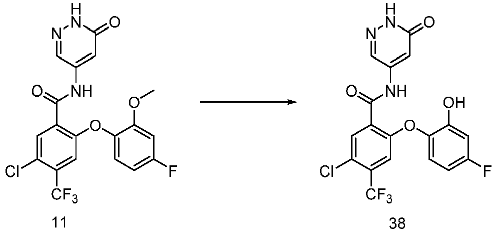

Compound **11** (100 mg, 0.22 mmol) was added to dichloromethane (2 mL). The resulting solution was cooled to -78°C, and boron tribromide (1 M, 1.09 mL, Adamas Beta (Shanghai) Reagent, Co., Ltd.) was added dropwise. The reaction solution was warmed up to 0°C and reacted for 3 hours. Additional boron tribromide (1 M, 1.09 mL) was added at 0°C, and the reaction solution was reacted at room temperature overnight. The reaction solution was concentrated, and 10 mL of methanol was added dropwise to the resulting residue. The resulting solution was stirred well and concentrated. The resulting residue was purified by preparative high performance liquid chromatography (Waters 2767-SQ Detecor2, eluent system: ammonium bicarbonate, water, acetonitrile) to obtain the title compound **38** (50 mg, yield: 51%).
MS m/z (ESI): 444.1 [M+1]
¹H NMR (400 MHz, DMSO-*d*₆): *δ* 12.88 (s, 1H), 10.99 (s, 1H), 10.47 (s, 1H), 8.04 (s, 1H), 7.94 (s, 1H), 7.26-7.22 (m, 2H), 6.97 (s, 1H), 6.80 (dd, 1H), 6.75-6.70 (m, 1H).

### Example 39

### 5-Chloro-2-((7-fluoro-2,3-dihydro-1H-inden-4-yl)oxy)-N-(6-oxo-1,6-dihydropyridazin-4-yl)-4-(trifluoromethyl)benzamide 39

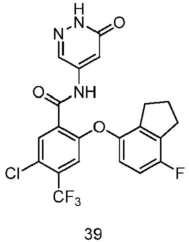

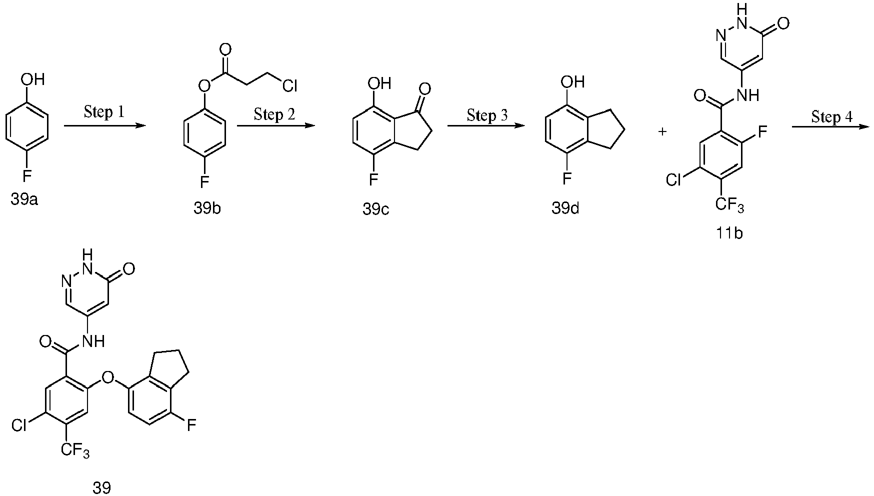

### Step 1

### 4-Fluorophenyl 3-chloropropanoate 39b

The starting compound 4-fluorophenol **39a** (15 g, 133.8 mmol, Accela ChemBio (Shanghai) Inc.) was dissolved in dichloromethane (80 mL), followed by the addition of pyridine (12 g, 151.7 mmol). 3-Chloropropionyl chloride (18.9 g, 134 mmol, Accela ChemBio (Shanghai) Inc.) was slowly added dropwise under an ice bath. After completion of the addition, the reaction solution was stirred at room temperature for 1 hour. Saturated sodium bicarbonate solution (60 mL) was added, and the reaction solution was extracted with ethyl acetate (80 mL×2). The organic phases were combined, dried over anhydrous sodium sulfate and filtered. The filtrate was concentrated under reduced pressure, and the resulting residue was purified by silica gel column chromatography with eluent system A to obtain the title compound **39b** (25 g, yield: 86%).
MS m/z (ESI): 203.1[M+1]

### Step 2

### 4-Fluoro-7-hydroxy-2,3 -dihydro-1H-inden-1-one 39c

Compound **39b** (10 g, 49.4 mmol) and aluminum trichloride (20 g, 150 mmol, Sinopharm Chemical Reagent Co., Ltd.) were mixed well, and the reaction mixture was heated to 100°C, and stirred for 15 minutes. The reaction mixture was heated to 180°C and reacted for 3 hours. The reaction mixture was cooled to room temperature, and slowly added to ice water. Ethyl acetate was added, and the resulting solution was stirred for 2 hours. The organic phase was dried over anhydrous sodium sulfate and concentrated under reduced pressure. The resulting residue was purified by silica gel column chromatography with eluent system A to obtain the title compound **39c** (3 g, yield: 37%).
MS m/z (ESI): 167.3 [M+1]

### Step 3

### 7-Fluoro-2,3-dihydro-1H-inden-4-ol 39d

Compound **39c** (8 g, 48.2 mmol) was dissolved in trifluoroacetic acid (80 mL), followed by the addition of triethylsilane (14 g, 120.4 mmol). The reaction solution was stirred at 80°C overnight. The reaction solution was concentrated under reduced pressure, and the resulting residue was purified by silica gel column chromatography with eluent system A to obtain the title compound **39d** (6 g, yield: 82%).
MS m/z (ESI): 151.3 [M-1]

### Step 4

### 5-Chloro-2-((7-fluoro-2,3-dihydro-1H-inden-4-yl)oxy)-N-(6-oxo-1,6-dihydropyridazin-4-yl)-4-(trifluoromethyl)benzamide 39

Compound **11b** (1.02 g, 3.03 mmol), compound **39d** (0.54 g, 3.03 mmol) and cesium carbonate (0.99 g, 3.13 mmol) were added to *N*-methylpyrrolidone (10 mL). The reaction solution was reacted at 60°C overnight. The reaction solution was cooled, followed by the addition of ethyl acetate (150 mL), and washed with water (50 mL×3). The organic phase was dried over anhydrous sodium sulfate and filtered. The filtrate was concentrated under reduced pressure, and the resulting residue was purified by preparative high performance liquid chromatography (Waters 2767-SQ Detecor2, eluent system: ammonium bicarbonate, water, acetonitrile) to obtain the title compound **39** (400 mg, yield: 29%).
MS m/z (ESI): 468.0 [M+1]
1H NMR (400 MHz, DMSO-*d*₆) *δ* 12.86 (s, 1H), 11.03 (s, 1H), 8.08 (s, 1H), 7.874-7.868 (d, 1H), 7.27 (m,1H), 7.15 (m, 1H), 7.04-7.00 (m, 1H), 6.92-6.89 (m, 1H), 2.92-2.88 (m, 2H), 2.77-2.73 (m, 2H), 2.05-1.99 (m, 2H).

### Example 40

### 5-Chloro-2-(2-(cyclopentyloxy)-4-fluorophenoxy)-N-(6-oxo-1,6-dihydropyridazin-4-yl) -4-(trifluoromethyl)benzamide 40

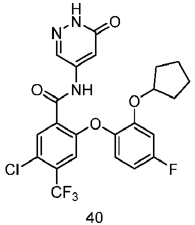

In accordance with the synthetic route in Example **35,** the starting compound deuterated methyl iodide in Step 1 was replaced with compound bromocyclopentane, accordingly, the title compound **40** (30 mg) was prepared.
MS m/z (ESI): 511.9[M+1].
¹H NMR (400 MHz, DMSO-*d*₆) *δ* 12.87 (s, 1H), 10.99 (s, 1H), 8.05 (s, 1H), 7.95 (s, 1H), 7.30-7.35 (m, 1H), 7.29 (s, 1H), 7.11 (dd,1H), 6.98 (s, 1H), 6.77-6.86 (m, 1H), 4.80-4.90 (m, 1H), 1.72-1.85 (m, 2H), 1.35-1.55 (m, 4H),1.15-1.35 (m, 2H).

### Example 41

### 5-Chloro-2-(2-cyclobutoxy-4-fluorophenoxy)-N-(6-oxo-1,6-dihydropyridazin-4-yl)-4-(t rifluoromethyl)benzamide 41

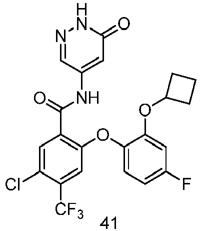

In accordance with the synthetic route in Example **35,** the starting compound deuterated methyl iodide in Step 1 was replaced with compound bromocyclobutane, accordingly, the title compound **41** (110 mg) was prepared.
MS m/z (ESI): 498.0 [M+1]
¹H NMR (400 MHz, DMSO-*d*₆): *δ* 12.87 (s, 1H), 11.02 (s, 1H), 8.06 (s, 1H), 7.94 (s, 1H), 7.29-7.23 (m, 2H), 7.09 (s, 1H), 6.95-6.92 (m, 1H), 6.92-6.83 (m, 1H), 4.73-4.70 (m, 1H), 2.37-2.33 (m, 2H), 1.79-1.72 (m, 2H), 1.68-1.52 (m, 2H).

### Example 42

### 5-Chloro-2-(4-fluoro-2-isopropylphenoxy)-N-(6-oxo-1,6-dihydropyridazin-4-yl)-4-(trifl uoromethyl)benzamide 42

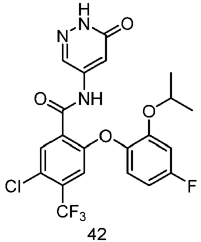

In accordance with the synthetic route in Example **35,** the starting compound deuterated methyl iodide in Step 1 was replaced with compound iodoisopropane, accordingly, the title compound **42** (100 mg) was prepared.
MS m/z (ESI): 485.9 [M+1]
1H NMR (400 MHz, DMSO-*d*₆): *δ* 12.87 (s, 1H), 11.00 (s, 1H), 8.05 (s, 1H), 7.95 (s, 1H), 7.32-7.16 (m, 3H), 7.01 (s, 1H), 6.86-6.82 (m, 1H), 4.67-4.62 (m, 1H), 1.08 (s, 3H), 1.06 (s, 3H).

### Example 43

### 2-(2-Bromo-4-fluorophenoxy)-5-chloro-N-(6-oxo-1,6-dihydropyridazin-4-yl)-4-(trifluor omethyl)benzamide 43

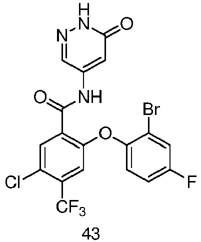

In accordance with the synthetic route in Example **11,** the starting compound 2-methoxy-4-fluorophenol in Step 3 was replaced with 2-bromo-4-fluorophenol, accordingly, the title compound **43** (100 mg) was prepared.
MS m/z (ESI): 505.7 [M+1]
1H NMR (400 MHz, DMSO-*d*₆) *δ* 12.86 (s, 1H), 11.08 (s, 1H), 8.14 (s, 1H), 7.90 (d, 1H), 7.75 (dd, 1H), 7.36-7.26 (m, 3H), 7.17(s, 1H).

### Example 44

### 5-Chloro-2-(4-fluoro-2-(methyl-d₃)phenoxy)-N-(6-oxo-1,6-dihydropyridazin-4-yl)-4-(tri fluoromethyl)benzamide 44

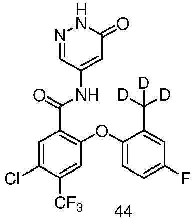

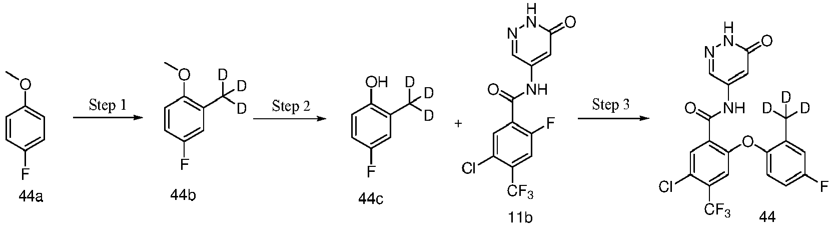

### Step 1

### 4-Fluoro-1-methoxy-2-(methyl-d₃)benzene 44b

Compound 1-fluoro-4-methoxybenzene (5 g, 39.6 mmol, Accela ChemBio (Shanghai) Inc.) was dissolved in tetrahydrofuran (50 mL). The resulting solution was cooled to -10°C, and *n*-butyllithium (2.5 M, 43.7 mmol, 17.5 mL) was added dropwise. The reaction solution was naturally warmed up to room temperature and reacted for 1 hour. Iodomethane-d3 (5.8 g, 40 mmol, Accela ChemBio (Shanghai) Inc.) was slowly added dropwise under an ice bath, and the reaction solution was reacted at room temperature for 2 hours. Water (50 mL) was slowly added, and the reaction solution was extracted with ethyl acetate (20 mL×3). The organic phases were combined, dried over anhydrous sodium sulfate and filtered. The filtrate was concentrated under reduced pressure, and the resulting residue was purified by silica gel column chromatography with eluent system A to obtain the title compound **44b** (1.5 g, yield: 26%).
¹H NMR (400 MHz, CDCl₃) *δ* 6.86-6.85 (m, 2H), 6.73-6.69 (m, 1H), 3.78 (s, 3H).

### Step 2

### 4-Fluoro-2-(methyl-d₃)phenol 44c

Compound **44a** (1.5 g, 10.5 mmol) was added to dichloromethane (30 mL). A solution of boron tribromide in dichloromethane (1 M, 21.2 mmol, 21.2 mL) was added dropwise at room temperature, and the reaction solution was reacted at room temperature for 1 hour. The reaction solution was concentrated under reduced pressure, and the resulting residue was purified by silica gel column chromatography with eluent system A to obtain the title compound **44c** (900 mg, yield: 67%).

### Step 3

### 5-Chloro-2-(4-fluoro-2-(methyl-d₃)phenoxy)-N-(6-oxo-1,6-dihydropyridazin-4-yl)-4-(tri fluoromethyl)benzamide 44

Compound **11b** (100 mg, 0.3 mmol), compound **44c** (39 mg, 0.3 mmol) and cesium carbonate (100 mg, 0.33 mmol) were added to *N*-methylpyrrolidone (2 mL). The reaction solution was reacted at 60°C overnight. The reaction solution was cooled, followed by the addition of ethyl acetate (150 mL), and washed with water (50 mL×3). The organic phase was dried over anhydrous sodium sulfate and filtered. The filtrate was concentrated under reduced pressure, and the resulting residue was purified by preparative high performance liquid chromatography (Waters 2767-SQ Detecor2, eluent system: ammonium bicarbonate, water, acetonitrile) to obtain the title compound **44** (21 mg, yield: 16%).
MS m/z (ESI): 445.0[M+1]
¹H NMR (400 MHz, DMSO-*d*₆) *δ* 8.09 (s, 1H), 7.89-7.88 (d, 1H), 7.23-7.21 (d, 1H), 7.18-7.18 (dd, 1H), 7.13 (m,1H), 7.10-7.08 (m, 2H).

### Example 45

### 5-Chloro-2-(2-chloro-4-fluorophenoxy)-N-(6-oxo-1,6-dihydropyridazin-4-yl)-4-(trifluor omethyl)benzamide 45

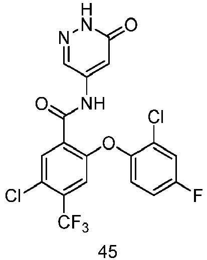

In accordance with the synthetic route in Example **11,** the starting compound 2-methoxy-4-fluorophenol in Step 3 was replaced with 2-chloro-4-fluorophenol, accordingly, the title compound **45** (40 mg) was prepared.
MS m/z (ESI): 461.8 [M+1]
1H NMR (400 MHz, DMSO-*d*₆) δ 12.87 (s, 1H), 11.08 (s, 1H), 8.14 (s, 1H), 7.89 (d, 1H), 7.64 (dd, 1H), 7.33-7.26 (m, 3H), 7.17 (s, 1H).

### Example 46

### 5-Chloro-2-(2,4-difluorophenoxy)-N-(6-oxo-1,6-dihydropyridazin-4-yl)-4-(trifluoromet hyl)benzamide 46

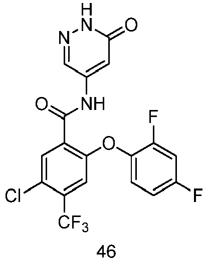

In accordance with the synthetic route in Example **11,** the starting compound 2-methoxy-4-fluorophenol in Step 3 was replaced with 2,4-difluorophenol, accordingly, the title compound 46 (70 mg) was prepared.
MS m/z (ESI): 445.8 [M+1]
1H NMR (400 MHz, DMSO-*d*₆) *δ* 12.87 (s, 1H), 11.10 (s, 1H), 8.12 (s, 1H), 7.89 (d, 1H), 7.53-7.47 (m, 1H), 7.38-7.32 (m, 2H), 7.18-7.12 (m, 2H).

### Biological Assay

The present disclosure will be further described with reference to the following test examples, but the examples should not be considered as limiting the scope of the present disclosure.

Test Example 1. Determination of the inhibitory activity of the compounds of the present disclosure on Naᵥ1.8

The purpose of the experiment is to investigate the effect of the compounds on Na_{V}1.8 ion channel in an *in vitro* experiment, wherein the Naᵥ1.8 ion channel is stably expressed on HEK293 cells. After the Naᵥ1.8 current becomes stable, the Naᵥ1.8 currents before and after the administration of the compound are compared so as to obtain the effect of the compound on the Naᵥ1.8 ion channel.

### 1 Experimental materials and instruments

1) Patch clamp amplifier: patch clamp PC-505B (WARNER instruments)/MultiClamp 700A (Axon instrument)
2) Digital-to-analog converter: Digidata 1440A (Axon CNS)/Digidata 1550A (Axon instruments)
3) Micro-manipulator: MP-225 (SUTTER instrument)
4) Inverted microscope: TL4 (Olympus)
5) Glass microelectrode puller: PC-10 (NARISHIGE)
6) Microelectrode glass capillary: B12024F (Wuhan Weitan Scientific Instrument Co., Ltd.)
7) Dimethyl sulfoxide (DMSO) D2650 (Sigma-Aldrich)
8) TTX AF3014 (Affix Scientific)

### 2 Experimental procedures

### 2.1 Formulation of the compounds

Except for NaOH and KOH used for acid titration and base titration, all the compounds used for formulating the extracellular fluid and intracellular fluid were purchased from Sigma (St. Louis, MO). Extracellular fluid (mM): NaCl, 137; KCl, 4; CaCl₂, 1.8; MgCl₂, 1; HEPES, 10; glucose, 10; pH 7.4 (NaOH titration). Intracellular fluid (mM): aspartic acid, 140; MgCl₂, 2; EGTA, 11; HEPES, 10; pH 7.2 (CsOH titration). All solutions of test compound and control compound contained 1 µM TTX.

The test compound was dissolved in dimethyl sulfoxide (DMSO) at a stock concentration of 9 mM. The stock solution of the test compound was dissolved in the extracellular fluid on the day of the test and formulated into the required concentration.

### 2.2 Test process of the manual patch clamp

1) The compound was formulated into solutions with specified concentrations, the solutions were added to the pipelines respectively in order from low to high concentration, and the pipelines were marked.
2) The cell was transferred to the perfusion tank. A positive pressure was applied to the electrode. The tip of the electrode touched the cell. The three-way valve of the air extracting device was adjusted to a three-way state. A negative pressure was applied to the electrode, so that a high-resistance seal was formed between the electrode and the cell. The negative pressure was applied continuously, thereby causing the cell membrane to rupture and forming a current path.
3) After the current for rupturing the cell membrane became stable, perfusion of different concentrations was carried out in sequence. Once the current was stable for at least one minute, perfusion of the next concentration was carried out. The duration of the perfusion of each concentration did not exceed five minutes.
4) The perfusion tank was cleaned. The perfusion tank was rinsed with the drug solutions in order from high to low concentration, and the rinse duration for each concentration of drug solution was 20 seconds. The perfusion tank was finally rinsed with the extracellular fluid for 1 mintue.

### 2.3 Test voltage equation (resting) and results

The cell was clamped at -80 mV. The cell was depolarized to 10 mV with a square wave lasting 10 milliseconds to obtain the Naᵥ1.8 current. This procedure was repeated every 5 seconds. The maximum current caused by the square wave was measured. After the current became stable, the test compound was perfused. After the response became stable, the blocking intensity was calculated.

### 3. Data analysis

The data was stored in the computer system for analysis. Data collection and analysis were carried out by pCLAMP 10 (Molecular Devices, Union City, CA), and the analysis results were reviewed by the administrator. Stable current means that the current changes within a limited range over time. The magnitude of stable current was used to calculate the effect of the compound at the concentration.

The inhibitory activity of the compounds of the present disclosure on Naᵥ1.8 was determined by the above test, and the resulting IC₅₀ values are shown in Table 1.

**Table 1. IC₅₀ of the compounds of the present disclosure on inhibiting the Naᵥ1.8 channel activity**

| Example No. | IC₅₀ (nM) |
|---|---|
| 1 | 1.6 |
| 2 | 1.3 |
| 3 | 3.3 |
| 4 | 14.1 |
| 5 | 23.7 |
| 6 | 24.4 |
| 7 | 30.8 |
| 8 | 45.8 |
| 9 | 83.4 |
| 11 | 1.3 |
| 12 | 0.26 |
| 13 | 2.3 |
| 14 | 2.5 |
| 15 | 4.7 |
| 16 | 17.9 |
| 17 | 18.0 |
| 18 | 22.2 |
| 19 | 31.7 |
| 20 | 58.5 |
| 21 | 59.4 |
| 22 | 87.8 |
| 23 | 92.3 |
| 27 | 5.7 |
| 28 | 5.2 |
| 29 | 90.4 |
| 30 | 16.2 |
| 31 | 0.94 |
| 32 | 0.37 |
| 33 | 0.86 |
| 34 | 11.5 |
| 35 | 0.54 |
| 36 | 1.54 |
| 37 | 2.38 |
| 39 | 0.24 |
| 40 | 0.32 |
| 41 | 1.86 |
| 42 | 2.98 |
| 43 | 3.36 |
| 44 | 4.04 |
| 45 | 5.08 |
| 46 | 8.95 |

Conclusion: The compounds of the present disclosure have a significant inhibitory effect on the Na_{V}1.8 channel activity.

### Pharmacokinetics Evaluation

Test Example 2. Pharmacokinetics assay of the compounds of the present disclosure

### 1. Abstract

Rats were used as test animals. The drug concentration in plasma at different time points was determined by LC/MS/MS method after intragastrical administration of the compounds of Example 2, Example 11, Example 12, Example 15, Example 31 and Example 33 to rats. The pharmacokinetic behavior of the compounds of the present disclosure was studied and evaluated in rats.

### 2. Test protocol

### 2.1 Test compounds

Compounds of Example 2, Example 11, Example 12, Example 15, Example 31 and Example 33.

### 2.2 Test animals

Twenty-four healthy adult SD rats (half male and half female, equally divided into six groups, four rats per group) were purchased from Shanghai Jiesijie Laboratory Animal Co., LTD.

### 2.3 Preparation of the test compound

A certain amount of the test compound was weighed, to which 5% of DMSO, 5% of tween 80 and 90% of normal saline were added to prepare a 0.2 mg/mL colorless, clear and transparent solution.

### 2.4 Administration

After an overnight fast, SD rats were intragastrically administered the test compound at an administration dose of 2.0 mg/kg and an administration volume of 10.0 mL/kg.

### 3. Process

The rats were intragastrically administered the compounds of Example 2, Example 11, Example 12, Example 15, Example 31 and Example 33. 0.2 ml of blood was taken from the orbit before the administration and at 0.5, 1.0, 2.0, 4.0, 6.0, 8.0, 11.0 and 24.0 hours after the administration. The samples were stored in heparinized tubes, and centrifuged for 10 minutes at 10000 rpm and 4°C to separate the blood plasma. The plasma samples were stored at -20°C. The rats were fed 2 hours after the administration.

The content of the test compound in the plasma of rats after intragastrical administration of the test compound at different concentrations was determined: 25 µL of rat plasma at each time point after the administration was taken, to which 30 µL of the internal standard solution and 175 µL of acetonitrile were added. The resulting solution was vortex-mixed for 5 minutes, and centrifuged for 10 minutes (3700 rpm). 0.5 µL of the supernatant was taken from the plasma samples for LC/MS/MS analysis.

### 4. Results of pharmacokinetic parameters

Pharmacokinetic parameters of the compounds of the present disclosure are shown below:

| No. | Pharmacokinetics assay (2 mg/kg) | | | | | | |
|---|---|---|---|---|---|---|---|
| | Plasma concentration | Area under curve | Half-life | Residence time | Clearance | Apparent distribution volume | Bioavail ability |
| | Cmax (ng /mL) | AUC (ng /mL^{∗}h) | T1/2 (h) | MRT(h) | CL/F (ml/min/kg) | Vz/F (ml/kg) | F (%) |
| 2 | 399±153 | 5963±4279 | 47±54.6 | 68.2±78.3 | 6.24±6.29 | 4711±1293 | 149 |
| 11 | 218±103 | 1234±621 | 2.3±1.14 | 4..36±1.46 | 32.3±15.2 | 5332±720 | 98 |
| 12 | 435±64 | 5981±1126 | 13.2±7.8 | 19.8±10.4 | 4.3±1.65 | 4174±587 | 111 |
| 15 | 295±44 | 5689±552 | 56.7±6.6 | 82.4±10.2 | 1.46±0.11 | 7154±682 | 94 |
| 31 | 360±65 | 5619±1233 | 33.3±34.8 | 48.3±50.3 | 3.77±2.70 | 5436±705 | 66 |
| 33 | 382±88 | 5554±3021 | 18.6±17.5 | 27.7±24.2 | 6.4±5.57 | 3990±788 | 99 |
| 35 | 392±43 | 4328±910 | 7.5±1.92 | 10.6±2.3 | 7.93±1.47 | 5008±875 | 107 |

Conclusion: The compound of the present disclosure is well absorbed, and has a significant pharmacokinetic advantage.

## Claims

1. A compound of formula (I) or a tautomer, mesomer, racemate, enantiomer, diastereomer thereof, or mixture thereof, or a pharmaceutically acceptable salt thereof: 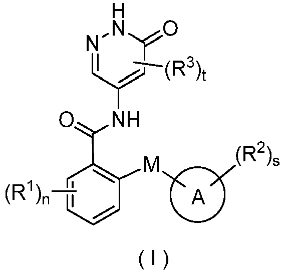 wherein:
M is selected from the group consisting of O atom, CR⁴R⁵ and S atom;
ring A is an aryl or heteroaryl, wherein the aryl or heteroaryl is optionally fused to a cycloalkyl or heterocyclyl;
each R¹ is identical or different and each is independently selected from the group consisting of hydrogen atom, halogen, alkyl, deuterated alkyl, deuterated alkoxy, alkoxy, haloalkyl, haloalkoxy, cyano, amino, nitro, hydroxy, hydroxyalkyl, cycloalkyl, heterocyclyl, aryl and heteroaryl;
each R² is identical or different and each is independently selected from the group consisting of hydrogen atom, halogen, alkyl, alkoxy, deuterated alkyl, deuterated alkoxy, haloalkyl, haloalkoxy, cyano, amino, nitro, hydroxy, hydroxyalkyl, cycloalkyl, cycloalkyloxy, heterocyclyl, aryl and heteroaryl, wherein the alkyl, cycloalkyl, heterocyclyl, aryl and heteroaryl are each optionally substituted by one or more substituents selected from the group consisting of alkyl, haloalkyl, halogen, amino, nitro, cyano, hydroxy, alkoxy, haloalkoxy, hydroxyalkyl, cycloalkyl, heterocyclyl, aryl and heteroaryl;
each R³ is identical or different and each is independently selected from the group consisting of hydrogen atom, halogen, alkyl, alkoxy, haloalkyl, cyano, amino, nitro, hydroxy, hydroxyalkyl, cycloalkyl, heterocyclyl, aryl and heteroaryl;
R⁴ and R⁵ are identical or different and are each independently selected from the group consisting of hydrogen atom, deuterium atom, halogen, alkyl, alkoxy, haloalkyl, cyano, amino, nitro, hydroxy, hydroxyalkyl, cycloalkyl, heterocyclyl, aryl and heteroaryl;
n is 0, 1, 2, 3 or 4;
s is 0, 1, 2, 3 or 4; and
t is 0, 1 or 2.

2. The compound of formula (I) or the tautomer, mesomer, racemate, enantiomer, diastereomer thereof, or mixture thereof, or the pharmaceutically acceptable salt thereof according to claim 1, wherein ring A is selected from the group consisting of phenyl, 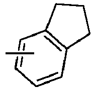 and pyridyl.

3. The compound of formula (I) or the tautomer, mesomer, racemate, enantiomer, diastereomer thereof, or mixture thereof, or the pharmaceutically acceptable salt thereof according to claim 1 or 2, wherein M is selected from the group consisting of O atom, CH₂ and S atom.

4. The compound of formula (I) or the tautomer, mesomer, racemate, enantiomer, diastereomer thereof, or mixture thereof, or the pharmaceutically acceptable salt thereof according to any one of claims 1 to 3, being a compound of formula (II) or a tautomer, mesomer, racemate, enantiomer, diastereomer thereof, or mixture thereof, or a pharmaceutically acceptable salt thereof: 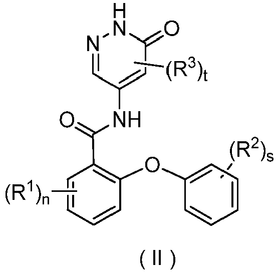 wherein:
R¹, R², R³, n, s and t are as defined in claim 1.

5. The compound of formula (I) or the tautomer, mesomer, racemate, enantiomer, diastereomer thereof, or mixture thereof, or the pharmaceutically acceptable salt thereof according to any one of claims 1 to 3, being a compound of formula (III) or a tautomer, mesomer, racemate, enantiomer, diastereomer thereof, or mixture thereof, or a pharmaceutically acceptable salt thereof: 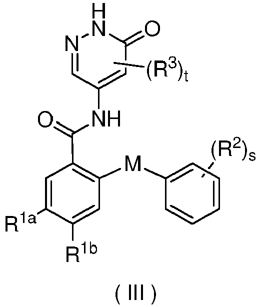 wherein:
M is selected from the group consisting of O atom, CH₂ and S atom;
R^{1a} is a halogen;
R^{1b} is selected from the group consisting of halogen, alkyl, alkoxy, haloalkyl and haloalkoxy; and
R², R³, s and t are as defined in claim 1.

6. The compound of formula (I) or the tautomer, mesomer, racemate, enantiomer, diastereomer thereof, or mixture thereof, or the pharmaceutically acceptable salt thereof according to any one of claims 1 to 4, wherein each R¹ is identical or different and each is independently selected from the group consisting of hydrogen atom, halogen, alkyl, alkoxy, haloalkyl and haloalkoxy.

7. The compound of formula (I) or the tautomer, mesomer, racemate, enantiomer, diastereomer thereof, or mixture thereof, or the pharmaceutically acceptable salt thereof according to any one of claims 1 to 6, wherein each R² is identical or different and each is independently selected from the group consisting of hydrogen atom, halogen, alkyl, deuterated alkyl, alkoxy, deuterated alkoxy, hydroxy, haloalkyl, haloalkoxy, cycloalkyl and cycloalkyloxy; preferably, each R² is identical or different and each is independently selected from the group consisting of hydrogen atom, halogen, C₁₋₆ alkyl, deuterated C₁₋₆ alkyl, C₁₋₆ alkoxy, deuterated C₁₋₆ alkoxy, haloC₁₋₆ alkyl, haloC₁₋₆ alkoxy, hydroxy, C₃₋₆ cycloalkyl and C₃₋₆ cycloalkyloxy; and more preferably, each R² is identical or different and each is independently selected from the group consisting of hydrogen atom, halogen, C₁₋₆ alkyl, C₁₋₆ alkoxy and deuterated C₁₋₆ alkoxy.

8. The compound of formula (I) or the tautomer, mesomer, racemate, enantiomer, diastereomer thereof, or mixture thereof, or the pharmaceutically acceptable salt thereof according to any one of claims 1 to 7, wherein s is 2.

9. The compound of formula (I) or the tautomer, mesomer, racemate, enantiomer, diastereomer thereof, or mixture thereof, or the pharmaceutically acceptable salt thereof according to any one of claims 1 to 8, being a compound of formula (IV) or a tautomer, mesomer, racemate, enantiomer, diastereomer thereof, or mixture thereof, or a pharmaceutically acceptable salt thereof: 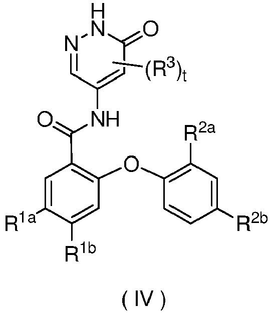 wherein:
R^{1a} is a halogen;
R^{1b} is selected from the group consisting of halogen, alkyl, alkoxy, haloalkyl and haloalkoxy;
R^{2a} is an alkoxy or deuterated alkoxy;
R^{2b} is selected from the group consisting of hydrogen atom, halogen, alkyl, alkoxy and haloalkoxy; and
R³ and t are as defined in claim 1.

10. The compound of formula (I) or the tautomer, mesomer, racemate, enantiomer, diastereomer thereof, or mixture thereof, or the pharmaceutically acceptable salt thereof according to any one of claims 1 to 9, wherein R³ is a hydrogen atom.

11. The compound of formula (I) or the tautomer, mesomer, racemate, enantiomer, diastereomer thereof, or mixture thereof, or the pharmaceutically acceptable salt thereof according to any one of claims 1 to 10, selected from the group consisting of:

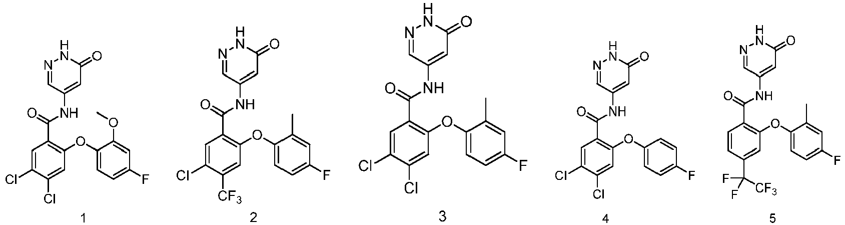

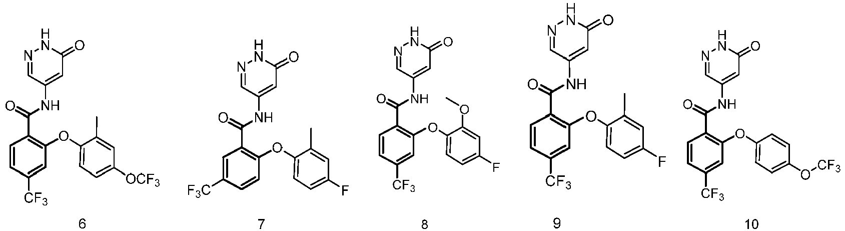

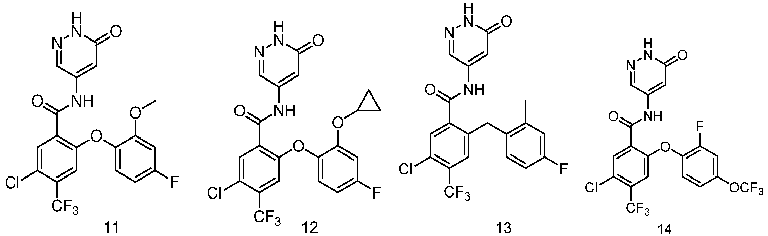

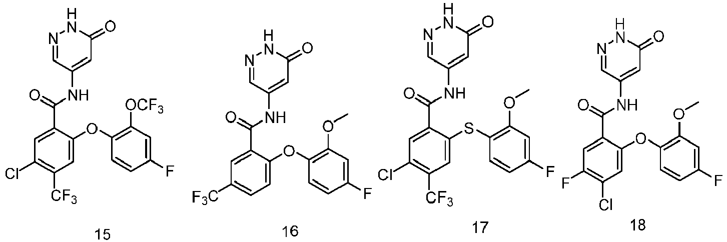

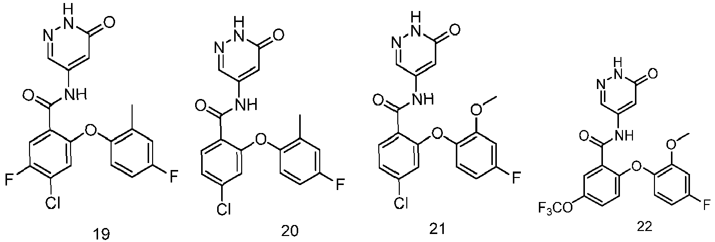

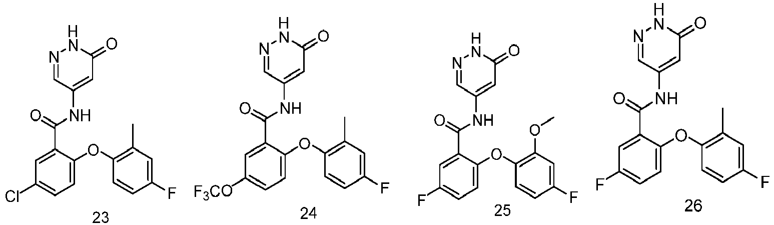

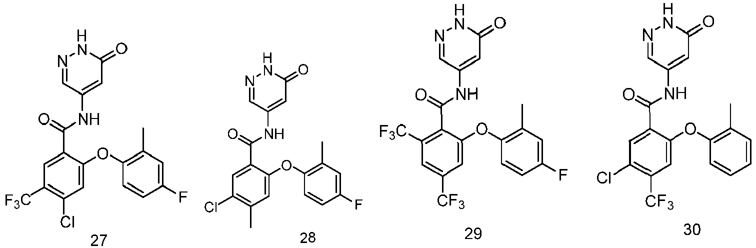

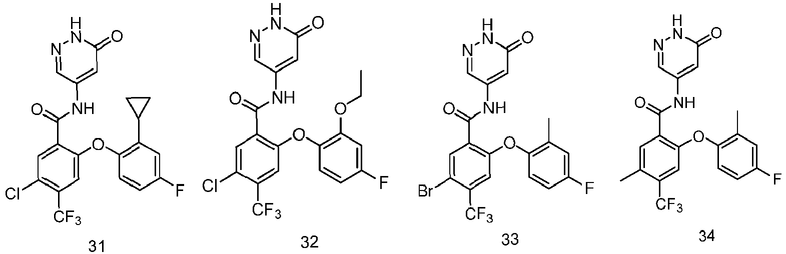

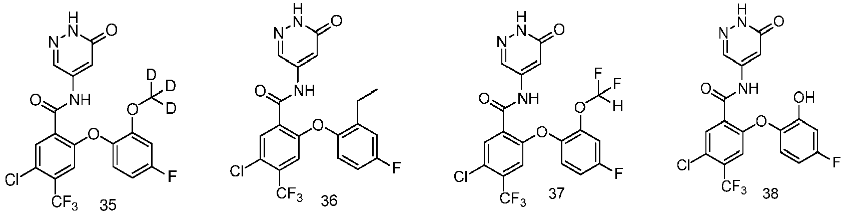

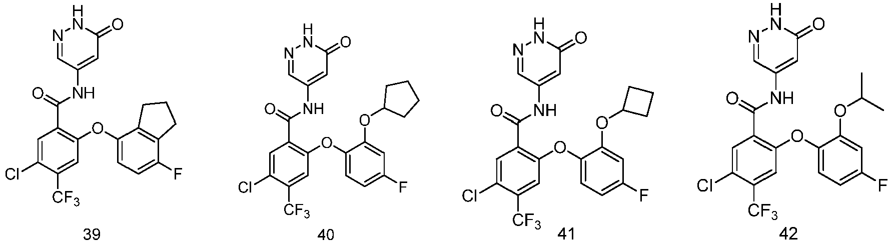

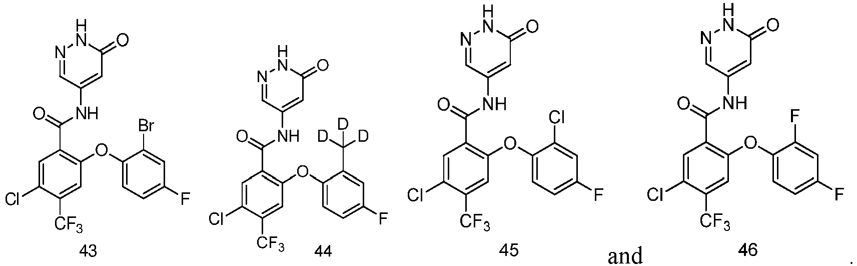

12. A compound of formula (IA) or a tautomer, mesomer, racemate, enantiomer, diastereomer thereof, or mixture thereof, or a pharmaceutically acceptable salt thereof: 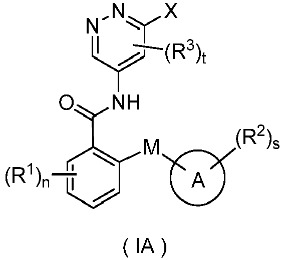 wherein:
X is a halogen; and
ring A, M, R¹, R², R³, n, s and t are as defined in claim 1.

13. The compound of formula (IA) or the tautomer, mesomer, racemate, enantiomer, diastereomer thereof, or mixture thereof, or the pharmaceutically acceptable salt thereof according to claim 12, selected from the group consisting of:

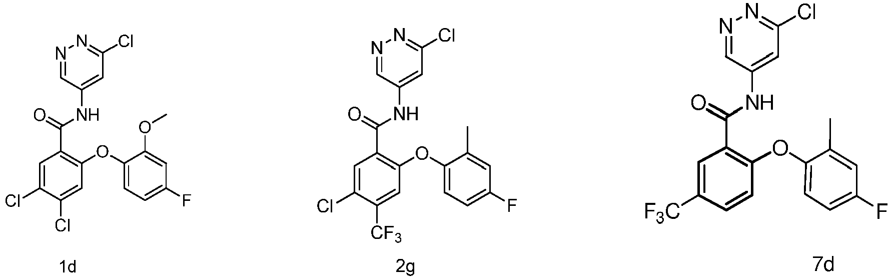

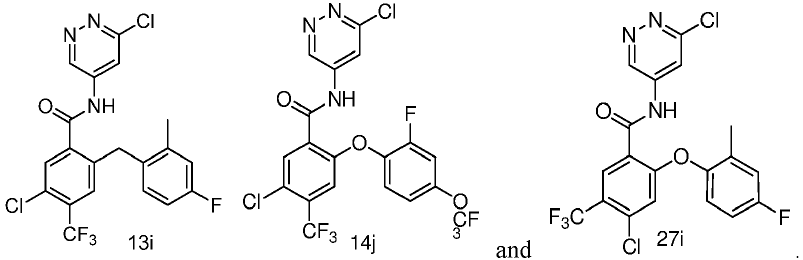

14. A compound of formula (IB) or a tautomer, mesomer, racemate, enantiomer, diastereomer thereof, or mixture thereof, or a pharmaceutically acceptable salt thereof: 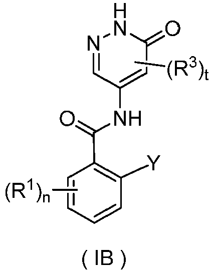 wherein:
Y is a halogen; and
R¹, R³, n and t are as defined in claim 1.

15. The compound of formula (IB) or the tautomer, mesomer, racemate, enantiomer, diastereomer thereof, or mixture thereof, or the pharmaceutically acceptable salt thereof according to claim 14, selected from the group consisting of:

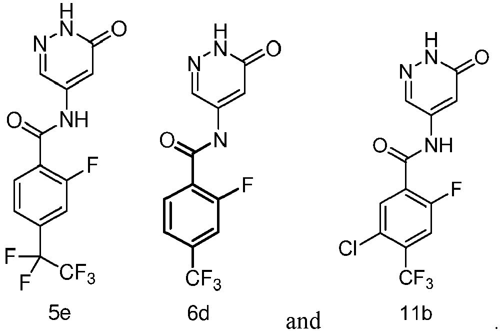

16. A method for preparing the compound of formula (I) or the tautomer, mesomer, racemate, enantiomer, diastereomer thereof, or mixture thereof, or the pharmaceutically acceptable salt thereof according to claim 1, comprising a step of: 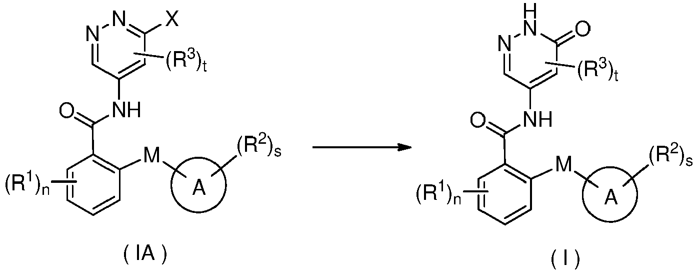 reacting a compound of formula (IA) to obtain the compound of formula (I); wherein:
X is a halogen; and
ring A, M, R¹, R², R³, n, s and t are as defined in claim 1.

17. A method for preparing the compound of formula (I) or the tautomer, mesomer, racemate, enantiomer, diastereomer thereof, or mixture thereof, or the pharmaceutically acceptable salt thereof according to claim 1, comprising a step of: 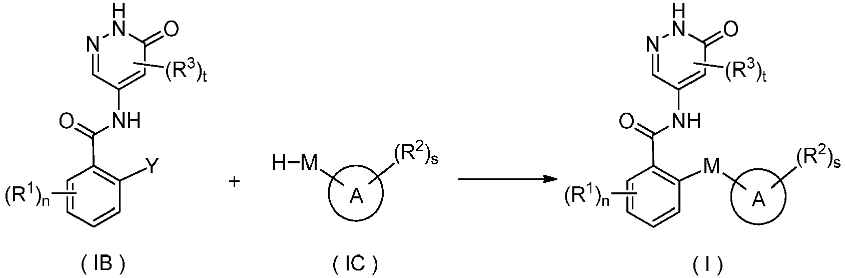 reacting a compound of formula (IB) and a compound of formula (IC) to obtain the compound of formula (I);
wherein:
Y is a halogen; and
ring A, M, R¹, R², R³, n, s and t are as defined in claim 1.

18. A pharmaceutical composition, comprising the compound of formula (I) or the tautomer, mesomer, racemate, enantiomer, diastereomer thereof, or mixture thereof, or the pharmaceutically acceptable salt thereof according to any one of claims 1 to 11, and one or more pharmaceutically acceptable carriers, diluents or excipients.

19. Use of the compound of formula (I) or the tautomer, mesomer, racemate, enantiomer, diastereomer thereof, or mixture thereof, or the pharmaceutically acceptable salt thereof according to any one of claims 1 to 11 or the pharmaceutical composition according to claim 18 in the preparation of a medicament for inhibiting the voltage-gated sodium channel in a subject, wherein the voltage-gated sodium channel is preferaby Na_{V}1.8.

20. Use of the compound of formula (I) or the tautomer, mesomer, racemate, enantiomer, diastereomer thereof, or mixture thereof, or the pharmaceutically acceptable salt thereof according to any one of claims 1 to 11 or the pharmaceutical composition according to claim 18 in the preparation of a medicament for treating and/or alleviating pain and pain-related diseases, multiple sclerosis, Charcot-Marie-Tooth syndrome, incontinence or cardiac arrhythmia, wherein the pain is preferaby selected from the group consisting of chronic pain, acute pain, inflammatory pain, cancer pain, neuropathic pain, musculoskeletal pain, primary pain, intestinal pain and idiopathic pain.
